(19) Europäisches Patentamt / European Patent Office / Office européen des brevets

(11) **EP 4 223 777 A1**

(12) **EUROPEAN PATENT APPLICATION**
published in accordance with Art. 153(4) EPC

(43) Date of publication:
**09.08.2023 Bulletin 2023/32**

(21) Application number: **21874469.6**

(22) Date of filing: **28.09.2021**

(51) International Patent Classification (IPC):
*C07K 16/28* (2006.01)   *C12N 5/10* (2006.01)
*C12N 15/13* (2006.01)   *C12N 15/63* (2006.01)
*A61K 39/395* (2006.01)   *A61P 35/00* (2006.01)

(52) Cooperative Patent Classification (CPC):
**A61K 39/395; A61P 35/00; C07K 16/00;
C07K 16/28; C12N 5/10; C12N 15/63**

(86) International application number:
**PCT/CN2021/121285**

(87) International publication number:
**WO 2022/068809 (07.04.2022 Gazette 2022/14)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.09.2020   CN 202011054187**

(71) Applicant: **Innovent Biologics (Suzhou) Co., Ltd.
Suzhou, Jiangsu 215123 (CN)**

(72) Inventors:
• **ZHOU, Shuaixiang**
  **Suzhou, Jiangsu 215123 (CN)**
• **GUAN, Zhe**
  **Suzhou, Jiangsu 215123 (CN)**
• **FU, Fenggen**
  **Suzhou, Jiangsu 215123 (CN)**
• **HU, Siyi**
  **Suzhou, Jiangsu 215123 (CN)**

(74) Representative: **Sagittarius IP
Marlow International
Parkway
Marlow, SL7 1YL (GB)**

Remarks:
The complete document including Reference Table(s) and the Sequence Listing(s) can be downloaded from the EPO website

(54) **ANTI-CD3 ANTIBODY AND USES THEREOF**

(57)   A novel antibody and antibody fragment specifically biding to CD3, and composition comprising the antibody or antibody fragment. Nucleic acids encoding the antibody or the antibody fragment thereof, a host cell comprising the nucleic acids, relevant uses, and therapeutic and diagnostic uses of the antibody and of the antibody fragment.

EP 4 223 777 A1

**Description**

The invention relates to a novel humanized antibody and an antibody fragment that specifically bind to CD3 and a composition comprising the antibody or the antibody fragment. In addition, the invention also relates to a bispecific antibody against CD3 and other antigens. Further, the invention relates to a nucleic acid encoding the antibody or the antibody fragment thereof, a host cell comprising the nucleic acid, and related uses. Furthermore, the invention relates to therapeutic and diagnostic uses of these antibodies and antibody fragments.

**Background of the invention**

CD3 (differentiation cluster 3) is a protein complex, which constitutes the T cell receptor complex with T cell antigen receptor $\alpha$, T cell antigen receptor $\beta$ and two $\zeta$ chains together and participates in the activation of cytotoxic T cells (CD8+ naive T cells) and T helper cells (CD4+ naive T cells).

CD3 protein complex is the definitive marker of T cell lineage, so anti-CD3 antibody can be effectively used as T cell marker.

CD3 antibody recognizes all T cells and reacts with 70% - 80% of human peripheral blood lymphocytes and 65% - 85% of thymocytes. T cells activated by CD3 antibody are directed to the periphery of tumor cells and the two cells contact and form synapses to trigger the activation of T cell receptor (TCR) signal pathway, and the expression and release of granzyme, whereby causing the perforation of tumor cell membrane, leading to cytolysis and apoptosis of the latter. The activation of TCR signal pathway simultaneously causes the expression and release of a series of cytokines, such as the release feedback of IL-2 to stimulate the proliferation of T cells and amplify the immune killing effect. The data of preclinical studies showed that CD3 bispecific antibody molecules targeting tumor associated antigen can effectively activate T cells, stimulate their proliferation, and cause the death of target cell s in the presence of target cells.

Many kinds of CD3 binding antibody molecules are known, especially the bispecific antibody molecules comprising CD3 binding specificity. At present, the commonly used public CD3 antibodies come from mouse antibodies on hybridoma platform in the 1980s, including the following: OKT3, TR66, UCHT1, L2K, SP34, etc. The species cross-reactivity of CD3 monoclonal antibody is critical to the development of CD3 bispecific antibody.

The affinity of CD3 antibody to CD3 complex is the first key factor for the success of CD3 related bispecific antibody. CD3 antibodies with over-high affinity will, on the one hand, cause non-specific activation of T cells, resulting in unnecessary cytokine release syndrome, and on the other hand, it will preferentially target peripheral T cells in vivo and less act on tumor cells, resulting in decreased efficacy. However, CD3 antibody with over-low affinity is not enough to activate T cells and make them play a role of killing. It is necessary to adjust the CD3 affinity of CD3-related bispecific antibodies based on the molecular weight, expression level, antibody epitopes and tissue distribution characteristics of different tumor-related antigens.

Therefore, there are needs in the art to develop anti-CD3 monoclonal antibodies with different binding affinity and thus different T cell activation capabilities, which can be used to develop multispecific antibodies that meet different tumor-related antigens, such as bispecific antibodies or trispecific antibodies.

**Summary of The invention**

In some aspects, the invention relates to antibodies binding to CD3 or antigen-binding fragments thereof, which comprise three heavy chain variable region CDRs and three light chain variable region CDRs described in the invention.

In some aspects, the CD3-binding antibodies or antigen-binding fragment thereof of the invention comprises the heavy chain variable region and/or light chain variable region described in the invention.

In some aspects, the CD3-binding antibodies or antigen-binding fragment thereof of the invention further comprises the heavy chain constant region and/or light chain constant region described in the invention.

In some embodiments, the CD3-binding antibodies or antigen-binding fragment thereof of the invention bind to CD3 antigen, such as human or cynomolgus monkey CD3, with various binding affinity, such as low binding affinity, such as undetectable binding affinity.

**Description of figures:**

Figure 1: Figures 1A-1C show the binding affinity of sp34 humanized antibody to human CD3 at the cell level, and Figure 1D shows the binding of humanized antibodies with different CD3 affinity at the cell level.

Figure 2: T cell activation test of Sp34 humanized antibody.

Figure 3: Figure 3A and Figure 3B show the binding affinity of the CDR region mutant of sp34 humanized antibody to human CD3 at the cell level, and Figure 3C further shows the affinity of some CDR mutants to CD3 at the cell level.

Figure 4: T cell activation test of sp34 humanized antibody CDR mutant.

Figure 5: Figure 5A is the schematic diagram of Her2/CD3 bispecific antibody molecule; Figure 5B shows the T cell activation ability of the bispecific antibody molecule.

Figure 6: Figure 6Ais the schematic diagram of CD70/CD3 bispecific antibody molecule; Figure 6B shows the T cell activation ability of the bispecific antibody molecule.

Figure 7 shows the structure diagram of CD3/Claudin18.2 bispecific antibody.

Figure 8 shows that the bispecific antibody of the invention specifically kills CLDN18.2-positive gastric cancer cell NUGC-4.

Figure 9 shows that the bispecific antibody of the invention specifically kills CLDN18.2-positive pancreatic cancer cell DAN-GCLDN18.2.

Figure 10 shows that the bispecific antibody has no non-specific killing effect on CLDN18.2 negative cells.

Figure 11 shows the cytokine release mediated by T cells on which bispecific antibody depends in NUGC-4.

Figure 12 shows the cytokine release mediated by T cells on which bispecific antibody depends in DAN-G-CLDN18.2.

Figure 13 shows T-cell activation mediated by bispecific antibody on which CLDN18.2 expression depends.

Figure 14 shows the efficacy results of bispecific antibody in the humanized model of NUGC-4 gastric cancer in vivo.

Figure 15 shows the efficacy results of the bispecific antibody in the humanized model of DAN-G-CLDN18.2 pancreatic cancer in vivo.

Figure 16 shows the PK of bispecific antibodies in mice.

## Detailed Description of The Invention

### I. Definition

Before the invention is described in detail below, it should be understood that the invention is not limited to the particular methodology, protocols, and reagents described herein, as these may vary. It should also be understood that the terminology used herein is for the purpose of describing particular embodiments only, and is not intended to limit the scope of the invention, which will be limited only by the appended claims. Unless otherwise defined, all technical and scientific terms used herein have the same meaning as commonly understood by those of ordinary skill in the art to which the invention belongs.

For the purpose of explaining this specification, the following definitions will be used, and wherever appropriate, terms used in the singular may also include the plural and vice versa. It is understood that the terminology used herein is for the purpose of describing particular embodiments only and is not intended to be limiting.

The term "about" used in combination with a numerical value is intended to encompass the numerical values in a range from a lower limit less than the specified numerical value by 5% to an upper limit greater than the specified numerical value by 5%.

The term "and/or" as used herein, means any of the options or two or more of the options.

The term "comprise" or "include" as used herein means including the elements, integers or steps described, but does not exclude any other elements, integers or steps. The term also covers the combination of the elements, integers or steps mentioned herein when the term "comprises" or "include" is used, unless otherwise specified. For example, it is also intended to cover the antibody variable region composed of the specific sequence when referring to the antibody variable region "comprises" a specific sequence.

The term "CD3" as used herein refers to the antigen expressed on T cells as part of the multi-molecule T cell receptor (TCR), and it is composed of homodimer or heterodimer formed by two of the following four receptor chains: CD3-$\varepsilon$, CD3-$\delta$, CD3-$\zeta$ and CD3-$\gamma$. Human CD3-$\varepsilon$ n (hCD3 $\varepsilon$) comprises the amino acid sequence described in Uni-ProtKB/Swiss-Prot: P07766.2. Human CD3- $\delta$ (hCD3 $\delta$) comprises the amino acid sequence described in Uni-ProtKB/Swiss-Prot: P04234.1. In some embodiments, the CD3 described in this invention refers to CD3 from human or cynomolgus monkeys.

The term " antibody binding to CD3" or "anti-CD3 antibody" as used herein includes the antibody specifically recognizing or binding to a single CD3 subunit (e.g $\varepsilon$, $\delta$, $\gamma$ or $\zeta$) and the antigen-binding fragment thereof, and the antibody specifically recognizing and binding to the dimer complex of two CD3 subunits (for example, $\gamma/\varepsilon$, $\delta/\varepsilon$ and $\zeta/\zeta$ CD3 dimer) and the antigen-binding fragment thereof. The antibody and antigen-binding fragment of the invention can bind to soluble CD3, binding CD3 and/or CD3 expressed on the cell surface. Soluble CD3 comprises natural CD3 protein and recombinant CD3 protein variants, such as monomer and dimer CD3 structures that lack transmembrane regions or otherwise do not bind to cell membranes. The invention provides antibodies that bind human and cynomolgus monkey CD3 with low or undetectable binding affinity to activate human and cynomolgus monkey T cells. In some embodiments, the binding is measured, for example, by radioimmunoassay (RIA), biomembrane thin-layer interferometry (BLI), MSD assay or surface plasmon resonance (SPR) or flow cytometry.

The term "CD3 expressed on the cell surface" refers to one or more CD3 proteins, which are expressed on the cell surface in vivo or in vitro, so that at least part of CD3 proteins is exposed to the outside of the cell membrane and are easy to approach the antigen-binding part of the antibody. "CD3 expressed on the cell surface" comprise CD3 protein contained in the functional T cell receptor environment in the cell membrane. The term "CD3 expressed on the cell surface" comprises CD3 protein expressed as a part of homodimer or heterodimer on the cell surface (for example, $\delta/\varepsilon$, $\gamma/\varepsilon$ and $\zeta/\zeta$ CD3 dimer).

The effector cells include effector T cells (T lymphocytes), such as CD4+T cells, CD8+T cells, Th1, Th2 and regulatory T cells (Tregs). Effector cells further comprise natural killer cells, macrophages, granulocytes, plasma cells or B cells (lymphocytes).

"Anti CD3 antibody" or " antibody binding to CD3" includes monovalent antibody with single specificity, bispecific antibody containing the first antigen-binding domain binding to CD3 and the second antigen-binding domain binding to the second (target) antigen, and multispecific antibody binding to CD3 and one or more other (for example, two) targets.

The term "multi-specific antibody" refers to an antibody that is at least bispecific, that is, the antibody comprises at least the first binding domain and the second binding domain, wherein the first binding domain binds one target or antigen and the second binding domain binds another antigen or target. Therefore, the antibody according to the invention comprises specificity for at least two different antigens or targets. The antibody according to the invention also covers a multi-specific antibody including a plurality of binding domains/binding sites, such as a trispecific antibody, wherein the antibody comprises three binding domains.

The term "linker" as used herein refers to any molecule that enables directly joining the different parts of a bispecific antibody. Examples of linker that establish covalent join between different antibody parts include peptide linker and non-protein liner, including but not limited to polyethylene glycol (PEG), polypropylene glycol, polyethylene oxide or copolymers of polyethylene glycol and polypropylene glycol.

The term "peptide linker" according to the invention refers to the sequence of amino acids, wherein the sequence joins the amino acid sequence of the first part of the antibody to the second part of the antibody. For example, the peptide linker may join the first (variable and/or binding) domain of the antibody to the second variable and/or binding) domain. For example, the peptide linker can also join one part of the antibody to another part of the antibody, such as join the antigen-binding domain to the Fc domain or fragment thereof. Preferably, the peptide liner has such a length that it is sufficient to join two entities in such a way that they maintain their conformation relative to each other, so as not to hinder the desired activity.

The term "valence" according to the invention means that there is a specified number of binding sites in the antibody molecule. Therefore, the terms bivalent, trivalent and tetravalent respectively indicate that there are two, three or four binding sites in the antibody construct. The bispecific antibody according to the invention is at least bivalent and can be multivalent, such as bivalent, trivalent, tetravalent or hexavalent.

The term "binding region" as used herein refers to any part of a bispecific antibody that binds to a specific target or antigen. Binding regions are antigen-binding sites. The binding region may be, for example, an antibody or immunoglobulin itself or an antibody fragment. Such binding regions may have or not have a tertiary structure independent of the rest of BsAB, and can be used as a separate entity binding to or not binding to its target.

The term "antibody fragment" comprises a portion of the complete antibody. In a preferred embodiment, the antibody fragment is an antigen-binding fragment.

"Antigen-binding fragment" refers to a molecule different from an intact antibody, which comprises a portion of the intact antibody and binds to an antigen to which the intact antibody binds. Examples of the antibody fragment include but are not limited to Fv, Fab, Fab', Fab'-SH, F(ab')$_2$; dAb(domain antibody); a linear antibody; a single-chain variable fragment (e.g., scFv); a single-domain antibody, e.g., VHH; a bivalent antibody or a fragment thereof; a Camelidae antibody.

The term "antigen" refers to the molecule that triggers the immune response. Such immune response may involve antibody production or activation of specific immune cells, or both. Technicians will understand that any macromolecule, including basically all proteins or peptides, can be used as an antigen. In addition, antigens can be derived from recombinant or genomic DNA. The term "epitope" as used herein refers to the part of an antigen (e.g. CD3) that specifically interacts with the antibody molecule.

"Antibody that binds to the same or overlapping epitope" as a reference antibody refers to an antibody that blocks 50%, 60%, 70%, 80%, 90%, or 95% or more of the binding of the reference antibody to its antigen in a competition assay, or conversely, the reference antibody blocks 50%, 60%, 70%, 80%, 90%, or 95% or more of the binding of the antibody to its antigen in a competition assay.

An antibody that competes with a reference antibody to bind to its antigen refers to an antibody that blocks 50%, 60%, 70%, 80%, 90%, or 95% or more of the binding of the reference antibody to its antigen in a competition assay. Conversely, the reference antibody blocks 50%, 60%, 70%, 80%, 90%, or 95% or more of the binding of the antibody to its antigen in a competition assay. Numerous types of competitive binding assays can be used to determine whether an antibody competes with another, such as direct or indirect solid-phase radioimmunoassay (RIA), direct or indirect solid-phase enzyme immunoassay (EIA), and sandwich competition assay.

An antibody that inhibits (e.g., competitively inhibits) the binding of a reference antibody to its antigen refers to an antibody that inhibits 50%, 60%, 70%, 80%, 90%, or 95% or more of the binding of the reference antibody to its antigen. Conversely, the reference antibody inhibits 50%, 60%, 70%, 80%, 90%, or 95% or more of the binding of the antibody to its antigen. The binding of an antibody to its antigen can be measured by affinity (e.g., equilibrium dissociation constant). Methods for determining affinity are known in the art.

An antibody that shows the same or similar binding affinity and/or specificity as a reference antibody refers to an antibody that is capable of having at least 50%, 60%, 70%, 80%, 90%, or 95% or more of the binding affinity and/or specificity of the reference antibody. This can be determined by any methods known in the art for determining binding affinity and/or specificity.

"Complementarity determining region" or "CDR region" or "CDR" is a region in an antibody variable domain that is highly variable in sequence and forms a structurally defined loop ("hypervariable loop") and/or comprises antigen contact residues ("antigen contact point"). CDRs are primarily responsible for binding to epitopes. The CDRs of the heavy and light chains are generally referred to as CDR1, CDR2, and CDR3, and are numbered sequentially from N-terminus. The CDRs located in the variable domain of the antibody heavy chains are referred to as HCDR1, HCDR2, and HCDR3, while the CDRs located in the variable domain of the antibody light chains are referred to as LCDR1, LCDR2, and LCDR3. In a given amino acid sequence of a light chain variable region or a heavy chain variable region, the exact amino acid sequence boundaries of each CDR can be determined using any one or a combination of many well-known antibody CDR assignment systems including, e.g., Chothia based on the three-dimensional structure of antibodies and the topology of the CDR loops (Chothia et al. (1989) Nature 342: 877-883; Al-Lazikani et al., "Standard conformations for the canonical structures of immunoglobulins", Journal of Molecular Biology, 273, 927-948 (1997)), Kabat based on antibody sequence variability (Kabat et al., Sequences of Proteins of Immunological Interest, 4th edition, U.S. Department of Health and Human Services, National Institutes of Health (1987)), AbM (University of Bath), Contact (University College London), International ImMunoGeneTics database (IMGT) (imgt.cines.fr/ on the World Wide Web), and North CDR definition based on the affinity propagation clustering using a large number of crystal structures.

For example, according to different CDR determination schemes, the residues of each CDR are as follows.

| CDR | Kabat scheme | AbM scheme | Chothia scheme | Contact scheme |
|---|---|---|---|---|
| LCDR1 | L24-L34 | L24-L34 | L26-L32 | L30-L36 |
| LCDR2 | L50-L56 | L50-L56 | L50-L52 | L46-L55 |
| LCDR3 | L89-L97 | L89-L97 | L91-L96 | L89-L96 |
| HCDR1 | H31-H35B | H26-H35B | H26-H32 | H30-H35B |
| (Kabat numbering system) | | | | |
| HCDR1 | H31-H35 | H26-H35 | H26-H32 | H30-H35 |
| (Chothia numbering system) | | | | |
| HCDR2 | H50-H65 | H50-H58 | H53-H55 | H47-H58 |
| HCDR3 | H95-H102 | H95-H102 | H96-H101 | H93-H101 |

(continued)

| CDR | Kabat scheme | AbM scheme | Chothia scheme | Contact scheme |
|---|---|---|---|---|
| (Kabat numbering system) | | | | |

CDRs can also be determined based on having the same Kabat numbering positions as a reference CDR sequence (e.g., any of the exemplary CDRs of the invention).

The term "CDR" or "CDR sequence" encompasses CDR sequences determined by any of the manners described above in the invention, unless otherwise stated.

Unless otherwise stated, in the invention, when referring to the positions of residues in an antibody variable region (including residues in a heavy chain variable region and residues in a light chain variable region), it refers to the numbering positions according to the Kabat numbering system (Kabat et al., Sequences of Proteins of Immunological Interest, 5th Ed. Public Health Service, National Institutes of Health, Bethesda, Md. (1991)).

In one embodiment, the heavy chain variable region CDR of the antibody of the invention is determined according to the following rules:
VH CDR1 is determined according to AbM rules; and VH CDR2 and 3 are determined according to Kabat rules.

In one embodiment, the light chain variable region CDR of the antibody of the invention is determined according to the Kabat rule.

In one embodiment, the heavy chain variable region CDR of the antibody of the invention is determined according to the following rules: VH CDR1 is determined according to the AbM rule; and VH CDR2 and 3 are determined according to Kabat rules; and the CDR of light chain variable area is determined according to Kabat rule.

It should be noted that boundaries of CDRs of variable regions of an antibody obtained by different assignment systems may differ. That is, CDR sequences of variable regions of an antibody defined by different assignment systems differ. Therefore, when it comes to defining an antibody with specific CDR sequences defined in the invention, the scope of the antibody also encompasses such antibody whose variable region sequences comprise the specific CDR sequences, but having claimed CDR boundaries different from the specific CDR boundaries defined by the invention as a different protocol (e.g., different assignment system rules or their combinations) is applied.

Antibodies with different specificities (i.e., different binding sites for different antigens) have different CDRs (under the same assignment system). However, although CDRs differ from antibody to antibody, only a limited number of amino acid positions within the CDRs are directly involved in antigen binding. The smallest overlapping region can be determined using at least two of the Kabat, Chothia, AbM, Contact, and North methods, thereby providing a "minimal binding unit" for antigen binding. The minimal binding unit may be a sub-portion of the CDR. As will be clear to those skilled in the art, residues of the rest CDR sequences can be determined by antibody structure and protein folding. Therefore, any variants of the CDRs given herein will also be considered in the invention. For example, in one CDR variant, the amino acid residues in the minimal binding unit may remain unchanged, while other CDR residues defined by Kabat or Chothia may be substituted by conservative amino acid residues.

The term "Fc region" is used herein to define the constant regions of CH2 and CH3 of the immunoglobulin heavy chain and the term includes the natural sequence Fc region and the variant Fc region. The natural Fc region can bind to different Fc receptors on the surface of immune cells, which can cause CDC\ADCC\ADCP effector function. Such effector functions generally require the combination of Fc region and binding domain (such as antibody variable domain). In some embodiments, the Fc region is mutated to enhance its CDC\ADCC\ADCP effector function. In some embodiments, the Fc region is mutated to weaken or delete its CDC\ADCC\ADCP effector function.

"Antibody in the form of IgG " refers to the IgG form that the heavy chain constant region of the antibody belonging to. Heavy chain constant regions of all antibodies of the same type are identical, and heavy chain constant regions of antibodies of different types are different. For example, an antibody in the form of IgG4 refers to the Ig domain of its heavy chain constant region from IgG4, or the antibody in the form of IgG1 refers to its heavy chain constant region from IgG1.

"Humanized" antibody refers to an antibody comprising amino acid residues from non-human CDR and human FR. In some embodiments, humanized antibodies will comprise basically all of at least one, usually two variable domains, where all or substantially all of the CDRs (for example, CDR) correspond to those of non-human antibodies, and all or substantially all of the FRs correspond to those of human antibodies. The humanized antibody can optionally comprise at least a portion of the antibody constant region derived from the human antibody. The "humanized form" of antibody (such as non-human antibody) refers to the antibody that has been humanized.

"Knobs-into-holes" technology was described in such as US 5731168; US 7695936. Generally, this method involves introducing a "knob" at the interface of the first polypeptide and a corresponding "hole" at the interface of the second polypeptide, so that the knob can be placed in the hole, thus promoting the formation of heterodimer and blocking the formation of homodimer. The knob is constructed by replacing the small amino acid side chain from the interface of the first polypeptide with a larger side chain (such as tyrosine or tryptophan). By replacing the large amino acid side chain with the smaller side chain (such as alanine or threonine), a compensating hole of the same or similar size as the knob is created in the interface of the second polypeptide. The knob and hole can be generated by changing the nucleic acid encoding the polypeptide, for example, by site-specific mutagenesis, or by peptide synthesis.

The term "binding" or "specific binding" as used herein means that binding interactions to antigen are selective and can be distinguished from unwanted or non-specific interactions. The ability of antigen binding sites to bind to specific antigens can be determined by enzyme-linked immunosorbent assay (ELISA) or conventional binding assay known in the art, such as radioimmunoassay (RIA), thin-layer biomembrane interference assay, MSD assay or surface plasmon resonance (SPR).

"Immunoconjugate" is an antibody that is conjugated with one or more other substances (including but not limited to cytotoxic agents or labels).

The term "therapeutic agent" as described herein comprises any substance effective in preventing or treating tumors (such as cancer), including a chemotherapeutic agent, a cytokine, a cytotoxic agent, other antibodies, a small molecule drug or an immunomodulatory agent (such as an immunosuppressant).

The term "cytotoxic agent" used in the invention refers to a substance that inhibits or prevents the cell function and/or causes cell death or destruction.

"Chemotherapeutic agents" include chemical compounds useful in treatment of immune system disease.

The term "small molecule drugs" refers to organic compounds with low molecular weight that can regulate biological processes. "Small molecule" is defined as a molecule with molecular weight less than 10kD, generally less than 2kD and preferably less than 1kD. Small molecules include but are not limited to inorganic molecules, organic molecules, organic molecules containing inorganic components, molecules containing radioactive atoms, synthetic molecules, peptide mimics and antibody mimics. As a therapeutic agent, small molecules can penetrate cells more easily than large molecules, and are less susceptible to degradation and less prone to trigger immune response.

The term "immunomodulators" as used herein refer to natural or synthetic active agents or drugs that inhibit or regulate immune response. The immune response can be humoral or cellular. Immunomodulators include immunosuppressants.

"Immunosuppressants", "immunosuppressive drugs", or "immunosuppressors" as used herein are therapeutic agents used to suppress or block immune system activity in immunosuppressive therapy.

The term "effective amount" refers to the amount or dose of the antibody or fragment or conjugate or composition or combination of the invention, which will produce the expected effect in patients needing such treatment or prevention after being administered to patients in a single or multiple dose.

"Therapeutically effective amount" refers to the amount that can effectively achieve the desired results at the required dose and for the required period of time. The therapeutically effective amount is also such an amount, where any toxic or harmful effect of antibody or antibody fragment or conjugate or composition or combination is less than the therapeutic beneficial effect. "Therapeutically effective amount" preferably inhibits measurable parameters (such as tumor volume) by at least about 20%, more preferably by at least about 40%, or even more preferably by at least 50%, 60%, or 70% compared to untreated objects.

"Preventively effective amount" refers to the amount that can effectively achieve the desired prevention results at the required dose and for the required period of time. Generally, since the preventive dose is used before or at an earlier stage of the disease in the objects, the preventively effective amount will be less than the therapeutically effective amount.

The terms "host cell", "host cell line" and "host cell culture" are used interchangeably and refer to the cells in which foreign nucleic acids are introduced, including the descendants of such cells. Host cells include "transformants" and "transformed cells", which include primary transformed cells and offspring derived from them, regardless of the number of passages. The nucleic acid content of the offspring may not be exactly the same as that of the parent cell, but may contain mutations. The mutant progeny with the same function or biological activity screened or selected from the initially transformed cells are included herein.

The term "label" as used herein refers to a compound or composition that is directly or indirectly conjugated or fused to a reagent (such as a polynucleotide probe or antibody) and facilitates the detection of the conjugated or fused reagent. The label itself can be detectable (for example, radioisotope label or fluorescent label) or can catalyze the chemical changes of detectable substrate compounds or compositions in the case of enzymatic labeling. The term is intended to cover the direct labeling of probes or antibodies by coupling (i.e., physically connecting) detectable substances to probes or antibodies and the indirect labeling of probes or antibodies by reacting with another directly labeled reagent.

"Individuals" or "subjects" include mammals. Mammals include, but are not limited to, domestic animals (such as cattle, sheep, cats, dogs and horses), primates (such as human and non-human primates, such as monkeys), rabbits, and rodents (such as mice and rats). In some embodiments, the individuals or subjects are human.

"Isolated" antibodies are antibodies that have been separated from their natural environment components. In some embodiments, the antibody is purified to more than 95% or 99% purity, such as by electrophoresis (for example, SDS-PAGE, isoelectric focusing (IEF), capillary electrophoresis) or chromatography (for example, ion exchange or reverse phase HPLC).

" Isolated nucleic acid encoding anti-CD3 antibody or fragments thereof" refers to one or more nucleic acid molecules, which encode the heavy chain or light chain of the antibody (or fragments thereof, such as the heavy chain variable region or light chain variable region), including such nucleic acid molecules in a single vector or separate vectors, and such nucleic acid molecules present in one or more positions in the host cell.

The calculation of sequence identity between sequences is performed as follows.

To determine the percent identity of two amino acid sequences or two nucleic acid sequences, the sequences are aligned for optimal comparison purposes (e.g., for optimal alignment, gaps can be introduced in the first and second amino acid sequences or in one or both of nucleic acid sequences, or non-homologous sequences can be discarded for comparison purposes). In one preferred embodiment, for comparison purposes, the length of the aligned reference sequence is at least 30%, preferably at least 40%, more preferably at least 50%, 60%, and even more preferably at least 70%, 80%, 90%, 100% of the length of the reference sequence. Amino acid residues or nucleotides at corresponding amino acid positions or nucleotide positions are then compared. When a position in the first sequence is occupied by the same amino acid residue or nucleotide at the corresponding position in the second sequence, then the molecules are identical at this position.

A mathematical algorithm can be used to achieve the sequence comparison and calculation of percent identity between two sequences. In one preferred embodiment, the percent identity between two amino acid sequences is determined with the Needlema and Wunsch ((1970) J. Mol. Biol., 48:444-453) algorithm (available at http://www.gcg.com) which has been integrated into the GAP program of the GCG software package, using the Blossom 62 matrix or PAM250 matrix and gap weights of 16, 14, 12, 10, 8, 6, or 4 and length weights of 1, 2, 3, 4, 5, or 6. In yet another preferred embodiment, the percent identity between two nucleotide acid sequences is determined with the GAP program (available at http://www.gcg.com) of the GCG software package, using the NWSgapdna.CMP matrix and gap weights of 40, 50, 60, 70, or 80 and length weights of 1, 2, 3, 4, 5 or 6. A particularly preferred parameter set (and one that should be used unless otherwise stated) is a Blossom 62 scoring matrix with a gap penalty of 12, a gap extension penalty of 4, and a frameshift gap penalty of 5. The percent identity between two amino acid sequences or nucleotide sequences can also be determined with PAM120 weighted remainder table, gap length penalty of 12 and gap penalty of 4, using the E. Meyers and W. Miller algorithms which have been incorporated into the ALIGN program (version 2.0) ((1989) CABIOS, 4:11-17). Additionally or alternatively, the nucleic acid sequences and protein sequences described herein can be further used as "query sequences" to perform searches against public databases to, e.g., identify other family member sequences or related sequences.

As used herein, the term "hybridization under stringent conditions, such as under conditions of low stringency, medium stringency, high stringency, or extreme stringency" describes hybridization and washing conditions. Instructions for performing hybridization reactions can be found in Current Protocols in Molecular Biology, John Wiley & Sons, N.Y. (1989), 6.3.1-6.3.6, which is incorporated by reference. Aqueous and non-aqueous methods are described in the references and either method can be used. The specific hybridization conditions mentioned herein are as followed: 1) low stringency hybridization conditions are in 6 X sodium chloride/sodium citrate (SSC) at about 45 °C, followed by two washes in 0.2 X SSC, 0.1% SDS at least at 50 °C (for low stringency conditions, the temperature of the washes can be increased to 55 °C); 2) medium stringency hybridization conditions are in 6 X SSC at about 45 °C, followed by one or more washes in 0.2 X SSC, 0.1% SDS at about 60 °C; 3) high stringency hybridization conditions are in 6 X SSC at about 45 °C, followed by one or more washes in 0.2 X SSC, 0.1% SDS at 65 °C; and preferably 4) extreme stringency hybridization conditions are in 0.5 M sodium phosphate, 7% SDS at 65 °C, followed by one or more washes in 0.2X SSC, 0.1% SDS at 65 °C. Extreme stringency condition (4) is a preferred condition and the one that should be used unless otherwise stated.

The term "anti-tumor effect" refers to biological effects that can be demonstrated by various means, including but not limited to, for example, reduction of tumor volume, tumor cell number tumor cell proliferation or tumor cell survival.

The terms "tumor" and "cancer" are used interchangeably herein, covering solid tumors and liquid tumors.

The terms "cancer" and "cancerous" refer to or describe physiological diseases in mammals characterized by unregulated cell growth. In some embodiments, cancers suitable for treatment by the antibodies of the invention include gastric cancer or pancreatic cancer, including metastatic forms of those cancers.

The term "tumor" refers to the growth and proliferation of all neoplastic cells, whether malignant or benign, as well as all pre-cancerous and cancerous cells and tissues. The terms "cancer", "cancerous" and "tumor" are not mutually exclusive when mentioned herein.

As used herein, "tumor associated antigen" refers to the antigenic determinant exhibited on the surface of the target cell, where the target cell is the cell in the tumor, such as cancer cells and tumor matrix cells. In some respects, tumor associated antigens are HER2 or CD70 or CLAUDIN18.2.

The term "pharmaceutical supplementary material" refers to diluents, adjuvants (e.g., Freund's adjuvants (complete and incomplete)), excipients, carriers, or stabilizers, etc., which are co-administered with active substance.

The term "pharmaceutical composition" refers to such a composition that exists in a form which allows the biological activity of the active ingredient contained therein to be effective, and does not comprise additional ingredients having unacceptable toxicity to a subject to which the composition is administered.

The term "pharmaceutical combination" refers to non-fixed combination products or fixed combination products, including but not limited to drug kits and drug compositions. The term "unfixed combination" means that the active ingredients (for example, (i) the anti-CD3 antibody or fragments thereof in the invention, and (ii) other therapeutic agents) are administered to patients simultaneously, without specific time limits or at the same or different time intervals, in sequence, in separate entities, where these two or more active agents are administered to provide effective levels of prevention or treatment in patients. In some embodiments, the anti-CD3 antibody or fragments thereof and other therapeutic agents of the invention used in the pharmaceutical combination are administered at a level not exceeding the level when they are used alone. The term "fixed combination" means that two or more active agents are administered simultaneously to patients in the form of a single entity. It is preferred to select the dose and/or time interval of two or more active agents, so that the combined use of each component can produce greater effect than the single use of any one component in the treatment of disease or disorder. Each component can take its own form of preparation, which can be the same or different.

The term "combination therapy" refers to the application of two or more therapeutic agents or therapeutic modes (such as radiotherapy or surgery) to treat the diseases described herein. Such administration includes the co-administration of these therapeutic agents in a substantially simultaneous manner, such as in a single capsule with a fixed proportion of active ingredients. Alternatively, such application includes the joint application of each active ingredient in multiple or separate containers (such as tablets, capsules, powders and liquids). The powder and/or liquid can be reconstituted or diluted to the required dose before application. In addition, this application also includes the use of each type of therapeutic agent at approximately the same time or at different times in a sequential manner. In either case, the treatment plan will provide the beneficial effect of pharmaceutical combination in treating the disease or condition described herein.

As used herein, "treatment" (or "treat" or "treating") refers to slowing, interrupting, arresting, alleviating, stopping, reducing, or reversing the progression or severity of an existing symptom, disorder, condition, or disease.

As used herein, "prevention" (or "prevent" or "preventing") includes the inhibition of the onset or progression of a disease or disorder or a symptom of a particular disease or disorder. In some embodiments, subjects with family history of cancer are candidates for preventive regimens. Generally, in the context of cancer, the term "prevention" refers to the administration of a drug prior to the onset of signs or symptoms of a cancer, particularly in subjects at risk of cancer.

The term "vector" as used herein refers to a nucleic acid molecule capable of proliferating another nucleic acid to which it is linked. The term includes vectors that serve as self-replicating nucleic acid structures as well as vectors binding to the genome of a host cell into which they have been introduced. Some vectors are capable of directing the expression of a nucleic acid to which they are operably linked. Such vectors are called "expression vectors" herein.

"Subject/patient/individual sample" refers to a collection of cells or fluids obtained from a patient or subject. The source of the tissue or cell samples can be solid tissues, e.g., from fresh, frozen and/or preserved organ or tissue samples or biopsy samples or puncture samples; blood or any blood component; body fluids such as cerebrospinal fluids, amniotic fluids, peritoneal fluids, or interstitial fluids; cells from a subject at any time during pregnancy or development. Tissue samples may comprise compounds which are naturally not mixed with tissues, such as preservatives, anticoagulants, buffers, fixatives, nutrients, antibiotics, and the like.

## II. Antibodies

In some embodiments, the anti-CD3 antibody of the invention or antigen-binding fragment thereof binds to CD3 (such as human CD3 or cynomolgus monkey CD3) with the required affinity. In some embodiments, the anti-CD3 antibody of the invention or the antigen-binding fragment thereof can bind human CD3 and cynomolgus monkey CD3 both. In some embodiments, the affinity of the antibody is determined by Biolayer Interferometry or surface plasmon resonance.

In some embodiments, the anti-CD3 antibody of the invention binds to human CD3 or cynomolgus monkey CD3 with an equilibrium dissociation constants ($K_D$) between 0.5nM to 200nM, preferably between 1nM, 5nM, 10nM, 15nM, 20nM, 25nM, 30nM, 35nM, 40nM, 45nM or 50nM to 180nM, 190nM or 200nM, such as 100nM-200nM. In some embodiments, the anti-CD3 antibody of the invention binds to human CD3 E&G complex or human CD3E&D complex with $K_D$ between 10nM to 150nM, or 10nM-120nM or 10nM-100nM. In some embodiments, the anti-CD3 antibody of the invention binds to human CD3 or cynomolgus monkey CD3 with undetectable affinity.

In some embodiments, the antibody or the antigen-binding fragment thereof of the invention binds to CD3 on the surface of effector cells. In some embodiments, the antibody or the antigen-binding fragment thereof of the invention can activate effector cells. In some embodiments, the effector cells are T cells, such as T lymphocytes, or CD4+T cells or CD8+T cells. In some embodiments, the said binding is detected by flow cytometry.

In some embodiments, the antibody or the antigen-binding fragment of the invention can activate effector cells to induce the killing of tumor cells.

In some embodiments, the anti-CD3 antibody or the antigen-binding fragment thereof of the invention comprises three complementary determining regions from the heavy chain variable region (HCDRs), HCDR1, HCDR2 and HCDR3.

In some embodiments, the anti-CD3 antibody or the antigen-binding fragment thereof of the invention comprises three complementary determining regions from the light chain variable region (LCDRs), LCDR1, LCDR2 and LCDR3.

In some embodiments, the anti-CD3 antibody or the antigen-binding fragment thereof of the invention comprises three complementary determining regions (HCDRs) from the heavy chain variable region and three complementary determining regions from the light chain variable region (LCDRs).

In some aspects, the anti-CD3 antibody or the antigen-binding fragment thereof of the invention comprises a heavy chain variable region (VH). In some aspects, the anti-CD3 antibody or the antigen-binding fragment thereof of the invention comprises a light chain variable region (VL). In some aspects, the anti-CD3 antibody of the invention or the antigen-binding fragment thereof comprises a heavy chain variable region (VH) and a light chain variable region (VL). In some embodiments, the said heavy chain variable region comprises three complementary determining regions (CDRs) from the heavy chain variable region, HCDR1, HCDR2 and HCDR3. In some embodiments, the light chain variable region comprises three complementary determining regions (CDRs) from the light chain variable region, LCDR1, LCDR2 and LCDR3.

In some embodiments, the anti-CD3 antibody or the antigen-binding fragment thereof of the invention further comprises a constant region HC of the antibody heavy chain. In some embodiments, the anti-CD3 antibody or the antigen-binding fragment thereof of the invention further comprises a constant region LC of the antibody light chain. In some embodiments, the anti-CD3 antibody or the antigen-binding fragment thereof of the invention further comprises a heavy chain constant region HC and a light chain constant region LC.

In some embodiments, the heavy chain variable region of the invention

(i) comprises or consists of an amino acid sequence having at least 90%, 91%, 92%, 93%, 94%, 95%, 96%, 97%, 98% or 99% identity with the amino acid sequence selected from SEQ ID NO: 47-75; or

(ii) comprises or consists of an amino acid sequence selected from SEQ ID NO: 47-75; or

(iii) comprises or consists of an amino acid sequence having one or more (preferably no more than 10, more preferably no more than 5, 4, 3, 2, 1) amino acid changes (preferably amino acid substitution, more preferably amino acid conservative substitution), compared to the amino acid sequence selected from SEQ ID NO: 47-75, preferably, the said amino acid changes do not occur in the CDR region.

In some embodiments, the light chain variable region of the invention

(i) comprises or consists of an amino acid sequence having at least 90%, 91%, 92%, 93%, 94%, 95%, 96%, 97%, 98% or 99% identity with the amino acid sequence selected from SEQ ID NO: 76-99; or

(ii) comprises or consists of an amino acid sequence selected from SEQ ID NO: 76-99; or

(iii) comprises or consists of an amino acid sequence having one or more (preferably no more than 10, more preferably no more than 5, 4, 3, 2, 1) amino acid changes (preferably amino acid substitution, more preferably amino acid conservative substitution), compared to the amino acid sequence selected from SEQ ID NO: 76-99, preferably, the said amino acid changes do not occur in the CDR region.

In some embodiments, the three complementary determining regions (HCDRs) from the heavy chain variable region of the invention, HCDR1, HCDR2 and HCDR3 are selected from

(i) the three complementary determining regions HCDR1, HCDR2 and HCDR3 contained in VH as shown in anyone of SEQ ID NO: 50-75, or

(ii) a sequence wherein said three HCDR regions comprise at least one but no more than 5, 4, 3, 2 or 1 amino acid change (preferably amino acid substitution, preferably conservative substitution) compared to anyone sequence in (i).

In some embodiments, the three complementary determining regions (LCDRs) from the light chain variable region of the invention, LCDR1, LCDR2 and LCDR3 are selected from

(i) the three complementary determining regions LCDR1, LCDR2 and LCDR3 contained in VL as shown in anyone of SEQ ID NO: 85-99, or

(ii) a sequence wherein said three LCDR regions comprise at least one but no more than 5, 4, 3, 2 or 1 amino acid change (preferably amino acid substitution, preferably conservative substitution) compared to any sequence in (i).

In some embodiments, HCDR1 comprises or consists of an amino acid sequence of SEQ ID NO: 1, 4, 5, 6 or 22, or HCDR1 comprises an amino acid sequence with one, two or three changes (preferably amino acid substitution, preferably conservative substitution) compared to the amino acid sequence of SEQ ID NO: 1, 4, 5, 6 or 22.

In some embodiments, HCDR1 of the invention comprises or consists of an amino acid sequence of SEQ ID NO: 103, wherein the amino acid sequence of SEQ ID NO: 103 is as follows:

GFTFX$_1$X$_2$X$_3$AMN (SEQ ID NO: 103), wherein

X$_1$ is selected from N, G, S, D or E, preferably N, G or S;

X$_2$ is selected from T, G, L or R, preferably T or G;

X$_3$ is selected from Y, G, A or S, preferably Y, A or S,

and SEQ ID NO: 103 is different from SEQ ID NO: 1 at 1, 2 or 3 amino acids.

In some embodiments, HCDR2 comprises or consists of an amino acid sequence of SEQ ID NO: 2, 7, 9, 10, 11, 12, 23 or 24, or HCDR2 comprises an amino acid sequence with one, two or three changes (preferably amino acid substitution, preferably conservative substitution) compared to the amino acid sequence of SEQ ID NO: 2, 7, 9, 10, 11, 12, 23 or 24.

In some embodiments, HCDR2 of the invention comprises or consists of an amino acid sequence of SEQ ID NO: 104, wherein the amino acid sequence of SEQ ID NO: 104 is as follows:

RIX$_1$X$_2$KX$_3$X$_4$X$_5$YATYYADSVKD (SEQ ID NO:104), wherein

X$_1$ is selected from R, G, A, or S, preferably R or S;

X$_2$ is selected from S, G, L or R, preferably S or L;

X$_3$ is selected from Y, G, A or S, preferably Y or A;

X$_4$ is selected from N, G, S, D or E, preferably N or G;

X$_5$ is selected from N, G, S, D or E, preferably N or G;

and SEQ ID NO: 104 is different from SEQ ID NO: 2 at 1, 2 or 3 amino acids.

In some embodiments, HCDR3 comprises or consists of an amino acid sequence of anyone of SEQ ID NO: 3, 8, 13-21, 25-28, or HCDR3 comprises an amino acid sequence with one, two or three changes (preferably amino acid substitution, preferably conservative substitution) compared to the amino acid sequence of anyone of SEQ ID NO: 3, 8, 13-21, 25-28.

In some embodiments, HCDR3 of the invention comprises or consists of an amino acid sequence of SEQ ID NO: 105, wherein the amino acid sequence of SEQ ID NO: 105 is as follows:

X$_1$X$_2$X$_3$X$_4$X$_5$X$_6$X$_7$X$_8$X$_9$SWFAY (SEQ ID NO: 105), wherein

X$_1$ is selected from H, G, A or S, preferably H or A;

X$_2$ is GorY;

X$_3$ is selected from N, G, S, D or E, preferably N or G;

X$_4$ is selected from F, G, A or S, preferably F or A;

$X_5$ is GorY;

$X_6$ is selected from N, G, S, D or E, preferably N, Q or G;

$X_7$ is selected from S, G, L or R, preferably S or R;

$X_8$ is selected from Y, G, A or S, preferably Y or A;

$X_9$ is selected from V or A;

and SEQ ID NO: 105 is different from SEQ ID NO: 3 at 1, 2 or 3 amino acids.

In some embodiments, LCDR1 comprises or consists of an amino acid sequence of anyone of SEQ ID NO: 29, 32-36, 41 and 42, or LCDR1 comprises an amino acid sequence with one, two or three changes (preferably amino acid substitution, preferably conservative substitution) compared to the amino acid sequence of anyone of SEQ ID NO: 29, 32-36, 41 and 42.

In some embodiments, LCDR1 of the invention comprises or consists of an amino acid sequence of SEQ ID NO: 106, wherein the amino acid sequence of SEQ ID NO: 106 is as follows:

$X_1$SSTGAV $X_2X_3X_4$YAN (SEQ ID NO:106), wherein

$X_1$ is selected from R, G, A or S, preferably R or G;

$X_2$ is selected from T, G, L or R, preferably T or G;

$X_3$ is selected from T, G, L or R, preferably T or G;

$X_4$ is selected from S, G, L or R, preferably S or R;

and SEQ ID NO: 106 is different from SEQ ID NO: 29 at 1, 2 or 3 amino acids.

In some embodiments, LCDR2 comprises or consists of an amino acid sequence of anyone of SEQ ID NO: 30 or 45, or LCDR2 comprises an amino acid sequence with one, two or three changes (preferably amino acid substitution, preferably conservative substitution) compared to the amino acid sequence of anyone of SEQ ID NO: 30 or 45.

In some embodiments, LCDR3 comprises or consists of an amino acid sequence of anyone of SEQ ID NO: 31, 3 7, 38, 39, 40, 43, 44 or 46, or LCDR3 comprises an amino acid sequence with one, two or three changes (preferably amino acid substitution, preferably conservative substitution) compared to the amino acid sequence of anyone of SEQ ID NO: 31, 37, 38, 39, 40, 43, 44 or 46.

In some embodiments, LCDR3 of the invention comprises or consists of an amino acid sequence of SEQ ID NO: 107, wherein the amino acid sequence of SEQ ID NO: 107 is as follows:

AL$X_1$ $X_2X_3X_4$LWV (SEQ ID NO:107), wherein

$X_1$ is selected from W, G, A or S, preferably W or A;

$X_2$ is selected from Y, G, A or S, preferably Y or A;

$X_3$ is selected from S, G, L or R, preferably S or R;

$X_4$ is selected from N, G, S, D or E, preferably N, G or D;

and SEQ ID NO: 107 is different from SEQ ID NO: 31 at 1, 2 or 3 amino acids.

In some embodiments, the heavy chain constant region HC of the antibody of invention is that of IgG1 or IgG2 or IgG3 or IgG4, preferably of IgG1, such as a IgG1 constant region with LALA mutation. In some embodiments, the light chain constant region LC of the antibody of the invention is Lambda or Kappa light chain constant region, preferably Lambda light chain constant region.

In some preferred embodiments, the heavy chain constant region HC of the antibody of invention

(i) comprises or consists of an amino acid sequence having at least 85%, 90%, 91%, 92%, 93%, 94%, 95%, 96%, 97%, 98% or 99% identity with the amino acid sequence selected from SEQ ID NO: 100;

(ii) comprises or consists of an amino acid sequence selected from SEQ ID NO: 100; or

(iii) comprises or consists of an amino acid sequence having one or more (preferably no more than 20 or 10, more preferably no more than 5, 4, 3, 2, 1) amino acid changes (preferably amino acid substitution, more preferably amino acid conservative substitution), compared to the amino acid sequence selected from SEQ ID NO: 100.

In some embodiments, the light chain constant region LC of the antibody of invention

(i) comprises or consists of an amino acid sequence having at least 85%, 90%, 91%, 92%, 93%, 94%, 95%, 96%, 97%, 98% or 99% identity with the amino acid sequence selected from SEQ ID NO: 101 or 102;

(ii) comprises or consists of an amino acid sequence selected from SEQ ID NO: 101 or 102; or

(iii) comprises or consists of an amino acid sequence having one or more (preferably no more than 20 or 10, more preferably no more than 5, 4, 3, 2, 1) amino acid changes (preferably amino acid substitution, more preferably amino acid conservative substitution), compared to the amino acid sequence selected from SEQ ID NO: 101 or 102.

In some specific embodiments of the invention, the anti-CD3 antibody or antigen-binding fragment thereof of the invention comprises:

(i) A heavy chain variable region VH, which comprises or consists of an amino acid sequence of anyone of SEQ D NO: 47-51; and

(ii) A light chain variable region VL, which comprises or consists of an amino acid sequence of any item of SEQ ID NO: 76-84.

In some specific embodiments of the invention, the anti-CD3 antibody or antigen-binding fragment thereof of the invention comprises:

(i) VH comprising or consisting of an amino acid sequence shown in SEQ ID NO: 47 or an amino acid sequence having at least 90%, 91%, 92%, 93%, 94%, 95%, 96%, 97%, 98% or 99% identity with SEQ ID NO: 47, and VL comprising or consisting of an amino acid sequence shown in SEQ ID NO: 76 or an amino acid sequence having at least 90%, 91%, 92%, 93%, 94%, 95%, 96%, 97%, 98% or 99% identity with SEQ ID NO: 76;

(ii) VH comprising or consisting of an amino acid sequence shown in SEQ ID NO: 48 or an amino acid sequence having at least 90%, 91%, 92%, 93%, 94%, 95%, 96%, 97%, 98% or 99% identity with SEQ ID NO: 48, and VL comprising or consisting of an amino acid sequence shown in anyone of SEQ ID NO: 77-84 or an amino acid sequence having at least 90%, 91%, 92%, 93%, 94%, 95%, 96%, 97%, 98% or 99% identity with anyone of SEQ ID NO: 77-84;

(iii) VH comprising or consisting of an amino acid sequence shown in SEQ ID NO: 49 or an amino acid sequence having at least 90%, 91%, 92%, 93%, 94%, 95%, 96%, 97%, 98% or 99% identity with SEQ ID NO: 49, and VL comprising or consisting of an amino acid sequence shown in anyone of SEQ ID NO: 77-84 or an amino acid sequence having at least 90%, 91%, 92%, 93%, 94%, 95%, 96%, 97%, 98% or 99% identity with anyone of SEQ ID NO: 77-84;

(iv) VH comprising or consisting of an amino acid sequence shown in SEQ ID NO: 50 or an amino acid sequence having at least 90% identity with SEQ ID NO: 50, and VL comprising or consisting of an amino acid sequence shown in anyone of SEQ ID NO: 77-84 or an amino acid sequence having at least 90%, 91%, 92%, 93%, 94%, 95%, 96%, 97%, 98% or 99% identity with anyone of SEQ ID NO: 77-84;

(v) VH comprising or consisting of an amino acid sequence shown in SEQ ID NO: 51 or an amino acid sequence having at least 90% identity with SEQ ID NO: 51, and VL comprising or consisting of an amino acid sequence shown in anyone of SEQ ID NO: 77-84 or an amino acid sequence having at least 90%, 91%, 92%, 93%, 94%, 95%, 96%, 97%, 98% or 99% identity with anyone of SEQ ID NO: 77-84.

In some specific embodiments of the invention, the anti-CD3 antibody or antigen-binding fragment thereof of the invention comprises:

(i) a heavy chain variable region VH, which comprises or consists of the following amino acid sequence: compared to an amino acid sequence of anyone of SEQ ID NO: 47-51, said amino acid sequence has the mutations selected from the following at 1, 2 or 3 positions of H31, H32, H33, H52, H52A, H52C, H53, H54, H95, H96, H97, H98, H99, H100, H100A, H100B, H100C (Kabat number) according to the Kabat numbering:
amino acid Y, W or F is mutated into amino acid G, A, S; amino acid R, K or H is mutated into amino acid G, A or S; amino acid G is mutated to amino acid Y; amino acid N or Q is mutated into amino acid G, S, D or E; and/or amino acid T or S is mutated into G, L, R;

(ii) a light chain variable region VL, which comprises or consists of the following amino acid sequence: compared to an amino acid sequence of anyone of SEQ ID NO: 76-84, said amino acid sequence has the mutations selected from the following at 1, 2 or 3 positions of L24, L28, L29, L30, L31, L53, L91, L92, L93, L94 (Kabat number) according to the Kabat numbering:
amino acid Y, W or F is mutated into amino acid G, A, S; amino acid R, K or H is mutated into amino acid G, A or S; amino acid G is mutated to amino acid Y; amino acid N or Q is mutated into amino acid G, S, D or E; and/or amino acid T or S is mutated into G, L, R.

In some specific embodiments of the invention, the anti-CD3 antibody or antigen-binding fragment thereof of the invention comprises:

(i) a heavy chain variable region VH, which comprises or consists of the following amino acid sequence: an amino acid sequence of SEQ ID NO:50 with the mutations selected from the following at 1, 2 or 3 positions of H31, H32, H33, H52, H52A, H52C, H53, H54, H95, H96, H97, H98, H99, H100, H100A, H100B, H100C (Kabat number) according to the Kabat numbering:
amino acid Y, W or F is mutated into amino acid G, A, S; amino acid R, K or H is mutated into amino acid G, A or S; amino acid G is mutated to amino acid Y; amino acid N or Q is mutated into amino acid G, S, D or E; and/or amino acid T or S is mutated into G, L, R;

(ii) a light chain variable region VL, which comprises or consists of the following amino acid sequence: an amino acid sequence of SEQ ID NO: 80 with the mutations selected from the following at 1, 2 or 3 positions of L24, L28, L29, L30, L31, L53, L91, L92, L93, L94 (Kabat number) according to the Kabat numbering:
amino acid Y, W or F is mutated into amino acid G, A, S; amino acid R, K or H is mutated into amino acid G, A or S; amino acid G is mutated to amino acid Y; amino acid N or Q is mutated into amino acid G, S, D or E; and/or amino acid T or S is mutated into G, L, R.

In some specific embodiments of the invention, the anti-CD3 antibody or antigen-binding fragment thereof of the invention comprises:

(i) three complementary determining regions HCDR1, HCDR2 and HCDR3 contained in VH as shown in anyone of SEQ ID NO: 52-75, and three complementary determining regions LCDR1, LCDR2 and LCDR3 contained in VL as shown in SEQ ID NO: 80;

(ii) three complementary determining regions HCDR1, HCDR2 and HCDR3 contained in VH as shown in SEQ ID NO: 50, and three complementary determining regions LCDR1, LCDR2 and LCDR3 contained in VL as shown in anyone of SEQ ID NO: 85-99.

In some specific embodiments of the invention, the anti-CD3 antibody or antigen-binding fragment thereof of the invention comprises:

(1) HCDR1 as shown in anyone of SEQ ID NO: 4-6, HCDR2 as shown in SEQ ID NO: 2, and HCDR3 as shown in SEQ ID NO: 8; LCDR1 as shown in SEQ ID NO: 29, LCDR2 as shown in SEQ ID NO: 30 and LCDR3 as shown in SEQ ID NO: 31;

(2) HCDR1 as shown in SEQ ID NO: 1, HCDR2 as shown in anyone of SEQ ID NO: 7, 9, 10, 11 or 12, and HCDR3 as shown in SEQ ID NO: 8; LCDR1 as shown in SEQ ID NO: 29, LCDR2 as shown in SEQ ID NO: 30 and LCDR3 as shown in SEQ ID NO: 31;

(3) HCDR1 as shown in SEQ ID NO: 1, HCDR2 as shown in SEQ ID NO: 2, and HCDR3 as shown in anyone of SEQ ID NO: 13-21; LCDR1 as shown in SEQ ID NO: 29, LCDR2 as shown in SEQ ID NO: 30 and LCDR3 as shown in SEQ ID NO: 31;

(4) HCDR1 as shown in SEQ ID NO: 1, HCDR2 as shown in SEQ ID NO: 23 or 24, and HCDR3 as shown in SEQ ID NO: 8; LCDR1 as shown in SEQ ID NO: 29, LCDR2 as shown in SEQ ID NO: 30 and LCDR3 as shown in SEQ ID NO: 31;

(5) HCDR1 as shown in SEQ ID NO: 22, HCDR2 as shown in SEQ ID NO: 2, and HCDR3 as shown in SEQ ID NO: 8; LCDR1 as shown in SEQ ID NO: 29, LCDR2 as shown in SEQ ID NO: 30 and LCDR3 as shown in SEQ ID NO: 31;

(6) HCDR1 as shown in SEQ ID NO: 1, HCDR2 as shown in SEQ ID NO: 2, and HCDR3 as shown in anyone of SEQ ID NO: 25-28; LCDR1 as shown in SEQ ID NO: 29, LCDR2 as shown in SEQ ID NO: 30 and LCDR3 as shown in SEQ ID NO: 31;

(7) HCDR1 as shown in SEQ ID NO: 1, HCDR2 as shown in SEQ ID NO: 2, and HCDR3 as shown in SEQ ID NO: 8; LCDR1 as shown in anyone of SEQ ID NO: 32-36, 41 and 42, LCDR2 as shown in SEQ ID NO: 30 and LCDR3 as shown in SEQ ID NO: 31;

(8) HCDR1 as shown in SEQ ID NO: 1, HCDR2 as shown in SEQ ID NO: 2, and HCDR3 as shown in SEQ ID NO: 8; LCDR1 as shown in SEQ ID NO: 29, LCDR2 as shown in SEQ ID NO: 30 and LCDR3 as shown in anyone of SEQ ID NO: 37-40, 43 and 44; or

(9) HCDR1 as shown in SEQ ID NO: 1, HCDR2 as shown in SEQ ID NO: 2, and HCDR3 as shown in SEQ ID NO: 8; LCDR1 as shown in SEQ ID NO: 29, LCDR2 as shown in SEQ ID NO: 45 and LCDR3 as shown in SEQ ID NO: 31 or 46.

In some specific embodiments of the invention, the anti-CD3 antibody or antigen-binding fragment thereof of the invention comprises:

(1) HCDR1 as shown in SEQ ID NO: 103, HCDR2 as shown in SEQ ID NO: 104, and HCDR3 as shown in SEQ ID NO: 105; LCDR1 as shown in SEQ ID NO: 29, LCDR2 as shown in SEQ ID NO: 30 and LCDR3 as shown in SEQ ID NO: 31;

(2) HCDR1 as shown in SEQ ID NO: 103, HCDR2 as shown in SEQ ID NO: 2, and HCDR3 as shown in SEQ ID NO: 3 or 8; LCDR1 as shown in SEQ ID NO: 29, LCDR2 as shown in SEQ ID NO: 30 and LCDR3 as shown in SEQ ID NO: 31;

(3) HCDR1 as shown in SEQ ID NO: 1, HCDR2 as shown in SEQ ID NO: 104, and HCDR3 as shown in SEQ ID NO: 3 or 8; LCDR1 as shown in SEQ ID NO: 29, LCDR2 as shown in SEQ ID NO: 30 and LCDR3 as shown in SEQ ID NO: 31;

(4) HCDR1 as shown in SEQ ID NO: 1, HCDR2 as shown in SEQ ID NO: 2, and HCDR3 as shown in SEQ ID NO: 105; LCDR1 as shown in SEQ ID NO: 29, LCDR2 as shown in SEQ ID NO: 30 and LCDR3 as shown in SEQ ID NO: 31;

(5) HCDR1 as shown in SEQ ID NO: 103, HCDR2 as shown in SEQ ID NO: 104, and HCDR3 as shown in SEQ ID NO: 3 or 8; LCDR1 as shown in SEQ ID NO: 29, LCDR2 as shown in SEQ ID NO: 30 and LCDR3 as shown in SEQ ID NO: 31;

(6) HCDR1 as shown in SEQ ID NO: 103, HCDR2 as shown in SEQ ID NO: 2, and HCDR3 as shown in SEQ ID NO: 105; LCDR1 as shown in SEQ ID NO: 29, LCDR2 as shown in SEQ ID NO: 30 and LCDR3 as shown in SEQ ID NO: 31;

(7) HCDR1 as shown in SEQ ID NO: 1, HCDR2 as shown in SEQ ID NO: 104, and HCDR3 as shown in SEQ ID NO: 105; LCDR1 as shown in SEQ ID NO: 29, LCDR2 as shown in SEQ ID NO: 30 and LCDR3 as shown in SEQ ID NO: 31;

(8) HCDR1 as shown in SEQ ID NO: 1, HCDR2 as shown in SEQ ID NO: 2, and HCDR3 as shown in SEQ ID NO:

3 or 8; LCDR1 as shown in SEQ ID NO: 106, LCDR2 as shown in SEQ ID NO: 30 or 45 and LCDR3 as shown in SEQ ID NO: 107;

(9) HCDR1 as shown in SEQ ID NO: 1, HCDR2 as shown in SEQ ID NO: 2, and HCDR3 as shown in SEQ ID NO: 3 or 8; LCDR1 as shown in SEQ ID NO: 106, LCDR2 as shown in SEQ ID NO: 30 or 45 and LCDR3 as shown in SEQ ID NO: 31;

(10) HCDR1 as shown in SEQ ID NO: 1, HCDR2 as shown in SEQ ID NO: 2, and HCDR3 as shown in SEQ ID NO: 3 or 8; LCDR1 as shown in SEQ ID NO: 29, LCDR2 as shown in SEQ ID NO: 30 or 45 and LCDR3 as shown in SEQ ID NO: 107;

(11) HCDR1 as shown in SEQ ID NO: 103, HCDR2 as shown in SEQ ID NO: 104, and HCDR3 as shown in SEQ ID NO: 105; LCDR1 as shown in SEQ ID NO: 106, LCDR2 as shown in SEQ ID NO: 30 or 45 and LCDR3 as shown in SEQ ID NO: 107.

In some specific embodiments of the invention, the anti-CD3 antibody or antigen-binding fragment thereof of the invention comprises:

(i) VH comprising or consisting of an amino acid sequence shown in anyone of SEQ ID NO: 52-75 or an amino acid sequence having at least 90% identity with anyone of SEQ ID NO: 52-75, and VL comprising or consisting of an amino acid sequence shown in SEQ ID NO: 80 or an amino acid sequence having at least 90%, 91%, 92%, 93%, 94%, 95%, 96%, 97%, 98% or 99% identity with SEQ ID NO: 80;

(vii) VH comprising or consisting of an amino acid sequence shown in SEQ ID NO: 50 or an amino acid sequence having at least 90% identity with SEQ ID NO: 50, and VL comprising or consisting of an amino acid sequence shown in anyone of SEQ ID NO: 85-99 or an amino acid sequence having at least 90%, 91%, 92%, 93%, 94%, 95%, 96%, 97%, 98% or 99% identity with anyone of SEQ ID NO: 85-99.

In one embodiment of the invention, the amino acid change described herein includes amino acid substitution, insertion, or deletion.

In a preferred embodiment, the amino acid change described herein occurs in CDR region, such that the affinity of the antibody of the invention to CD3 can be adjusted to the required degree through the amino acid change in the CDR region, especially the degree required to construct a multispecific antibody. In some embodiments, the number of amino acid change in each CDR is not more than 3, 2 or 1. In some embodiments, the number of amino acid changes in the combination of heavy chain HCDRs is no more than 3, 2 or 1. In some embodiments, the number of amino acid changes in the combination of light chain HCDRs is no more than 3, 2 or 1. In some embodiments, the amino acid position of the above amino acid change is selected from one or more (preferably no more than 6, more preferably no more than 3 in heavy chain CDR combination, and/or no more than 3 in light chain CDR combination) of the heavy chain H31, H32, H33, H52, H52A, H52C, H53, H54, H95, H96, H97, H98, H99, H100, H100A, H100B, H100C (Kabat numbering) and light chain L24, L28, L29, L30, L31, L53, L91, L92, L93, L94 (Kabat numbering). In the preferred embodiment, the amino acid change is an amino acid substitution, wherein the aromatic amino acids Y, W, and/or F are mutated into amino acids G, A, and/or S with relatively small side chains; the positively charged amino acids R, K, and/or H are mutated into amino acids G, A, and/or S with relatively small side chains; the amino acid G with hydrogen atom in the side chain is mutated into aromatic amino acid Y; the amino acids N and/or Q containing amide groups in the side chain are mutated into amino acids G, S, D, and/or E; non-aromatic amino acids T and/or S containing hydroxyl in the side chain are mutated into G, L and R.

In a preferred embodiment, the amino acid change described in the invention occurs in a region outside the CDR (for example, in FR). More preferably, the amino acid change described in the invention occurs in the region outside the heavy chain variable region and/or the light chain variable region. Preferably, the amino acid change described herein is amino acid substitution, preferably conservative substitution.

In some embodiments, the substitution is a conservative substitution. The conservative substitution refers to the replacement of an amino acid by another amino acid in the same category. For example, one acidic amino acid is replaced by another acidic amino acid, one basic amino acid is replaced by another basic amino acid, or one neutral amino acid is replaced by another neutral amino acid. Exemplary substitution is shown in the following table:

| Original residue | Exemplary substitution | Preferred conservative amino acid substitution |
| --- | --- | --- |
| Ala (A) | Val, Leu, Ile | Val |

(continued)

| Original residue | Exemplary substitution | Preferred conservative amino acid substitution |
|---|---|---|
| Arg (R) | Lys, Gln, Asn | Lys |
| Asn (N) | Gln, His, Asp, Lys, Arg | Gln |
| Asp (D) | Glu, Asn | Glu |
| Cys (C) | Ser, Ala | Ser |
| Gln (Q) | Asn, Glu | Asn |
| Glu (E) | Asp, Gln | Asp |
| Gly (G) | Ala | Ala |
| His (H) | Asn, Gln, Lys, Arg | Arg |
| Ile (I) | Leu, Val, Met, Ala, Phe, N-leucine | Leu |
| Leu (L) | N-leucine, Ile, Val, Met, Ala, Phe | Ile |
| Lys (K) | Arg, Gln, Asn | Arg |
| Met (M) | Leu, Phe, Ile | Leu |
| Phe (F) | Trp, Leu, Val, Ile, Ala, Tyr | Tyr |
| Pro (P) | Ala | Ala |
| Ser (S) | Thr | Thr |
| Thr (T) | Val, Ser | Ser |
| Trp (W) | Tyr, Phe | Tyr |
| Tyr (Y) | Trp, Phe, Thr, Ser | Phe |
| Val (V) | Ile, Leu, Met, Phe, Ala, N-leucine | Leu |

In some embodiments, the substitution occurs in the CDR region of the antibody. Generally, the obtained variant has modification (for example, improvement) and/or will have some biological characteristics that are basically retained by the parent antibody in terms of some biological characteristics (for example, increased affinity) relative to the parent antibody. An example substitution variant is an affinity mature antibody.

In some embodiments, the antibody provided herein is modified to increase or decrease the degree of glycosylation of the antibody. Addition or deletion of glycosylation sites of antibodies can be easily realized by changing the amino acid sequence to produce or remove one or more glycosylation sites. When an antibody comprises Fc region, the carbohydrate attached to it can be modified. In some applications, the modification to remove an unwanted glycosylation site may be useful, such as removing the fucose motif to improve the function of antibody-dependent cytotoxicity (ADCC) (see Shield et al. (2002) JBC277:26733). In other applications, the modification of galactosylation can be used to modify complement dependent cytotoxicity (CDC).

In some embodiments, one or more amino acid modifications can be introduced into the Fc region of the antibody provided herein to produce Fc region variants to change one or more functional characteristics of the antibody, such as serum half-life, complement binding, complement dependent cytotoxicity, Fc receptor binding, and/or antibody dependent cytotoxicity. Fc region variants may include human Fc region sequences (such as human IgG1, IgG2, IgG3, or IgG4 Fc region) that comprise amino acid changes (such as substitutions) at one or more amino acid positions.

In one embodiment of the invention, the antibody described herein introduces changes into Fc region to improve the ADCC activity or CDC activity of the antibody.

In some embodiments, it may be necessary to produce antibodies modified by cysteine engineering, such as "thioMAb", in which one or more residues of the antibody are substituted by cysteine residues.

In some embodiments, the antibody provided herein may be further modified to comprise other non-protein components known in the art and readily available. Parts suitable for antibody derivatization include, but are not limited to, water-soluble polymers. Non-limiting examples of water-soluble polymers include, but are not limited to, polyethylene glycol (PEG), ethylene glycol/propylene glycol copolymer, carboxymethyl cellulose, dextran, polyvinyl alcohol, polyvinylpyrrolidone, poly-1,3-diane, poly-1,3,6-triane, ethylene/maleic anhydride copolymer, polyamino acid (homopolymer or random copolymer), and dextran or poly(n-vinylpyrrolidone)polyethylene glycol, propylene glycol homopolymer, polyethylene oxide/ethylene oxide copolymer, polyoxyethylated polyol (such as glycerin), polyvinyl alcohol, and mixtures thereof.

In some embodiments, the anti-CD3 antibody or antigen-binding fragment thereof of the invention has one or more of the following characteristics:

(i) displaying the same or similar binding affinity and/or specificity as the antibody of the invention to CD3;

(ii) inhibiting (for example, competitively inhibiting) the binding of the antibody of the invention to CD3;

(iii) binding to the same or overlapping epitopes as the antibody of the invention;

(iv) competing with the antibody of the invention to bind CD3;

(v) having one or more biological characteristics of the antibody of the invention.

In some embodiments, the anti-CD3 antibody of the invention is an antibody in an IgG1 form or an antibody in IgG2 form or an antibody in IgG3 form or an antibody in IgG4 form, preferably an antibody in an IgG1 form.

In some embodiments, the anti-CD3 antibody is a monoclonal antibody.

In some embodiments, the anti-CD3 antibody is humanized.

In some embodiments, at least part of the frame sequences of anti-CD3 antibody is human consensus frame sequences.

In one embodiment, the anti-CD3 antibody of the invention also covers its antibody fragments (such as antigen-binding fragments), preferably antibody fragment selected from the following antibody fragments: Fab, Fab', Fab' - SH, Fv, single-chain antibody (such as scFv), (Fab')$_2$, single-domain antibody such as VHH, dAb (domain antibody) or linear antibody.

In one embodiment, the antibody fragment of the present invention is scFv, comprising VH and VL described herein, and the linker sequence, wherein the linker is (GGGGS)$_n$, where n=1, 2, 3, 4 or 5, for example, n=4.

**III. Multispecific antibodies**

The CD3 antibody described in the invention covers a multispecific antibody that binds to CD3 and one or more other antigens or targets, such as a bispecific antibody or a trispecific antibody. In one embodiment, the multispecific antibody comprises the first antigen-binding domain that specifically binds to CD3, the second antigen-binding domain that specifically binds to another antigen, and optionally a third or more antigen-binding domains that specifically bind to other antigens.

In some embodiments, the other antigens are tumor related antigens. In some embodiments, the tumor associated antigen is selected from HER2 or CD70 or CLAUDIN18.2.

In one embodiment, the antibody of the invention is a bispecific antibody, which comprises a first antigen-binding region that specifically bind to CD3 and a second antigen-binding region. In some embodiments, the second antigen-binding region binds to tumor-related antigens. In some embodiments, the tumor associated antigen is HER2 or CD70 or CLAUDIN18.2.

In some embodiments, the first antigen-binding region that specifically binds CD3 comprises VH and/or VL as described above. In some embodiments, the first antigen-binding region specifically binding to CD3 comprises HCDR1, HCDR2 and HCDR3, and/or LCDR1, LCDR2 and LCDR3 as described above.

In some embodiments, the multi-specific antibody of the invention further comprises a heavy chain constant region. In some embodiments, the multi-specific antibody of the invention further comprises a light chain constant region. In some embodiments, the multi-specific antibody of the invention further comprises a heavy chain constant region and a light chain constant region. In some embodiments, the heavy chain constant region is selected from the heavy chain constant region described above. In some embodiments, the light chain constant region is selected from the light chain constant region described above. In some embodiments, the light chain constant region in the multi-specific antibody of the invention comprises or consists of an amino acid sequence of SEQ ID NO: 116, or an amino acid sequence having at least 90%, 91%, 92%, 93%, 94%, 95%, 96%, 97%, 98% or 99% identity with SEQ ID NO: 116. In some embodiments, the heavy chain constant region in the multi-specific antibody of the invention comprises or consists of an amino acid sequence of SEQ ID NO: 117, SEQ ID NO: 118, SEQ ID NO: 119 or SEQ ID NO: 120, or an amino acid sequence having at least 90%, 91%, 92%, 93%, 94%, 95%, 96%, 97%, 98% or 99% identity with them. In some embodiments, the heavy chain constant region binding to different antigen-binding regions may be the same or different. In some embodiments, the light chain constant region binding to different antigen-binding regions may be the same or different.

Any version or technology of a multispecific antibody can be used to prepare the multispecific antibody of the invention. For example, an antibody or fragment thereof with the first antigen-binding specificity can be functionally joined(for example, through chemical coupling, genetic fusion, non-covalent association or other ways) with one or more other molecular entities such as another antibody or antibody fragment or other antibodies or antibody fragments with another antigen-binding specificity or other antigen-binding specificities to produce multispecific antigen binding molecules.

In some embodiments, the forms of the bispecific antibody of the invention include IgG-like and non-IgG-like antibodies (Fan et al. (2015) Journal of Hematology & Oncology 8: 130). The most common IgG-like antibody form comprises two Fab regions and one Fc region. The heavy chain and light chain of each Fab can come from separate monoclonal antibodies. Non-IgG-like bispecific antibodies lack Fc region and each antigen or target binding domain thereof can be a Fab, or a single chain variable fragment (scFv), or a fusion protein that simulates a variable domain of two antibodies. The different binding domains are joined together by peptide connector, chemical coupling, non-covalent bond connection or other ways.

Specific exemplary bispecific forms that can be used in the context of the present invention include but are not limited to bispecific antibodies based on platforms such as TrioMab, CrossMab/KiH, KiH, DVD-Ig, IgG-scFv, FIT-Ig, mAb-Trap, BiTE, DART, TandAb, ImmTAC, TriKE, etc.

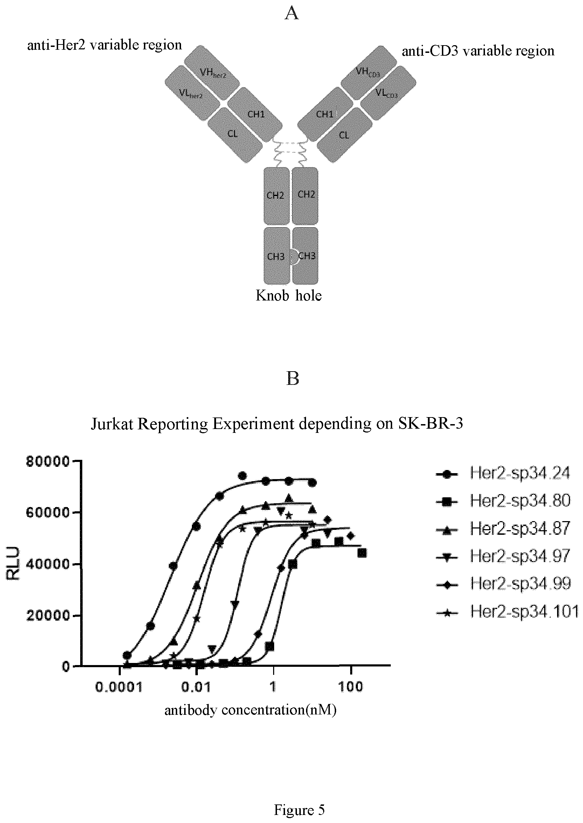

In some embodiments, the bispecific antibody of the invention is a bispecific antibody in the form of KiH. In some embodiments, the bispecific antibody of the invention comprises two Fabs and one Fc, wherein the first Fab comprises the first antigen-binding region that specifically binds to CD3, and the second Fab comprises the second antigen-binding region that specifically binds to a tumor-associated antigen, for example, in the form shown in Figure 5A.

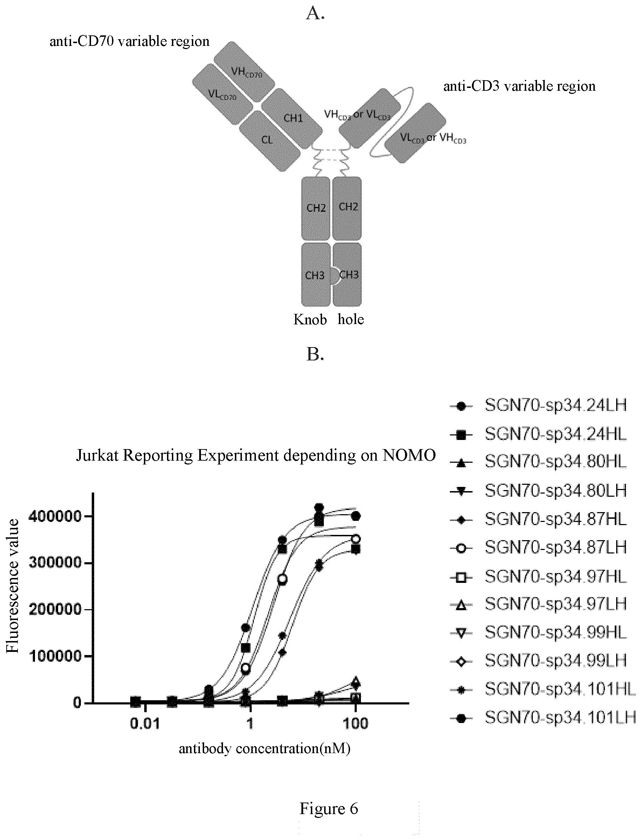

In another embodiment, the bispecific antibody of the invention comprises one Fab, one Fc, and one scFv, wherein Fab or scFv comprises a second antigen-binding region that specifically binds to a tumor-associated antigen, and scFv or Fab comprises a first antigen-binding region that specifically binds to CD3, for example, in the form shown in Figure 6A. In some embodiments, Fab comprises a second antigen-binding region that specifically binds to tumor-associated antigen, and scFv comprises a first antigen-binding region that specifically binds to CD3. In some embodiments, scFv comprises a second antigen-binding region that specifically binds to tumor associated-antigen, and Fab comprises a first antigen-binding region that specifically binds to CD3. In some embodiments, scFv can comprise VH-linker-VL or VL-linker-VH. In some embodiments, scFv is connected with Fc via VH to form a bispecific antibody. In some embodiments, scFv is connected with Fc via VL to form a bispecific antibody.

In some embodiments, the linker is a peptide linker. The peptide linker comprises a glycine-serine polymer, including, for example, (GS)n, (GSGGS)n, (GGGGS)n, (GGGS)n and (GGGGS)nG, wherein n is an integer of at least 1 (and preferably 2, 3, 4, 5, 6, 7, 8, 9, 10). Useful peptide linkers further include glycine-alanine polymer, alanine-serine polymer and other flexible connectors. In some embodiments, the linker is $(GGGGS)_4$.

In some embodiments, Fc is from IgG1 LALA sequence. In some embodiments, CL comprises or consists of the amino acid sequence of SEQ ID NO: 116.

In some embodiments, the tumor-associated antigen is HER2. In some embodiments, the second antigen-binding region that specifically binds to HER2 is from trastuzumab.

In some embodiments, the tumor-associated antigen is CD70. In some embodiments, the second antigen-binding region that specifically binds to CD70 is from SGN70 of WO2004073656.

In some embodiments, the tumor-associated antigen is CLAUDIN18.2. In some embodiments, the second antigen-binding region that specifically binds to CLAUDIN18.2 is from CN202010570517. X.

## IV. The nucleic acid of the invention and the host cell containing the same

In one aspect, the invention provides a nucleic acid encoding anyone of the above anti-CD3 antibodies or fragments thereof. In one embodiment, a vector comprising the nucleic acid is provided. In one embodiment, the vector is an expression vector, such as pcDNA3.1. In one embodiment, a host cell comprising the nucleic acid or the vector is provided. In one embodiment, the host cell is eukaryotic. In another embodiment, the host cell is selected from yeast cells, mammalian cells (such as CHO cells (such as CHO-S) or 293 cells (such as 293F, such as Expi293F) or other cells suitable for the preparation of antibodies or fragments thereof. In another embodiment, the host cell is prokaryotic.

In one aspect, the present invention provides a nucleic acid encoding anyone of the anti-CD3 antibody or the fragment thereof as described herein. Said nucleic acid of the invention may comprise a nucleic acid encoding the amino acid sequence of the light chain variable region and/or the heavy chain variable region of the antibody, or a nucleic acid encoding the amino acid sequence of the light chain and/or the heavy chain of the antibody.

For example, the nucleic acid of the invention comprises the nucleic acid encoding the amino acid sequence selected from anyone of SEQ ID NO: 48-75 and 77-99, or encoding the amino acid sequence having at least 85%, 90%, 91%, 92%, 93%, 94%, 95%, 96%, 97%, 98% or 99% identity with the amino acid sequence selected from anyone of SEQ ID NO: 48-75 and 77-99.

The invention also covers nucleic acids that hybridize with the following nucleic acids under strict conditions or have one or more substitutions (such as conservative substitutions), deletions or insertions compared to the following nucleic acids: nucleic acids comprising nucleic acid sequences encoding amino acid sequences selected from shown in anyone of SEQ ID NO: 48-75 and 77-99; or a nucleic acid comprising nucleic acid sequences encoding amino acid sequences having at least 85%, 90%, 91%, 92%, 93%, 94%, 95%, 96%, 97%, 98% or 99% identity with the amino acid sequence shown in anyone of SEQ ID NO: 48-75 and 77-99.

In one embodiment, one or more vectors comprising the nucleic acid are provided. In one embodiment, the vector is an expression vector, such as an eukaryotic expression vector. The vectors include but are not limited to viruses, plasmids, cosmid, λ Phage or yeast artificial chromosome (YAC). In one embodiment, the vector is pcDNA3.1.

In one embodiment, a host cell comprising the vector is provided. Suitable host cells for cloning or expressing vectors encoding antibodies include prokaryotic or eukaryotic cells described herein. For example, antibodies can be produced in bacteria.

In one embodiment, the host cell is eukaryotic. In another embodiment, the host cell is selected from yeast cells, mammalian cells or other cells suitable for preparing antibodies or fragments thereof. For example, eukaryotic microorganisms such as filamentous fungi or yeast are suitable hosts of cloning or expression for vectors encoding antibodies. For example, fungi and yeast strains whose glycosylation pathway has been "humanized" lead to the production of antibodies with partial or complete human glycosylation patterns. Host cells suitable for expressing glycosylation antibodies are also derived from multicellular organisms (invertebrates and vertebrates). Vertebrate cells can also be used as hosts. For example, mammalian cell lines adapted to suspension growth can be used. Other examples of useful mammalian host cell lines are monkey kidney CV1 line (COS-7) transformed with SV40; human embryonic kidney system (HEK293, 293F or 293T cells, such as Expi293F cells), etc. Other useful mammalian host cell lines include Chinese hamster ovary (CHO) cells, including DHFR-CHO cells, CHO-S cells, ExpiCHO, etc; and myeloma cell lines such as Y0, NS0 and Sp2/0. A mammalian host cell line suitable for producing antibodies is known in the art.

### V. Production and purification of antibody molecule of the invention

In one aspect, the invention provides a method for regulating the binding affinity of anti-CD3 antibody or fragments thereof, which includes introducing amino acid change(s) to the heavy chain variable region CDR and/or light chain variable region CDR of the antibody molecule.

In one embodiment, the invention provides a method for preparing the antibody molecule or fragment thereof (preferably antigen-binding fragment) of the invention, wherein the method comprises culturing the host cell under conditions suitable for expressing nucleic acid encoding the antibody molecule or fragment thereof (preferably antigen-binding fragment) of the invention, and optionally isolating the antibody or fragment thereof (for example, antigen-binding fragment). In a certain embodiment, the method further includes recovering the antibody molecule or fragment thereof (such as antigen-binding fragment) of the invention from the host cell.

In one embodiment, a method for preparing the antibody molecule of the invention is provided, wherein the method includes culturing a host cell comprising a nucleic acid encoding the antibody (such as any of one polypeptide chain and/or more polypeptide chains) or an expression vector comprising the nucleic acid under conditions suitable for antibody expression, as provided above, and optionally recovering the antibody from the host cell (or host cell culture medium).

In order to recombine and produce the antibody molecule of the invention, the nucleic acid encoding the antibody (such as the antibody described above, such as any of one polypeptide chain and/or multiple polypeptide chains) is separated and inserted into one or more vectors for further cloning and/or expression in the host cell. Such nucleic acids can be easily separated and sequenced using conventional procedures (for example, by using oligonucleotide probes that can specifically bind to genes encoding the heavy and light chains of antibodies).

In one embodiment, the antibody molecule of the invention is a multi-specific antibody molecule, such as a bispecific antibody molecule. Therefore, the invention also provides a method for preparing multi-specific antibody molecules (such as bispecific antibody molecules) that bind to CD3 and other cancer-related antigens, wherein the method includes culturing host cells comprising a nucleic acid encoding the antibody (such as any of one polypeptide chain and/or more polypeptide chains) or expression vectors containing the nucleic acid under conditions suitable for the expression of the multi-specific antibody, as provided above, and optionally recovering the antibody from the host cell (or host cell culture medium).

The antibody molecules prepared as described herein can be purified by known existing technologies such as high-performance liquid chromatography, ion exchange chromatography, gel electrophoresis, affinity chromatography, size exclusion chromatography, etc. The actual conditions used to purify specific proteins also depend on factors such as net charge, hydrophobicity, hydrophilicity, etc., which are obvious to those skilled in the art. The purity of the antibody molecule of the invention can be determined by any of a variety of well-known analytical methods, which include size exclusion chromatography, gel electrophoresis, high-performance liquid chromatography, etc.

## VI. Assays

The anti-CD3 antibody provided herein can be identified, screened, or characterized by its physical/chemical properties and/or biological activity through a variety of assays known in the art. On the one hand, the antigen-binding activity of the antibody of the invention is tested, for example, by known methods such as ELISA, Western blotting, etc. The methods known in the art can be used to determine the binding to CD3 and an exemplary method is disclosed herein. In some embodiments, radioimmunoassay (RIA) or biomembrane thin-layer interferometry or MSD or surface plasmon resonance (SPR) or flow cytometry are used.

On the other hand, a competitive assay can be used to identify antibodies that compete with any anti-CD3 antibody disclosed herein for binding to CD3. In some embodiments, such competitive antibodies bind to the same or overlapping epitopes (such as linear or conformational epitopes) as the any anti-CD3 antibody disclosed herein.

The invention also provides an assay for identifying anti-CD3 antibodies with biological activity. The biological activities can include, for example, binding to CD3 (for example, binding to human CD3 or cynomolgus monkey CD3), binding to cells (such as T cells, such as human T lymphocytes, such as Jurkat cells) expressing CD3, activation of T cells, etc. Antibodies with such biological activity in vivo and/or in vitro are also provided.

In some embodiments, the antibody of the invention is tested for such biological activity.

The cells used for any of the above in vitro assays include cell lines which naturally express CD3, or express or overexpress CD3 through modification. Such cells also include cell lines that express CD3 and those that do not normally express CD3 and are transfected by DNA coding CD3. In some embodiments, such cells are T cells, such as human T lymphocytes, such as Jurkat cells.

It can be understood that the immunoconjugate of the invention can be used for replace or supplement the anti-CD3 antibody for any of the above determination methods.

## VII. Immunoconjugates

In some embodiments, the invention provides immunoconjugates, which comprise any anti-CD3 antibody and other substances provided herein, such as therapeutic agents, including chemotherapeutic agents, cytokines, cytotoxic agents, other antibodies, small molecule drugs or immunomodulators (such as anti-inflammatory agents or immunosuppressants). In one embodiment, the said other substances, such as cytotoxic agents, include any agents harmful to cells.

In some embodiments, the said immunoconjugate is used to prevent or treat cancer.

## VIII. Pharmaceutical composition and pharmaceutical formulations

In some embodiments, the present invention provides a composition comprising any anti-CD3 antibody or fragment thereof (preferably antigen-binding fragment thereof) or immunoconjugates thereof described herein, preferably the composition is a pharmaceutical composition. In one embodiment, the composition further comprises pharmaceutically acceptable supplementary material. In one embodiment, the composition, for example, the pharmaceutical composition, comprises a combination of an anti-CD3 antibody or fragment thereof or immunoconjugate of invention, and one or more other therapeutic agents.

The invention further includes a composition comprising anti-CD3 antibody or immunoconjugate thereof (including a pharmaceutical composition or a pharmaceutical formulation), or a composition comprising polynucleotides encoding anti-CD3 antibody (including a pharmaceutical composition or a pharmaceutical formulation). In some embodiments, the composition comprises one or more CD3-binding antibodies or fragments thereof, or one or more polynucleotides encoding one or more anti-CD3 antibodies or fragments thereof. These compositions can further comprise suitable comprise suitable pharmaceutically acceptable suppl, such as pharmaceutically acceptable carriers and pharmaceutically acceptable excipients, including buffers known in the art.

As used herein, " pharmaceutically acceptable carrier" includes any and all physiologically compatible solvents, dispersion media, isotonic agents and absorption retardants.

For the use and use of pharmaceutically acceptable supplementary material, see also "Handbook of Pharmaceutical Excipients", 8th edition, R.C. Rowe, P.J. Seskey and S.C. Owen, Pharmaceutical Press, London, Chicago.

The composition of the present invention can be in various forms. These forms include, for example, liquid, semi-solid and solid dosage forms, such as liquid solution (for example, injectable solution and infusion solution), powder or suspension, liposome and suppository. The preferred form depends on the intended mode of administration and therapeutic use.

A pharmaceutical formulation comprising the antibody described herein can be prepared by mixing the antibody of the invention with the required purity with one or more optional pharmaceutically acceptable supplementary material, preferably in the form of lyophilized preparation or aqueous solution.

The pharmaceutical composition or formulation of the invention can further comprise more than one active ingredients, which are required for the specific indication to be treated, preferably those active ingredients with complementary activities that will not adversely affect each other. For example, it is ideal to also provide other therapeutic agents, such as chemotherapeutic agents, cytokines, cytotoxic agents, vaccines, other antibodies, small molecular drugs or immunomodulators etc. The said active ingredients are appropriately combined in an effective amount for the intended use.

Sustained release formulations can be prepared. Suitable examples of sustained release formulations include semi-permeable matrices comprising solid hydrophobic polymers containing antibodies, and the said matrices are in the form of shaped articles, such as films or microcapsules.

**IX. Pharmaceutical combination and kit**

In some embodiments, the invention further provides a pharmaceutical combination or a pharmaceutical combination product, which include the anti-CD3 antibodies or fragments thereof (preferably antigen-binding fragments) or immunoconjugate thereof of the invention, and one or more other therapeutic agents (such as chemotherapy agents, cytokines, cytotoxic agents, other antibodies, small molecule drugs or immunomodulators etc.).

Another object of the invention is to provide a kit comprising the pharmaceutical combination of the invention, preferably in the form of drug dose unit. Therefore, the dose unit can be provided according to the regimen or interval of the administration.

In one embodiment, the kit of the invention comprises:

-    a first container comprising a pharmaceutical composition containing the anti-CD3 antibody or the fragment thereof of the invention;

-    a second container comprising a pharmaceutical composition containing other therapeutic agent(s).

**X. Use and Method**

On the one hand, the invention provides a method for preventing or treating tumors (such as cancer) in a subject, including administering to the subject the therapeutically effective anti-CD3 antibody or fragment thereof, the immunoconjugate, the pharmaceutical composition, the pharmaceutical combination or the kit of the invention.

In some embodiments, the tumors, such as cancer, include solid tumors, blood tumors and metastatic lesions. In one embodiment, examples of solid tumors include malignant tumors. Cancer can be in the early, middle or late stage or metastatic cancer. In some embodiments, a tumor is at immune escape of the tumor.

In a specific embodiment, the anti-CD3 antibody of the invention can activate T cells. In a specific embodiment, the antibody of the invention can kill tumor cells, and/or inhibit the proliferation of tumor cells.

Therefore, the anti-CD3 antibody of the invention is applicable to prevent or treat any tumor or cancer in which the effector mechanism of cytotoxic T cells is required, or any tumor or cancer requiring T cell recruitment. In some embodiments, the tumor or cancer treatment will benefit from T cell activation, or effector mechanism of cytotoxic T cells or T cell recruitment.

In a specific aspect, the anti-CD3 antibody of the invention is a multi-specific antibody (such as a bispecific antibody), which specifically binds to CD3, and one or more cancer-related antigens (such as cancer-specific antigen/target or antigen/target over-expressed in cancer or cancer-related antigen/target), such as HER2 or CD70 or CLAUDIN18.2. In some embodiments, the said tumor (such as cancer) patients have cancer-related antigens (of altered, such as elevated levels, such as in nucleic acid or protein levels). In some embodiments, the tumor treatment will benefit from the increasing or inhibition of the nucleic acid level or protein level of the cancer-related antigen(s).

The subject can be a mammal, such as a primate, preferably a higher primate, such as a human (for example, an individual suffering from the disease described herein or at risk of suffering from the disease described herein). In one embodiment, the subject suffers from the disease described herein (for example, cancer) or is at risk of suffering from the disease described herein. In some embodiments, the subjects are receiving or have received other treatments, such as chemotherapy and/or radiotherapy. In some embodiments, the subjects have received immunotherapy before or are receiving immunotherapy.

In other aspects, the invention provides the use of the antibody molecule or fragment thereof or immunoconjugate thereof or pharmaceutical composition or pharmaceutical combination or kit in the production or preparation of a medicine, which are used for the purposes described herein, for example, for the prevention or treatment of related diseases or disorders mentioned herein.

In some embodiments, the antibody molecule or fragment thereof or immunoconjugate thereof or pharmaceutical composition or pharmaceutical combination or kit of the invention may delay the onset of the disease and/or symptoms related to the disease.

In some embodiments, the antibody molecule or fragment thereof or immunoconjugate thereof or pharmaceutical composition or pharmaceutical combination or kit of the invention can also be used in combination with one or more other therapies, such as therapeutic modes and/or other therapeutic agents, for the uses described herein, such as for the prevention and/or treatment of related diseases or disorders mentioned herein. In some embodiments, the therapeutic modes include surgery; radiotherapy, local irradiation or focused irradiation, etc. In some embodiments, the therapeutic agents are selected from chemotherapeutic agents, cytokines, cytotoxic agents, vaccines, other antibodies, small molecule drugs or immunomodulators. Exemplary immunomodulators include immunosuppressants or anti-inflammatory agents.

In some embodiments, the antibody combination described herein can be administered separately, for example, as separate antibodies.

Such combination therapy covers combination administration (for example, two or more therapeutic agents are included in the same or separate formulation), and separate administration. In this case, the administration of the antibody of the invention can occur before, at the same time, and/or after the administration of other therapeutic agents and/or drugs.

The route of administration of the pharmaceutical composition is based on known methods, such as oral, intravenous injection, intraperitoneal, intracerebral (parenchymal), intraventricular, intramuscular, ophthalmic, intra-arterial, intra-portal or intrafocal route; by continuous release system or by implantable device. In some embodiments, the composition may be administered by bolus injection or by continuous infusion or by an implant device.

The composition can also be applied locally via an implanted membrane, sponge or another suitable material on which the required molecules are absorbed or encapsulated. In some embodiments, when an implant device is used, the device can be implanted into any suitable tissue or organ, and the required molecules can be delivered through diffusion, timed release of bolus, or continuous administration.

## XI. Methods and compositions for diagnosis and detection

In some embodiments, the anti-CD3 antibodies or fragments thereof (preferably antigen-binding fragment) provided herein can be used to detect the presence of CD3 or cancer specific target in biological samples.

The term "detection" as used herein includes quantitative or qualitative detection, and exemplary detections may involve immunohistochemistry, immunocytochemistry, flow cytometry (e.g., FACS), magnetic beads complexed with antibody molecules, ELISA, and PCR techniques (e.g., RT-PCR). In some embodiments, the biological sample is blood, serum, or other fluid sample of biological source. In certain embodiments, the biological sample includes cells or tissues. In some embodiments, the biological sample is derived from a proliferative or cancerous lesion related lesion.

In one embodiment, the antibody of the invention can be used to diagnose tumors, such as cancers, e.g., to assess (e.g., monitor) the treatment or progression, diagnosis and/or staging of a disease described herein in a subject. In certain embodiments, a labeled anti-CD3 antibody or the fragment thereof is provided. The label includes, but is not limited to, a label or moiety (e.g., a fluorescent label, a chromophore label, an electron-dense label, a chemoluminescent label, and a radioactive label) that is detected directly, as well as a moiety that is detected indirectly, such as an enzyme or a ligand, for example, by an enzymatic reaction or a molecular interaction.

In some embodiments, the sample is formalin-fixed and paraffin-coated (FFPE). In some embodiments, samples are biopsies (such as core biopsies), surgical specimens (such as specimens from surgical resection), or fine needle aspirates.

These and other aspects and embodiments of the invention are described in the drawings (brief description of the drawings follows) and in the following detailed description of the invention and are illustrated in the following examples. Any or all of the features discussed above and throughout the application may be combined in various embodiments of the invention. The following examples further illustrate the invention. However, it is to be understood that the examples are described by way of illustration and not limitation, and various modifications may be made by those skilled in the art.

## Examples

## Example 1. Design of humanized sequence of mouse CD3 antibody sp34

The murine CD3 antibody sp34 (U.S. Pat. No. 8236308; J. Immunol. Methods., 1994, 178:195) has the function of activating T cells and can form a CD3 adaptor with the tumor cell-specific antigen molecules, so as to promote T cells to target and kill tumor cells. In order to reduce its immunogenicity, the invention firstly humanizes its sequence.

The CDR region of sp34 antibody is defined, in which the heavy chain CDR1 uses the AbM scheme comprehensively, and the other CDRs use the Kabat scheme. Through sequence similarity comparison, the antibody germ line with the highest similarity to sp34 is selected as the antibody template. The CDR regions of the template are replaced with the CDR region of the light chain and the heavy chain, and then the key amino acids are back mutated according to the simulated three-dimensional structure. The specific humanization process is as follows:

(1) Selecting IGHV3-73*01 and IGHJ6*01 (see the following table for sequence) as the heavy chain variable region antibody template of sp34, selecting IGKV3-7*02 and IGKJ1*01 of Kappa (see the following table for sequence) and IGLV7-46*02 and IGLJ3*02 of Lambda (see the following table for sequence) as an antibody template of light chain variable region of sp34 respectively, replacing the heavy chain or light chain CDR regions of the antibody template with the CDR regions of the sp34, and obtaining the variable region sequence husp34h.g0(SEQ ID NO: 48), Husp34k.g0(SEQ ID NO: 81) and husp34l.g0(SEQ ID NO: 77) respectively.

| Name of sequence | SEQ ID NO | Sequence |
|---|---|---|
| IGHV3-73*0 1 | 108 | EVQLVESGGGLVQPGGSLKLSCAASGFTFSGSAMHWVRQ ASGKGLEWVGRIRSKANSYATAYAASVKGRFTISRDDSKN TAYLQMNSLKTEDTAVYYCTR |
| IGKV3-7*02 | 109 | EIVMTQSPPTLSLSPGERVTLSCRASQSVSSSYLSWYQQKP GQAPRLLIYGASTRATGIPARFSGSGSGTDFTLTISSLQPED FAVYYCQQDYNLP |
| IGLU7-46*0 2 | 110 | QAVVTQEPSLTVSPGGTVTLTCGSSTGAVTSGHYPYWFQQ KPGQAPRTLIYDTSNKHSWTPARFSGSLLGGKAALTLLGA QPEDEAEYYCLLSYSGAR |
| IGHJ6*01 | 111 | WGQGTTVTVSS |
| IGKJ1*01 | 112 | FGQGTKVEIK |
| IGLJ3*02 | 113 | FGGGTKLTVL |

(2) Using Discovery Studio software to conduct homologous modeling of the variable region of sp34 antibody, and obtaining the three-dimensional structure model of the variable region of sp34.

(3) According to the variable region structure of sp34 antibody, determine the key amino acids that affect the interaction of heavy and light chains and the interaction with CDR, determining the amino acid sites of the back mutation, and obtaining husp34h.g1 (SEQ ID NO: 49). At the same time, the amino acid N in the potential deamidation site NS in the heavy chain CDR3 is mutated to Q, so as to obtain three heavy chain variable region sequences, husp34h.g2 (SEQ ID NO: 50) and husp34h.g3 (SEQ ID NO: 51) and six light chain variable region sequences, husp34k.g1 (SEQ ID NO: 82), husp34k.g2 (SEQ ID NO: 83), husp34k.g3 (SEQ ID NO: 84), husp34l.g1 (SEQ ID NO: 78), husp34l.g2 (SEQ ID NO: 79) and husp34l.g3 (SEQ ID NO: 80), respectively.

### Example 2 Preparation of humanized antibody

The light and heavy chains of antibody variable region sequence in Example 1 is combined with each other. See Table 1 for the specific combination. The human IgG1 L234AL235A sequence (SEQ ID NO: 100) is selected as the heavy chain constant region, and the corresponding constant region CL-Kappa (SEQ ID NO: 102) and CL-Lambda (SEQ ID NO: 101) are selected as the light chain constant region according to whether the variable region is Kappa or Lambda. Then the heavy chain sequence and light chain sequence of the antibody were respectively constructed into the expression vector pcDNA3.1 (Invitrogen, V790-20), and each plasmid was obtained. Expi293 cells (Invitrogen, A14527) were used to transiently transfect to obtain the corresponding humanized antibody. The specific transfection and purification process is as follows:

Expi293 cells were passaged according to the required transfection volume, and the cell density was adjusted to $1.5\times10^6$ cells/ml the day before transfection. The cell density on the day of transfection is about $3\times10^6$ cells/ml. Taking 1/10 of the final volume of Opti-MEM medium (Gibco, 31985-070) as the transfection buffer, appropriate plasmid at $1.0\mu g$/ml is added to the transfected cells and mixed well. Appropriate polyethylene imines (PEI) (23966) was added into the plasmid (the ratio of plasmid to PEI is 1:3 in 293F cells), mixed well and incubated at room temperature for 20 min to obtain the DNA/PEI mixture. The DNA/PEI mixture was added slowly into the cell, and the flask was shook gently while adding, and then the cells were cultured in a 36.5°C, 8% $CO_2$ incubator. After seven days, the cell culture was obtained and the cell supernatant was collected for purification.

The Protein A column (Hitrap Mabselect Sure, GE, 11-0034-95) for purification was treated with 0.1 M NaOH for 2h, and the glass bottles were washed with distilled water and dried at 180°C for 4h. Before purification, the collected cell culture was centrifuged at 4500 rpm for 30 min, and the cells were discarded and filtered with 0.22 μM filter. 10 column volumes of binding buffer (sodium phosphate 20mM. NaCl 150M, PH7.0) were used to equilibrate Protein A column. The filtered supernatant was added into the purification column and equilibrated by 10 column volumes of binding buffer. 5ml elution buffer (citric acid+sodium citrate 0.1M, pH3.5) was added and the eluent was collected, and 80μL Tris-HCl at the concentration of 2M was added to 1ml eluent. The collected antibody was exchanged into PBS (Gibco, 70011-044) by ultrafiltration and concentration, and the concentration was detected.

Table 1 Table of light and heavy chain combination of sp34 humanized antibody

| protein ID | VH | SEQ ID NO | VL | SEQ ID NO |
|---|---|---|---|---|
| sp34 | SP34 H | 47 | SP34_L | 76 |
| hzsp34.7 | husp34h.g0 | 48 | husp34l.g0 | 77 |
| hzsp34.8 | husp34h.g1 | 49 | husp34l.g0 | 77 |
| hzsp34.9 | husp34h.g2 | 50 | husp34l.g0 | 77 |
| hzsp34.10 | husp34h.g3 | 51 | husp34l.g0 | 77 |
| hzsp34.12 | husp34h.g0 | 48 | husp34l.g1 | 78 |
| hzsp34.13 | husp34h.g1 | 49 | husp34l.g1 | 78 |
| hzsp34.14 | husp34h.g2 | 50 | husp34l.g1 | 78 |
| hzsp34.15 | husp34h.g3 | 51 | husp34l.g1 | 78 |
| hzsp34.17 | husp34h.g0 | 48 | husp34l.g2 | 79 |
| hzsp34.18 | husp34h.g1 | 49 | husp34l.g2 | 79 |
| hzsp34.19 | husp34h.g2 | 50 | husp34l.g2 | 79 |
| hzsp34.20 | husp34h.g3 | 51 | husp34l.g2 | 79 |
| hzsp34.22 | husp34h.g0 | 48 | husp34l.g3 | 80 |
| hzsp34.23 | husp34h.g1 | 49 | husp34l.g3 | 80 |
| hzsp34.24 | husp34h.g2 | 50 | husp34l.g3 | 80 |
| hzsp34.25 | husp34h.g3 | 51 | husp34l.g3 | 80 |
| hzsp34.27 | husp34h.g0 | 48 | husp34k.g0 | 81 |
| hzsp34.28 | husp34h.g1 | 49 | husp34k.g0 | 81 |
| hzsp34.29 | husp34h.g2 | 50 | husp34k.g0 | 81 |
| hzsp34.30 | husp34h.g3 | 51 | husp34k.g0 | 81 |
| hzsp34.32 | husp34h.g0 | 48 | husp34k.g1 | 82 |
| hzsp34.33 | husp34h.g1 | 49 | husp34k.g1 | 82 |
| hzsp34.34 | husp34h.g2 | 50 | husp34k.g1 | 82 |
| hzsp34.35 | husp34h.g3 | 51 | husp34k.g1 | 82 |
| hzsp34.37 | husp34h.g0 | 48 | husp34k.g2 | 83 |
| hzsp34.38 | husp34h.g1 | 49 | husp34k.g2 | 83 |
| hzsp34.39 | husp34h.g2 | 50 | husp34k.g2 | 83 |
| hzsp34.40 | husp34h.g3 | 51 | husp34k.g2 | 83 |
| hzsp34.42 | husp34h.g0 | 48 | husp34k.g3 | 84 |
| hzsp34.43 | husp34h.g1 | 49 | husp34k.g3 | 84 |
| hzsp34.44 | husp34h.g2 | 50 | husp34k.g3 | 84 |
| hzsp34.45 | husp34h.g3 | 51 | husp34k.g3 | 84 |

In the antibody with a VH of husp34h.g0 or husp34h.g1 listed in Table 1, the sequence of heavy chain HCDR1 is shown in SEQ ID NO: 1, the sequence of HCDR2 is shown in SEQ ID NO: 2, the sequence of HCDR3 is shown in SEQ ID NO: 3, the sequence of light chain LCDR1 is shown in SEQ ID NO: 29, the sequence of LCDR2 is shown in SEQ ID NO: 30, and the sequence of light chain LCDR3 is shown in SEQ ID NO: 31. In the antibody with a VH of husp34h.g2 or husp34h.g3 listed in Table 1, the sequence of heavy chain HCDR1 is shown in SEQ ID NO: 1, the sequence of HCDR2 is shown in SEQ ID NO: 2, the sequence of HCDR3 is shown in SEQ ID NO: 8, the sequence of light chain LCDR1 is shown in SEQ ID NO: 29, the sequence of LCDR2 is shown in SEQ ID NO: 30, and the sequence of light chain LCDR3 is shown in SEQ ID NO: 31.

## Example 3 Affinity test of humanized antibody

3.1 Determination of the binding kinetics of the antibody of the invention with human CD3 protein and cynomolgus monkey CD3 protein by Biolayer Interferometry technology

The equilibrium dissociation constant (KD) of the antibody of the invention binding to human CD3 protein and cynomolgus monkey CD3 protein was determined by using the Biolayer Interferometry (BLI). The affinity determination of BLI method was carried out according to the existing methods (Estep, P et al., High throughput solution Based measurement of antibody-antigen affinity and affinity binding. MAbs, 2013.5 (2): 270-8).

Half an hour before the experiment, according to the number of samples, an appropriate number of AHC (18-5060, Fortebio) sensors were taken and soaked in SD buffer (1x PBS, BSA0.1%, Tween-20 0.05%).

100 $\mu$l SD buffer, various antibodies, human CD3 E&D complex protein (Sino Biological Inc., CT026H0323H), and cynomolgus monkey CD3 E&D complex protein (Sino Biological Inc., CT032-C0323H) were added into 96-well black polystyrene semi-quantitative micro-well plate (Greiner, 675076) respectively. Fortebio Octet Red 96 was used for detection, and the sensor position was selected according to the sample position. The setting parameters of the instrument are as follows: operation steps: Baseline, Loading-1 nm, Baseline, Association and Dissociation; the operation time of each step depends on the speed of sample association and dissociation. The rotational speed is 1000 rpm and the temperature is 30 °C. ForteBio Octet analysis software was used to analyze KD value.

In the experiment described in the above determination method, the affinity of the antibody is shown in Table 2, where N. B represents no binding, and the affinity of hzsp34.12~hzsp34.15, hzsp34.17~hzsp34.20, hzsp34.22~hzsp34.25 is comparable with that of mouse sp34 ($10^{-10}$-$10^{-9}$ M), while the affinity of hzsp34.37~hzsp34.40, hzsp34.42~hzsp34.45 is reduced ($10^{-9}$~$10^{-8}$ M).

Table 2 Binding kinetic constants of sp34 humanized antibody

|  | Affinity to human CD3E&D | | | Affinity to cynomolgus monkey CD3E&D | | |
|---|---|---|---|---|---|---|
|  | KD (M) | kon(1/Ms) | kdis(1/s) | KD (M) | kon(1/Ms) | kdis(1/s) |
| sp34 | 9.319E-10 | 2.04E+06 | 1.90E-03 | 6.46E-10 | 1.23E+06 | 7.95E-04 |
| hzsp34.7 | N.B | | | 1.655E-09 | 2.79E+05 | 4.63E-04 |
| hzsp34.8 | N.B | | | N.B | | |
| hzsp34.9 | N.B | | | N.B | | |
| hzsp34.10 | N.B | | | N.B | | |
| hzsp34.12 | 6.192E-09 | 6.40E+05 | 3.96E-03 | 4.732E-09 | 4.79E+05 | 2.26E-03 |
| hzsp34.13 | 4.329E-09 | 1.08E+06 | 4.66E-03 | 1.75E-09 | 9.37E+05 | 1.64E-03 |
| hzsp34.14 | 2.41E-09 | 6.61E+05 | 1.59E-03 | 1.946E-10 | 4.88E+05 | 9.49E-05 |
| hzsp34.15 | 3.128E-09 | 6.44E+05 | 2.02E-03 | 1.75E-09 | 6.58E+05 | 1.15E-03 |
| hzsp34.17 | 5.105E-10 | 4.04E+06 | 2.06E-03 | 5.145E-10 | 1.78E+06 | 9.15E-04 |
| hzsp34.18 | 6.286E-10 | 3.31E+06 | 2.08E-03 | 6.7E-10 | 1.31E+06 | 8.80E-04 |
| hzsp34.19 | 6.132E-10 | 3.80E+06 | 2.33E-03 | 6.023E-10 | 1.64E+06 | 9.90E-04 |
| hzsp34.20 | 8.473E-10 | 3.25E+06 | 2.75E-03 | 7.556E-10 | 1.46E+06 | 1.10E-03 |
| hzsp34.22 | 6.049E-10 | 3.54E+06 | 2.14E-03 | 5.845E-10 | 1.52E+06 | 8.86E-04 |
| hzsp34.23 | 5.538E-10 | 3.57E+06 | 1.98E-03 | 6.045E-10 | 1.31E+06 | 7.91E-04 |

(continued)

| | Affinity to human CD3E&D | | | Affinity to cynomolgus monkey CD3E&D | | |
|---|---|---|---|---|---|---|
| | KD (M) | kon(1/Ms) | kdis(1/s) | KD (M) | kon(1/Ms) | kdis(1/s) |
| hzsp34.24 | 7.175E-10 | 3.44E+06 | 2.47E-03 | 7.38E-10 | 1.28E+06 | 9.41E-04 |
| hzsp34.25 | 1.165E-09 | 2.35E+06 | 2.73E-03 | 8.569E-10 | 1.07E+06 | 9.13E-04 |
| hzsp34.27 | N.B | | | N.B | | |
| hzsp34.28 | N.B | | | 2.824E-09 | 4.15E+05 | 1.17E-03 |
| hzsp34.29 | N.B | | | N.B | | |
| hzsp34.30 | N.B | | | N.B | | |
| hzsp34.32 | N.B | | | N.B | | |
| hzsp34.33 | N.B | | | N.B | | |
| hzsp34.34 | N.B | | | N.B | | |
| hzsp34.35 | N.B | | | N.B | | |
| hzsp34.37 | 9.157E-09 | 4.36E+05 | 3.99E-03 | 6.238E-09 | 3.21E+05 | 2.00E-03 |
| hzsp34.38 | 1.271E-08 | 3.97E+05 | 5.05E-03 | 6.36E-09 | 2.88E+05 | 1.83E-03 |
| hzsp34.39 | 7.847E-09 | 4.51E+05 | 3.54E-03 | 7.098E-09 | 2.86E+05 | 2.03E-03 |
| hzsp34.40 | 1.679E-08 | 3.07E+05 | 5.15E-03 | 1.529E-08 | 1.96E+05 | 3.00E-03 |
| hzsp34.42 | 1.472E-08 | 3.57E+05 | 5.26E-03 | 1.021E-08 | 2.43E+05 | 2.48E-03 |
| hzsp34.43 | 1.264E-08 | 3.59E+05 | 4.53E-03 | 7.916E-09 | 2.53E+05 | 2.01E-03 |
| hzsp34.44 | 1.646E-08 | 3.53E+05 | 5.81E-03 | 1.212E-08 | 2.41E+05 | 2.93E-03 |
| hzsp34.45 | 1.842E-08 | 3.53E+05 | 6.50E-03 | 2.687E-08 | 1.94E+05 | 5.21E-03 |

3.2 Detection of the binding of the antibody of the invention to human CD3 with Jurkat cell line

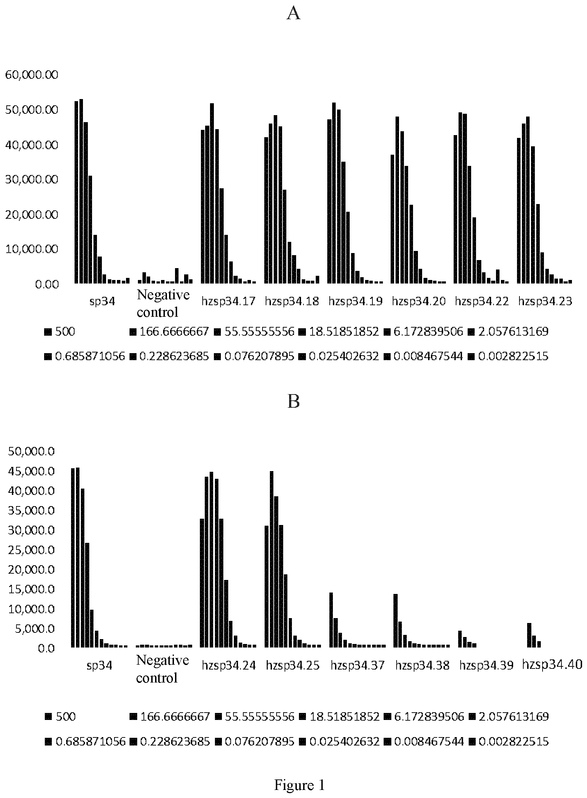

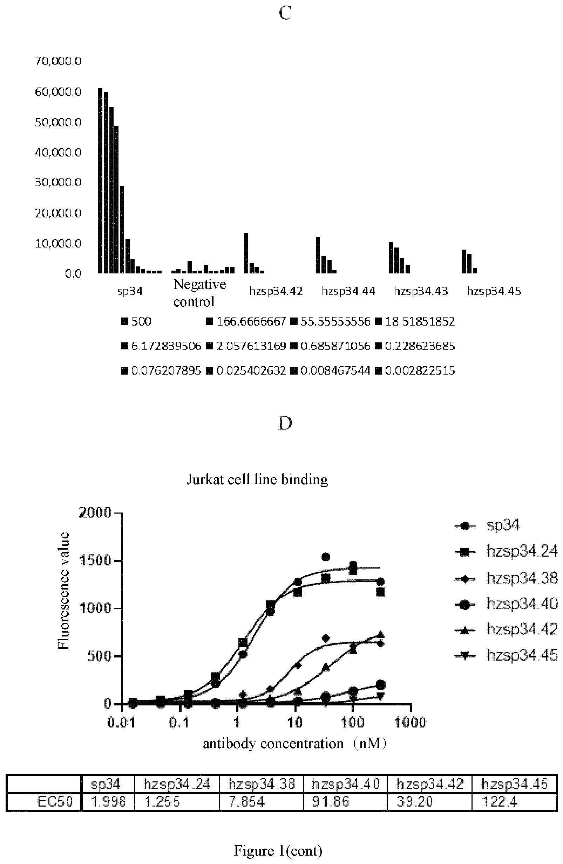

Jurkat cells are immortalized human T lymphocytes, which express human CD3 complex. The cell line is used to detect the binding of the antibody to the cell. The detailed operation was as follows: Jurkat cells (Promega, J1621) was inoculated into U-shaped 96-well plate, $2\times10^5$ for each well. The antibodies to be detected at a series of concentration gradients (the initial concentration of antibody molecule is 500 nM, 3 folds serial dilution) was added into the corresponding cell well, incubated at 4°C for 30 minutes, and then PBS was used to wash out the unbound part. The sheep anti-human Fc PE fluorescent second antibody (Southern Biotech, J2815-5H87B) was added, then incubated at 4 °C for 15 minutes, and detected by flow cytometry (FACSCELESTA BD). The results showed that the humanized antibodies hzsp34.17~hzsp34.20, hzsp34.22~hzsp34.25 showed comparable affinity at the cellular level as the chimeric antibody sp34, while the affinity of hzsp34.37~hzsp34.40, hzsp34.42~hzsp34.45 decreased, one-to-one corresponding to the protein level (Fig. 1A, B and C). Some of these antibodies were selected to make a further binding curve at cell level. The results were shown in Figure 1D, which better showed the binding of humanized antibodies with different CD3 affinity at cell level, and wherein the binding of hzsp34.24 and chimeric antibody sp34 to T cells was the closest.

**Example 4 Detection of T cell activation function of humanized antibody**

The invention uses Jurkat NFAT (Nuclear Factor of Activated T) reporter cell (Promega, J1621) to detect the T cell activation function of sp34 humanized antibody. The cell is an engineered Jurkat T cell. When the cell is activated through TCR-CD3 pathway, it releases luciferase substrate into the experimental system through the downstream signal NFAT, so that the activation degree of T cells can be detected.

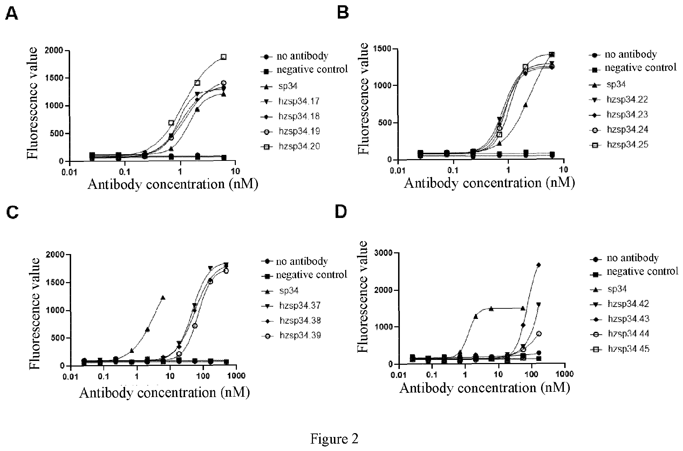

The detailed operation of this example is as follows: in a white 96-well flat plate, $4\times10^4$ Jurkat NFAT cells/well were mixed with each antibody molecule at corresponding concentration (the initial concentration of antibody molecules was 500 nM, 3 folds serial dilution) in each well, and incubated in 37 °C incubator for 6-8 hours. Afterwards, 100 $\mu$L of Bio-Glo (Promega, G7940) was added to each well. The wavelength detection was performed using the microplate reader (Spectra, Molecular Devices). Results were shown in Figures 2A and B. It can be seen that the T cell activation ability of sp34 humanized antibody has a corresponding relationship to its affinity.

**Example 5 CDR mutant of humanized antibody**

5.1 Design and preparation of mutants

At the same time, an amino acid mutation on the CDR region of the humanized sp34 antibody was performed in the invention, with the aim of reducing its affinity with CD3, so as to obtain molecules with different T cell activation abilities to meet the needs of CD3 adapters for different tumor specific antigens.

The specific operation is as follows: take hzsp34.24 as the initial sequence, select the amino acids at the positions of heavy chain H31, H32, H33, H52, H52A, H52C, H53, H54, H95, H96, H97, H98, H99, H100, H100A, H100B, H100C (Kabat number) and light chain L24, L28, L29, L30, L31, L53, L91, L92, L93, L94 (Kabat number) as the target amino acids, and then conduct point mutation and combined mutation respectively. The heavy chain variable region sequence husp34h. g2.1~husp34h. g2.24 (see the sequence listing for sequences) and the light chain variable region sequence husp34lg3.1~husp34lg3.15 (see the sequence listing for the sequences) were obtained respectively. The basic principle of point mutation is (Table 3): aromatic amino acids Y, W and F are mutated into amino acids G, A and S with relatively small side chains; amino acids with positively charged R, K, H are mutated into the amino acids G, A, S with relatively small side chain; amino acid G with hydrogen atom in the side chain is mutated into aromatic amino acid Y; amino acids N and Q containing amide groups in the side chain are mutated into amino acids G, S, D and E; non-aromatic amino acids T and S with hydroxyl groups in the side chain are mutated into G, L and R. The reason to mutate aromatic amino acids Y, W and F into amino acids G, A and S with relatively small side chains is because aromatic amino acids often participate in the hydrophobic interaction between molecules through the structure of hydrophobic benzene ring in the side chain, and the side chain occupies a large space, so the mutation of them into amino acids with relatively small side chains may potentially change the interaction between antibody and antigen;

The reason to mutate positively charged amino acids R, K and H into amino acids G, A and S with relatively small side chains is because positively charged amino acids often participate in the charge interaction between molecules through the positive charge in the side chain, and the side chain of R\K occupies a large space, so mutation into amino acids with relatively small side chain may potentially change the interaction between antibody and antigen;

The reason to mutate amino acid G with side chain of hydrogen atom into aromatic amino acid Y is because the side chain of amino acid G occupies the smallest space among all amino acids. Mutation into amino acid Y with larger side chain may potentially change the interaction between antibody and antigen due to increased steric hindrance;

The reason to mutate amino acids N and Q with amide group in the side chain to amino acids G, S, D and E is because the structure of amino acids N\Q with amide group in the side chain is similar to that of D\E. Mutation of N\Q to D\E may slightly weaken the interaction between antibody and antigen without complete destruction. The purpose of mutation to G and S is to directly reduce the side chain, which may potentially change the interaction between antibody and antigen;

The reason to mutate non-aromatic amino acids T and S containing hydroxyl groups in the side chain into G, L and R is because the side chain of amino acid T\S contained hydroxyl groups. The mutation into G with the smallest side chain, hydrophobic amino acid L with the middle side chain, and amino acid R with the larger side chain and positive charge, respectively, is to change the interaction between antibody and antigen in different degrees.

Table 3 Table of amino acid mutation

| Target amino acid | Mutated amino acid |
| --- | --- |
| Y,W, F | G, A, S |
| R, K, H | G, A, S |
| G | Y |
| N, Q | G, S, D, E |
| T, S | G, L, R |

The variable region sequence of the light and heavy chain of antibody obtained as described above is combined with each other. See Table 4 for the specific combination. The human IgG1 L234AL235A sequence (SEQ ID NO: 100) is selected as the heavy chain constant region, and the corresponding constant region CL-Kappa (SEQ I DNO: 102) and CL-Lambda (SEQ ID NO: 101) is selected as the light chain constant region according to whether the variable region is Kappa or Lambda. Then the heavy chain sequence and light chain sequence of the antibody were respectively constructed into the expression vector pcDNA3.1 (Invitrogen, V790-20), and transiently transfected with Expi293 cells (Invitrogen, A14527) to obtain the corresponding humanized antibody. The specific transfection process is the same as Example 2.

Table 4 Table of light and heavy chain combination of sp34 humanized CDR mutant antibody

| protein ID | VH | SEQ ID NO | HCDR1 SEQ ID NO | HCDR2 SEQ ID NO | HCDR3 SEQ ID NO | VL | SEQ ID NO | LCDR1 SEQ ID NO | LCDR2 SEQ ID NO | LCDR3 SEQ ID NO |
|---|---|---|---|---|---|---|---|---|---|---|
| hzsp34.78 | husp34h.g2.1 | 52 | 4 | 2 | 8 | husp34l.g3 | 80 | 29 | 30 | 31 |
| hzsp34.79 | husp34h.g2.2 | 53 | 5 | 2 | 8 | husp34l.g3 | 80 | 29 | 30 | 31 |
| hzsp34.80 | husp34h.g2.3 | 54 | 6 | 2 | 8 | husp34l.g3 | 80 | 29 | 30 | 31 |
| hzsp34.81 | husp34h.g2.4 | 55 | 1 | 7 | 8 | husp34l.g3 | 80 | 29 | 30 | 31 |
| hzsp34.82 | husp34h.g2.5 | 56 | 1 | 9 | 8 | husp34l.g3 | 80 | 29 | 30 | 31 |
| hzsp34.83 | husp34h.g2.6 | 57 | 1 | 10 | 8 | husp34l.g3 | 80 | 29 | 30 | 31 |
| hzsp34.84 | husp34h.g2.7 | 58 | 1 | 11 | 8 | husp34l.g3 | 80 | 29 | 30 | 31 |
| hzsp34.85 | husp34h.g2.8 | 59 | 1 | 12 | 8 | husp34l.g3 | 80 | 29 | 30 | 31 |
| hzsp34.86 | husp34h.g2.9 | 60 | 1 | 2 | 13 | husp34l.g3 | 80 | 29 | 30 | 31 |
| hzsp34.87 | husp34h.g2.10 | 61 | 1 | 2 | 14 | husp34l.g3 | 80 | 29 | 30 | 31 |
| hzsp34.88 | husp34h.g2.11 | 62 | 1 | 2 | 15 | husp34l.g3 | 80 | 29 | 30 | 31 |
| hzsp34.89 | husp34h.g2.12 | 63 | 1 | 2 | 16 | husp34l.g3 | 80 | 29 | 30 | 31 |
| hzsp34.90 | husp34h.g2.13 | 64 | 1 | 2 | 17 | husp34l.g3 | 80 | 29 | 30 | 31 |
| hzsp34.91 | husp34h.g2.14 | 65 | 1 | 2 | 18 | husp34l.g3 | 80 | 29 | 30 | 31 |
| hzsp34.92 | husp34h.g2.15 | 66 | 1 | 2 | 19 | husp34l.g3 | 80 | 29 | 30 | 31 |
| hzsp34.93 | husp34h.g2.16 | 67 | 1 | 2 | 20 | husp34l.g3 | 80 | 29 | 30 | 31 |
| hzsp34.94 | husp34h.g2.17 | 68 | 1 | 2 | 21 | husp34l.g3 | 80 | 29 | 30 | 31 |
| hzsp34.95 | husp34h.g2.18 | 69 | 22 | 2 | 8 | husp34l.g3 | 80 | 29 | 30 | 31 |
| hzsp34.96 | husp34h.g2.19 | 70 | 1 | 23 | 8 | husp34l.g3 | 80 | 29 | 30 | 31 |

(continued)

| protein ID | VH | SEQ ID NO | HCDR1 SEQ ID NO | HCDR2 SEQ ID NO | HCDR3 SEQ ID NO | VL | SEQ ID NO | LCDR1 SEQ ID NO | LCDR2 SEQ ID NO | LCDR3 SEQ ID NO |
|---|---|---|---|---|---|---|---|---|---|---|
| hzsp34. 97 | husp34h.g 2.20 | 71 | 1 | 24 | 8 | husp34l.g 3 | 80 | 29 | 30 | 31 |
| hzsp34. 98 | husp34h.g 2.21 | 72 | 1 | 2 | 25 | husp34l.g 3 | 80 | 29 | 30 | 31 |
| hzsp34. 99 | husp34h.g 2.22 | 73 | 1 | 2 | 26 | husp34l.g 3 | 80 | 29 | 30 | 31 |
| hzsp34. 100 | husp34h.g 2.23 | 74 | 1 | 2 | 27 | husp34l.g 3 | 80 | 29 | 30 | 31 |
| hzsp34. 101 | husp34h.g 2.24 | 75 | 1 | 2 | 28 | husp34l.g 3 | 80 | 29 | 30 | 31 |
| hzsp34. 102 | husp34h.g 2 | 50 | 1 | 2 | 8 | husp34lg 3.1 | 85 | 32 | 30 | 31 |
| hzsp34. 103 | husp34h.g 2 | 50 | 1 | 2 | 8 | husp34lg 3.2 | 86 | 33 | 30 | 31 |
| hzsp34. 104 | husp34h.g 2 | 50 | 1 | 2 | 8 | husp34lg 3.3 | 87 | 34 | 30 | 31 |
| hzsp34. 105 | husp34h.g 2 | 50 | 1 | 2 | 8 | husp34lg 3.4 | 88 | 35 | 30 | 31 |
| hzsp34. 106 | husp34h.g 2 | 50 | 1 | 2 | 8 | husp34lg 3.5 | 89 | 36 | 30 | 31 |
| hzsp34. 107 | husp34h.g 2 | 50 | 1 | 2 | 8 | husp34lg 3.6 | 90 | 29 | 30 | 37 |
| hzsp34. 108 | husp34h.g 2 | 50 | 1 | 2 | 8 | husp34lg 3.7 | 91 | 29 | 30 | 38 |
| hzsp34. 109 | husp34h.g 2 | 50 | 1 | 2 | 8 | husp34lg 3.8 | 92 | 29 | 30 | 39 |
| hzsp34. 110 | husp34h.g 2 | 50 | 1 | 2 | 8 | husp34lg 3.9 | 93 | 29 | 30 | 40 |
| hzsp34. 111 | husp34h.g 2 | 50 | 1 | 2 | 8 | husp34lg 3.10 | 94 | 41 | 30 | 31 |
| hzsp34. 112 | husp34h.g 2 | 50 | 1 | 2 | 8 | husp34lg 3.11 | 95 | 42 | 30 | 31 |
| hzsp34. 113 | husp34h.g 2 | 50 | 1 | 2 | 8 | husp34lg 3.12 | 96 | 29 | 30 | 43 |
| hzsp34. 114 | husp34h.g 2 | 50 | 1 | 2 | 8 | husp34lg 3.13 | 97 | 29 | 30 | 44 |
| hzsp34. 115 | husp34h.g 2 | 50 | 1 | 2 | 8 | husp34lg 3.14 | 98 | 29 | 45 | 31 |
| hzsp34. 116 | husp34h.g 2 | 50 | 1 | 2 | 8 | husp34lg 3.15 | 99 | 29 | 45 | 46 |

5.2 Affinity test and T cell activation function test of mutant

The affinity test of the humanized CDR mutant of Sp34 and the protein level of human CD3 was performed using the Biolayer Interferometry (BLI). The specific method is the same as Example 3.1. The results are shown in Table 5. The affinity level of the antibody to human CD3E&G complex (Sino Biological Inc., CT041-H0305H) is $1.11\times10^{-8}$ M~$9.50\times10^{-9}$ M, the affinity level to human CD3E&D complex (Sino Biological Inc., CT026H0323H) is $1.03\times10^{-8}$ M~$9.77\times10^{-10}$ M.

Table 5 Affinity of sp34 humanized CDR mutant antibody to human CD3

| Protein ID | human CD3E&G affinity | | | human CD3E&D affinity | | |
|---|---|---|---|---|---|---|
| | KD (M) | kon(1/Ms) | kdis(1/s) | KD (M) | kon(1/Ms) | kdis(1/s) |
| sp34 | 6.22E-09 | 2.40E+05 | 1.49E-03 | 6.38E-10 | 2.46E+05 | 1.57E-04 |
| hzsp34.24 | 8.00E-09 | 2.24E+05 | 1.79E-03 | 9.75E-10 | 2.40E+05 | 2.34E-04 |
| hzsp34.78 | 8.97E-09 | 2.50E+05 | 2.24E-03 | 1.35E-09 | 2.23E+05 | 3.00E-04 |
| hzsp34.79 | 6.36E-09 | 2.42E+05 | 1.54E-03 | 5.07E-10 | 2.33E+05 | 1.18E-04 |
| hzsp34.80 | 1.54E-08 | 1.38E+05 | 2.13E-03 | 6.55E-09 | 2.25E+05 | 1.47E-03 |
| hzsp34.81 | 1.11E-08 | 1.42E+05 | 1.57E-03 | 7.69E-09 | 1.86E+05 | 1.43E-03 |
| hzsp34.82 | 1.20E-08 | 1.59E+05 | 1.91E-03 | 1.03E-08 | 1.63E+05 | 1.68E-03 |
| hzsp34.83 | 3.60E-09 | 2.20E+05 | 7.91E-04 | 9.28E-10 | 2.16E+05 | 2.00E-04 |
| hzsp34.84 | 4.89E-09 | 2.33E+05 | 1.14E-03 | 8.12E-10 | 2.46E+05 | 2.00E-04 |
| hzsp34.85 | 8.08E-09 | 2.49E+05 | 2.01E-03 | 1.32E-09 | 2.30E+05 | 3.04E-04 |
| hzsp34.86 | 6.61E-09 | 2.19E+05 | 1.44E-03 | 1.39E-09 | 2.55E+05 | 3.54E-04 |
| hzsp34.87 | 6.06E-09 | 1.65E+05 | 9.97E-04 | 8.80E-10 | 1.73E+05 | 1.52E-04 |
| hzsp34.88 | 1.58E-08 | 1.30E+05 | 2.05E-03 | 4.55E-09 | 1.75E+05 | 7.97E-04 |
| hzsp34.89 | 1.38E-08 | 2.05E+05 | 2.82E-03 | 6.62E-09 | 1.42E+05 | 9.40E-04 |
| hzsp34.91 | 8.53E-09 | 2.28E+05 | 1.95E-03 | 1.88E-09 | 2.80E+05 | 5.28E-04 |
| hzsp34.92 | 4.82E-09 | 2.10E+05 | 1.01E-03 | 7.72E-10 | 2.59E+05 | 2.00E-04 |
| hzsp34.93 | 3.42E-09 | 2.45E+05 | 8.36E-04 | 9.76E-10 | 2.66E+05 | 2.60E-04 |
| hzsp34.94 | 4.63E-09 | 1.77E+05 | 8.21E-04 | 9.77E-10 | 2.05E+05 | 2.00E-04 |
| hzsp34.95 | N.B | | | N.B | | |
| hzsp34.96 | N.B | | | N.B | | |
| hzsp34.97 | 1.10E-08 | 1.35E+05 | 1.48E-03 | 5.62E-09 | 2.78E+05 | 1.56E-03 |
| hzsp34.98 | N.B | | | 9.15E-09 | 2.01E+05 | 1. 84E-03 |
| hzsp34.99 | N.B | | | 9.63E-09 | 1. 14E+05 | 1.10E-03 |
| hzsp34.100 | 8.33E-09 | 2.05E+05 | 1.71E-03 | 1.13E-09 | 2.43E+05 | 2.76E-04 |
| hzsp34.101 | 4.30E-09 | 1.48E+05 | 6.36E-04 | 3.77E-09 | 2.17E+05 | 8.20E-04 |
| hzsp34.102 | 8.52E-09 | 2.22E+05 | 1.90E-03 | 1.69E-09 | 2.50E+05 | 4.23E-04 |
| hzsp34.103 | 7.57E-09 | 2.53E+05 | 1.91E-03 | 1.34E-09 | 2.43E+05 | 3.25E-04 |
| hzsp34.104 | 8.54E-09 | 2.31E+05 | 1.98E-03 | 1.93E-09 | 2.81E+05 | 5.42E-04 |
| hzsp34.105 | 1.06E-08 | 1.99E+05 | 2.10E-03 | 1.30E-09 | 2.35E+05 | 3.07E-04 |
| hzsp34.106 | 1.03E-08 | 2.30E+05 | 2.37E-03 | 1.54E-09 | 2.36E+05 | 3.65E-04 |
| hzsp34.107 | 5.98E-08 | 7.46E+04 | 4.46E-03 | 8.44E-09 | 1.67E+05 | 1.41E-03 |

(continued)

| Protein ID | human CD3E&G affinity | | | human CD3E&D affinity | | |
|---|---|---|---|---|---|---|
| | KD (M) | kon(1/Ms) | kdis(1/s) | KD (M) | kon(1/Ms) | kdis(1/s) |
| hzsp34.108 | 7.19E-09 | 2.03E+05 | 1.46E-03 | 9.65E-10 | 2.07E+05 | 2.00E-04 |
| hzsp34.109 | 9.02E-09 | 2.33E+05 | 2.10E-03 | 1. 15E-09 | 2.25E+05 | 2.58E-04 |
| hzsp34.110 | 8.49E-09 | 2.22E+05 | 1.88E-03 | 9.51E-10 | 2.10E+05 | 2.00E-04 |
| hzsp34.111 | 9.50E-09 | 2.01E+05 | 1.91E-03 | 1.94E-09 | 2.35E+05 | 4.54E-04 |
| hzsp34.112 | 6.60E-09 | 2.35E+05 | 1.55E-03 | 7.22E-10 | 2.82E+05 | 2.04E-04 |
| hzsp34.113 | 3.71E-09 | 2.19E+05 | 8.11E-04 | 1.02E-09 | 1.96E+05 | 2.00E-04 |
| hzsp34.114 | 6.69E-09 | 2.00E+05 | 1.34E-03 | 1.08E-09 | 2.62E+05 | 2.82E-04 |
| hzsp34.115 | 7.70E-09 | 2.38E+05 | 1. 84E-03 | 1.26E-09 | 2.64E+05 | 3.31E-04 |
| hzsp34.116 | 1.60E-08 | 1.79E+05 | 2.86E-03 | 4.11E-09 | 2.23E+05 | 9.14E-04 |

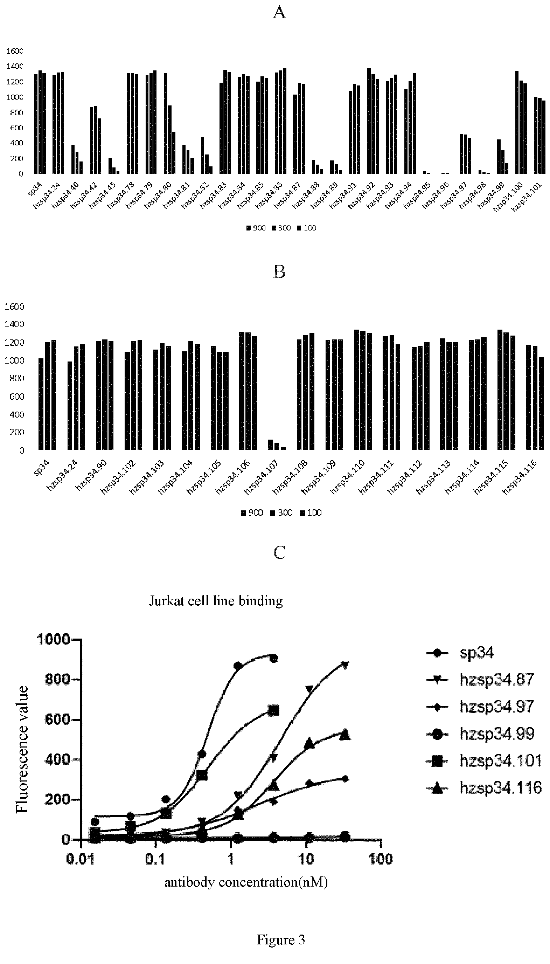

The affinity test method of the humanized CDR mutant of Sp34 to CD3 at the cell-level is the same as that of Example 3.2. The results are shown in Figure 3A and Figure 3B. Compared with the antibody of Sp34, the affinity of hzsp34.80, hzsp34.81, hzsp34.82, hzsp34.87, hzsp34.88, hzsp34.89, hzsp34.91, hzsp34.97, hzsp34.99, hzsp34.101, hzsp34.107 and hzsp34.116 decreased with different degrees, except for hzsp34.40, hzsp34.42 and hzsp34.45. Some clones were selected for binding at Jurkat cell level under more antibody concentration gradients. The results are shown in Figure 3C, showing the different affinity of different CDR mutants to CD3.

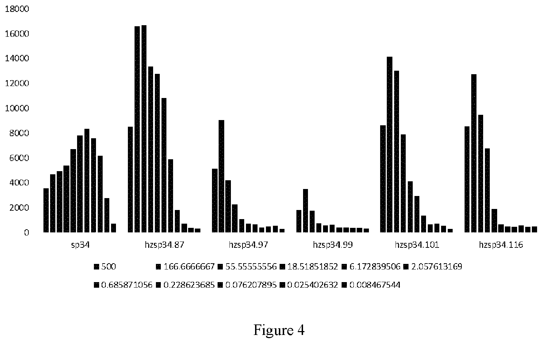

The T cell activation function of the Sp34 humanized CDR mutant was detected using Jurkat NFAT cells. The detection method was the same as that of Example 4. The results are shown in Figure 4. The CDR mutant of the Sp34 humanized antibody can all activate the downstream signal pathway of T cells, but the activation degree is different.

Example 6 Activity test of Her2×CD3 bispecific antibody *in vitro*

In order to test the monovalent affinity of sp34 humanized antibody and the activation ability of tumor-associated antigen-dependent T cells, the bispecific antibody molecule targeting Her2×CD3 in "1+1" form was constructed and expressed, in which the variable region sequence of anti-Her2 is from the marketed drug trastuzumab, and the heavy chain variable region sequence is EVQLUESGGGLUQPGGSLRL SCAASGFNIKDTYIHW VRQAPGKGLEW VARIY PTNGYTRYADSVKGRFTISADTSKNTAYLQMNSLRAEDTAVYYCSRWGGDGF YAMDYWGQGTLVTV(SEQ ID NO:114); the light chain variable region sequence is DIQMTQSPSSLSASVGDRVTITCRASQDVNTAVAWYQQKPGKAPKLLIYSASFL YSGVPSRFSGSRSGTDFTLTISSLQPEDFATYYCQQHYTTPPTFGQGTKVEIK (SEQ ID NO:115). The variable region sequence of anti-CD3 uses the variable region sequence of sp34 humanized antibody (hzsp34.24, hzsp34.80, hzsp34.87, hzsp34.97, hzsp34.99, hzsp34.101). The IgG1LALA sequence of Knob-into-hole (A. Margaret Merchant et al., Nature Biotechnology, 1998) is selected as the Fc segment of the antibody (the sequence of each domain is as follows). The schematic diagram of the antibody is shown in Figure 5A.

CL:

RTVAAPSVFIFPPSDEQLKSGTASVVCLLNNFYPREAKVQWKVDNALQSGNS QESVTEQDSKDSTYSLSSTLTLSKADYEKHKVYACEVTHQGLSSPVTKSFNRG

EC* （SEQ ID NO:116）

CH1+CH2+CH3 (knob):

ASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKVEPKSCDKTH
TCPPCPAPEAAGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKA
LPAPIEKTISKAKGQPREPQVYTLPPSRDELTKNQVSLWCLVKGFYPSDIAVEW
ESNGQPENNYKTTPPVLDSDGSFFLYSKLTVDKSRWQQGNVFSCSVMHEALH
NHYTQKSLSLSPGK*(SEQ ID NO:117)

CH1+CH2+CH3 (hole):

ASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKVEPKSCDKTH
TCPPCPAPEAAGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKA
LPAPIEKTISKAKGQPREPQVYTLPPSRDELTKNQVSLSCAVKGFYPSDIAVEW
ESNGQPENNYKTTPPVLDSDGSFFLVSKLTVDKSRWQQGNVFSCSVMHEALH
NHYTQKSLSLSPGK*(SEQ ID NO:118)

The CD3 equilibrium dissociation constant (KD) of Her2×CD3 bispecific antibody was determined using Biacore (GE Healthcare, T200), and the specific method is as follows:
The human CD3E&G antigen (Sino Biological Inc., CT041-H0305H) and Cynomolgus monkey CD3E&D antigen protein (Sino Biological Inc., CT032-C0323H) were coupled to the chip surface. Afterwards, the affinity and kinetic constants were obtained by detecting the binding and dissociation between the chip surface antigen and the antibody in the mobile phase. The method includes chip preparation and affinity detection. 10-fold diluted 10× HBS-EP+(BR-1006-69, GE Healthcare) was used as the experimental buffer during measurement. The amino coupling kit (BR-1006-33, GE Healthcare) was used in the chip preparation process to couple human CD3E&G antigen and cynomolgus monkey CD3E&D antigen on the surface of CM5 chip (29-1496-03, GE Healthcare), and the coupling level was not more than 100RU to avoid over-strong affinity caused by too high coupling density. After coupling, 1 M ethanolamine was injected to block the remaining activation sites. Affinity detection cycle is carried out for each concentration of antibody, and each cycle includes binding to antibody with this concentration and chip regeneration. The antibody after serial dilution (Her2-sp34.24: initial concentration 32nM, 2 folds serial dilution; Her2-sp34.87: initial concentration 200nM, 2 folds serial dilution; Her2-sp34.80, Her2-sp34.97, Her2-sp34.99, Her2-sp34.101: initial concentration 800nM, 2 folds serial dilution; all dilute 5 concentration points), flows through the chip surface from low concentration to high concentration at 30 μl/min of the flow rate, with a binding time of 180 s and a dissociation time of 600s. Finally, 10 mM Glycine pH 1.5 (BR-1003-54, GE Healthcare) was used to regenerate the chip. The data results were analyzed using the Biacore T200 analysis software (version 3.1), and the analysis model used was 1:1 combined model for dynamic analysis. The test results are shown in Table 6, the monovalent sp34 humanized antibody in Her2 × CD3 bispecific antibody and its CDR mutant showed different affinity gradients to human and cynomolgus monkey CD3.

Table 6 Affinity between sp34 humanized antibody and CD3 in Her2 × CD3 bispecific antibody

| Name of antibody | Affinity to human CD3E&G | | | Affinity to cynomolgus monkey CD3E&D | | |
| --- | --- | --- | --- | --- | --- | --- |
| | Ka(1/Ms) | Kd(1!s) | KD(M) | Ka(1/Ms) | Kd(1/s) | KD(M) |
| Her2-hzsp34.24 | 1.60E+06 | 7.79E-03 | 4.88E-09 | 1.11E+06 | 8.00E-03 | 7.20E-09 |
| Her2-hzsp34.80 | 4.97E+05 | 4.91E-01 | 9.87E-07 | 5.96E+05 | 7.80E-03 | 1.31E-06 |
| Her2-hzsp34.87 | 3.27E+05 | 4.62E-02 | 1.41E-07 | 1.45E+05 | 3.50E-02 | 2.42E-07 |
| Her2-hzsp34.97 | 5.28E+05 | 1.91E-01 | 3.61E-07 | 5.43E+05 | 1.77E-01 | 3.27E-07 |
| Her2-hzsp34.99 | 1.30E+05 | 8.17E-02 | 6.27E-07 | 8.34E+04 | 1.00E-01 | 1.20E-06 |
| Her2-hzsp34.101 | 6.37E+05 | 1.01E-01 | 1.59E-07 | 5.51E+05 | 9.87E-02 | 1.79E-07 |

**EP 4 223 777 A1**

To verify T cell activation ability of Her2 × CD3 bispecific antibody, the luciferase report system of Jurkat NFAT (Nuclear Factor of Activated T) cells (J1621, Promega) was used to detect the T cell activation ability of bispecific antibody molecules. The cell line activated the expression of luciferase through the NFAT signal pathway of TCR/CD3 intracellular downstream signal, thus detecting the activation of T cells.

The specific method is as follows: In 96-well white flat-bottom cell culture plate, $8 \times 10^5$ target cells SK-BR-3 (JCRB0834, JCRB cell bank) and $4 \times 10^6$ effector cell Jurkat NFAT cells were added into each well, and then Her2×CD3 bispecific antibody molecules at corresponding concentration (Her2-sp34.24, Her2-sp34.87 and Her2-sp34.101 have an initial concentration of 10nM, 4 folds dilution; Her2-sp34.80 has an initial concentration of 200nM, 4 folds dilution; Her2-sp34.97 and Her2-sp34.99 have an initial concentration of 100nM, 4 folds dilution) were added, and then cultured in incubator at 37 °C for 16 hours. Then the culture plate was taken out and Bio-Glo (G7940, Promega) was added. The microplate reader (Spectra, Molecular Devices) was used for wavelength detection. Results are shown in Figure 5B, Her2-sp34.24, Her2-sp34.87, Her2-sp34.101, Her2-sp34.97, Her2-sp34.99, Her2-sp34.80 have the activation ability of T cells from high to low.

**Example 7 CD70/CD3 bispecific antibody activity test *in vitro***

The bispecific antibody in "1+1" form targeting CD70 × CD3 was also constructed and expressed in the invention, wherein the variable region sequence of anti-CD70 is from SGN70 (SEQ ID NO: 14 and SEQ ID NO: 24 in WO2004073656), and the variable region sequence of anti-CD3 is from sp34 humanized antibody variable region sequence, and adopts scFv form (Figure 6A). The sequence of each type of sp34 adopts the sequence of "heavy chain-light chain (HL)" and "light chain-heavy chain (LH)" respectively (where sp34.24LH represents the sequence of light chain-heavy chain, sp34.24HL represents the sequence of heavy chain-light chain, and other molecules are the same), which is connected by (GGGGS)$_4$. The IgG1 LALA sequence of Knob-into-hole (A. Margaret Merchant et al., Nature Biotechnology, 1998) was used as Fc segment. The structure schematic of the antibody is shown in Figure 6A, wherein CH3 of Knob has point mutation Y349C (EU numbering system), and CH3 of Hole has point mutation S354C (EU numbering system). The sequence is as follows:

CL: SEQ ID NO: 116

CH1+CH2+CH3 (knob):

ASTKGPSVFPLAPSSKSTSGGTAALGCLVKDYFPEPVTVSWNSGALTSGVHTFP AVLQSSGLYSLSSVVTVPSSSLGTQTYICNVNHKPSNTKVDKKVEPKSCDKTH TCPPCPAPEAAGGPSVFLFPPKPKDTLMISRTPEVTCVVVDVSHEDPEVKFNW YVDGVEVHNAKTKPREEQYNSTYRVVSVLTVLHQDWLNGKEYKCKVSNKA LPAPIEKTISKAKGQPREPQVYTLPPCRDELTKNQVSLWCLVKGFYPSDIAVEW ESNGQPENNYKTTPPVLDSDGSFFLYSKLTVDKSRWQQGNVFSCSVMHEALH NHYTQKSLSLSPGK* (SEQ ID NO:119)

CH1+CH2+CH3 (hole):

ASTKGPSVFPLAPSSKSTSGGTAALGCLVKDYFPEPVTVSWNSGALTSGVHTFP AVLQSSGLYSLSSVVTVPSSSLGTQTYICNVNHKPSNTKVDKKVEPKSCDKTH TCPPCPAPEAAGGPSVFLFPPKPKDTLMISRTPEVTCVVVDVSHEDPEVKFNW YVDGVEVHNAKTKPREEQYNSTYRVVSVLTVLHQDWLNGKEYKCKVSNKA LPAPIEKTISKAKGQPREPQVCTLPPSRDELTKNQVSLSCAVKGFYPSDIAVEW ESNGQPENNYKTTPPVLDSDGSFFLVSKLTVDKSRWQQGNVFSCSVMHEALH NHYTQKSLSLSPGK* (SEQ ID NO:120)

The CD3 equilibrium dissociation constant (KD) of each bispecific antibody was determined using Biacore (GE

34

Healthcare, T200). The method is same as that in Example 6, and the measured results are shown in Table 7. Some bispecific antibodies SGN70-sp34.80HL and SGN70-sp34.99HL have low affinity due to structural changes.

Table 7 Affinity to CD3 of sp34 humanized antibody in the form of scFv in SGN70×CD3 bispecific antibody

| Name of antibody | Affinity to human CD3E&G | | | Affinity to cynomolgus monkey CD3E&D | | |
|---|---|---|---|---|---|---|
| | Ka(1/Ms) | Kd(1!s) | KD(M) | Ka(1/Ms) | Kd(1/s) | KD(M) |
| SGN70-sp34.24LH | 1.50E+05 | 1.46E-02 | 9.80E-08 | 1.13E+05 | 1.58E-02 | 1.41E-07 |
| SGN70-sp34.24HL | 2.65E+05 | 2.90E-02 | 1.10E-07 | 1.69E+05 | 3.02E-02 | 1.79E-07 |
| SGN70-sp34.80LH | 4.97E+04 | 2.01E-01 | 4.06E-06 | 1.15E+05 | 7.49E-01 | 6.49E-06 |
| SGN70-sp34.80HL | N.B | N.B | N.B | N.B | N.B | N.B |
| SGN70-sp34.87LH | 3.21E+05 | 1.07E-01 | 3.35E-07 | 8.57E+04 | 4.66E-02 | 5.44E-07 |
| SGN70-sp34.87HL | 5.04E+05 | 2.53E-01 | 5.01E-07 | 3.08E+05 | 1.95E-01 | 6.33E-07 |
| SGN70-sp34.97LH | 8.13E+04 | 2.53E-01 | 3.11E-06 | 6.38E+04 | 3.33E-01 | 5.22E-06 |
| SGN70-sp34.97HL | 7.99E+04 | 4.16E-01 | 5.20E-06 | 1.28E+05 | 7.05E-01 | 5.50E-06 |
| SGN70-sp34.99LH | 1.23E+05 | 9.93E-01 | 8.06E-06 | 1.49E+04 | 2.17E-01 | 1.46E-05 |
| SGN70-sp34.99HL | N.B | N.B | N.B | N.B | N.B | N.B |
| SGN70-sp34.101LH | 2.90E+05 | 1.19E-01 | 4.09E-07 | 2.35E+05 | 1.44E-01 | 6.14E-07 |
| SGN70-sp34.101HL | 3.47E+05 | 1.67E-01 | 4.80E-07 | 2.69E+05 | 1.77E-01 | 6.58E-07 |

The reporter system of Jurkat NFAT cells was used for detection. The specific method is as follows: In 96-well white flat-bottomed cell culture plate, $8\times10^5$ target cell NOMO-1 (CBP60515, Nanjing Cobioer Biosciences co., LTD) and $4\times10^6$ effector cells Jurkat NFAT cell were added into each well, and then CD70×CD3 bispecific antibody molecule at corresponding concentration(with initial concentration of 100mM and 5 folds dilution) was added and then cultured in 37°C incubator for 16 hours. Then the culture plate was taken out, Bio-Glo (G7940, Promega) was added. The microplate reader (Spectra, Molecular Devices) was used for wavelength detection. Results are shown in Figure 6B. Some bispecific antibodies SGN70-sp34.24LH, SGN70-sp34.24HL, SGN70-sp34.87LH, SGN70-sp34.87HL, SGN70-sp34.101HL and SGN70-sp34.101LH have strong T cell activation ability, while some bispecific antibodies such as SGN70-sp34.80HL, SGN70-sp34.80LH, SGN70-sp34.97HL, SGN70-sp34.97LH, SGN70-sp34.99HL and SGN70-sp34.99LH have relatively weak T cell activation, which may be related to the low affinity of SGN70 antibody at the end of CD70 and the low CD70 abundance on the surface of NOMO-1 cells.

**Example 8 Construction and preparation of CD3/Claudin18.2 bispecific antibody**

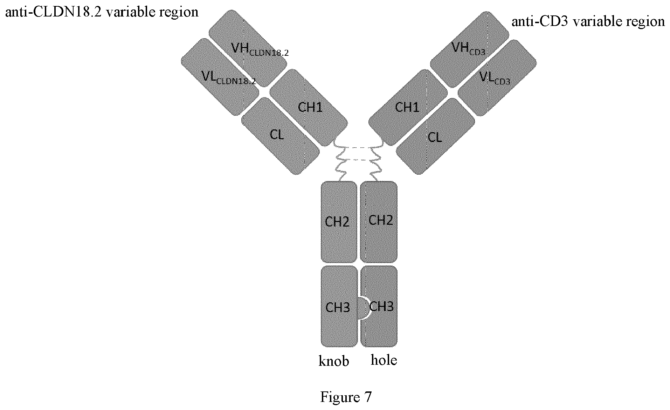

The sequence of the antigen-binding region of anti-Claudin18.2 (CLDN18.2) monoclonal antibody HB37A6 (see CN202010570517.X, and the sequence information is shown in Table 9) and of the three different anti-human CD3 monoclonal antibodies HzSP34.24, HzSP34.87, and HzSP34.97 were respectively used to construct bispecific antibodies 030, 032 and 033 in the "1+1" form targeting CLDN18.2×CD3 according to the combination in Table 8, and their antibody structure schematics are shown in Figure 7. Specifically, the IgG1 LALA sequence of knob-into-hole structure (A. Margaret Merchant et al., Nature Biotechnology, 1998) was selected as the Fc segment of the antibody. Therefore, the heavy chain at the CLDN18.2 end of the bispecific antibody is SEQ ID NO: 121, and the light chain is SEQ ID NO: 122. The heavy chain at the CD3 end of the bispecific antibody is SEQ ID NO: 123, 125 and 126, and the light chain is SEQ ID NO: 124.

The plasmid construction process of bispecific antibody is as follows: the heavy chain sequence of CLDN18.2 (SEQ ID NO: 121), the light chain sequence of CLDN18.2 (SEQ ID NO: 122), the heavy chain sequence of CD3 (SEQ ID NO: 123, 125 and 126), and the light chain sequence of CD3 (SEQ ID NO: 124) were inserted into the vector pcDNA3.1 (Invitrogen, V790-20) to obtain the plasmid for the heavy chain of the end of CLDN18.2, for the light chain plasmid of the end of CLDN18.2 , and for the heavy chain of the end of CD3 and for the light chain of the end of CD3. Then PEI (Polysciences, 23966) was used to transiently transfect the plasmid for the heavy chain of the end of CLDN18.2 plasmid, for the light chain plasmid of the end of CLDN18.2 at the end of CLDN18.2, and for the heavy chain plasmid of the end of CD3 and for the light chain of the end of CD3 into Expi293 cells (Invitrogen, A14527), and the semi-antibody molecule of CLDN18.2 end and three semi-antibody molecules of CD3 end were expressed. After 7 days, the cell fermentation broth was obtained, filtered and clarified, and captured with the Protein A column (GE Healthcare, 11-0034-95) of Hitrap Mabselect Sure respectively, to obtain the semi-antibody at the end of CLDN18.2 and CD3. After the concentration of the semiantibody was detected by A280 method, the semi-antibodies at both ends were mixed in a ratio of 1:1. An appropriate amount of reducing agent GSH were added and the reaction was overnight at room temperature. Ultrafiltration removes the reducing agent and terminates the reaction. After that, MonoS cation exchange chromatography (GE Healthcare, 17-5168-01) was used for fine purification. Liquid A was 20 mM sodium phosphate buffer (pH 6.6), and liquid B was 20 mM sodium phosphate buffer (pH 6.6) containing 1M sodium chloride. The elution gradient was 0%-50% (30 column volume). The eluted protein solution was ultrafiltration and transferred to PBS (Gibco, 70011-044). The molecular weight was determined by mass spectrometry and the purity was identified by SEC-HPLC. The obtained 030, 032 and 033 bispecific antibodies are used in the following embodiments.

Table 8. List of CD3/CLDN18.2 bispecific antibodies

|  | Anti-CLDN18.2 end | Anti-CD3 end |
|---|---|---|
| 030 | HB37A6 | HzSP34.24 |
| 032 | HB37A6 | HzSP34.87 |
| 033 | HB37A6 | HzSP34.97 |

**Table 9: Bispecific antibody information**

| SEQ ID NO |  | Knob (CH1+CH2+CH3) |
|---|---|---|
| 117 |  | ASTKGPSVFPLAPSSKSTSGGTAALGCLVKDYFPEPVTVSWNS GALTSGVHTFPAVLQSSGLYSLSSVVTVPSSSLGTQTYICNVNH KPSNTKVDKKVEPKSCDKTHTCPPCPAPEAAGGPSVFLFPPKP KDTLMISRTPEVTCVVVDVSHEDPEVKFNWYVDGVEVHNAK TKPREEQYNSTYRVVSVLTVLHQDWLNGKEYKCKVSNKALPA PIEKTISKAKGQPREPQVYTLPPSRDELTKNQVSLWCLVKGFYP SDIAVEWESNGQPENNYKTTPPVLDSDGSFFLYSKLTVDKSRW QQGNVFSCSVMHEALHNHYTQKSLSLSPGK |
| SEQ ID NO |  | Hole (CH1+CH2+CH3) |
| 118 |  | ASTKGPSVFPLAPSSKSTSGGTAALGCLVKDYFPEPVTVSWNS GALTSGVHTFPAVLQSSGLYSLSSVVTVPSSSLGTQTYICNVNH KPSNTKVDKKVEPKSCDKTHTCPPCPAPEAAGGPSVFLFPPKP KDTLMISRTPEVTCVVVDVSHEDPEVKFNWYVDGVEVHNAK TKPREEQYNSTYRVVSVLTVLHQDWLNGKEYKCKVSNKALPA PIEKTISKAKGQPREPQVYTLPPSRDELTKNQVSLSCAVKGFYP SDIAVEWESNGQPENNYKTTPPVLDSDGSFFLVSKLTVDKSRW QQGNVFSCSVMHEALHNHYTQKSLSLSPGK |
| SEQ ID NO |  | CLDN18.2 end HB37A6 in bispecific antibody |

(continued)

| SEQ ID NO | | Knob (CH1+CH2+CH3) |
|---|---|---|
| 121 | Heavy chain | EVQLLDSGGGLVQPGGSLRLSCAASGFTFSSYVMSWVRQAPG KGLNWVSTISHSGGSTYYADSVKGRFTISRDNSKNTLYLQMNS LRAEDTAVYYCAIDAPYYDILTGYRYWGQGTLVTVSSASTKGP SVFPLAPSSKSTSGGTAALGCLVKDYFPEPVTVSWNSGALTSG VHTFPAVLQSSGLYSLSSVVTVPSSSLGTQTYICNVNHKPSNTK VDKKVEPKSCDKTHTCPPCPAPEAAGGPSVFLFPPKPKDTLMIS RTPEVTCVVVDVSHEDPEVKFNWYVDGVEVHNAKTKPREEQ YNSTYRVVSVLTVLHQDWLNGKEYKCKVSNKALPAPIEKTISK AKGQPREPQVYTLPPSRDELTKNQVSLWCLVKGFYPSDIAVEW ESNGQPENNYKTTPPVLDSDGSFFLYSKLTVDKSRWQQGNVFS |
| | | CSVMHEALHNHYTQKSLSLSPGK |
| 122 | Light chain | DIQMTQSPSTLSASVGDRVTITCRASQSISSWLAWYQQKPGKA PKLLIYKASSLESGVPSRFSGSGSGTEFTLTISSLQPDDFATYYC QQYNSYSYTFGQGTKLEIKRTVAAPSVFIFPPSDEQLKSGTASV VCLLNNFYPREAKVQWKVDNALQSGNSQESVTEQDSKDSTYS LSSTLTLSKADYEKHKVYACEVTHQGLSSPVTKSFNRGEC |
| SEQ ID NO | | CD3 end HzSP34.24 in bispecific antibody |
| 123 | Heavy chain | EVQLVESGGGLVQPGGSLKLSCAASGFTFNTYAMNWVRQASG KGLEWVGRIRSKYNNYATYYADSVKDRFTISRDDSKSTLYLQM NSLKTEDTAVYYCARHGNFGQSYVSWFAYWGQGTTVTVSSAS TKGPSVFPLAPSSKSTSGGTAALGCLVKDYFPEPVTVSWNSGA LTSGVHTFPAVLQSSGLYSLSSVVTVPSSSLGTQTYICNVNHKPS NTKVDKKVEPKSCDKTHTCPPCPAPEAAGGPSVFLFPPKPKDT LMISRTPEVTCVVVDVSHEDPEVKFNWYVDGVEVHNAKTKPR EEQYNSTYRVVSVLTVLHQDWLNGKEYKCKVSNKALPAPIEK TISKAKGQPREPQVYTLPPSRDELTKNQVSLSCAVKGFYPSDIA VEWESNGQPENNYKTTPPVLDSDGSFFLVSKLTVDKSRWQQG NVFSCSVMHEALHNHYTQKSLSLSPGK |
| 124 | Light chain | QAVVTQEPSLTVSPGGTVTLTCRSSTGAVTTSNYANWVQQKPG QAPRGLIGGTNKRAPGVPARFSGSLLGDKAALTLLGAQPEDEA EYYCALWYSNLWVFGQGTKLTVLGQPKAAPSVTLFPPSSEELQ ANKATLVCLISDFYPGAVTVAWKADSSPVKAGVETTTPSKQSN NKYAASSYLSLTPEQWKSHRSYSCQVTHEGSTVEKTVAPTECS |
| SEQ ID NO | | CD3 end HzSP34.87 in bispecific antibody |

(continued)

| SEQ ID NO | | Knob (CH1+CH2+CH3) |
|---|---|---|
| 125 | Heavy chain | EVQLVESGGGLVQPGGSLKLSCAASGFTFNTYAMNWVRQASGKGLEWVGRIRSKYNNYATYYADSVKDRFTISRDDSKSTLYLQMNSLKTEDTAVYYCARHYNFGQSYVSWFAYWGQGTTVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPEAAGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLSCAVKGFYPSDIAVEWESNGQPENNYKTTPPVLDSDGSFFLVSKLTVDKSRWQQGNVFSCSVMHEALHNHYTQKSLSLSPGK |
| 124 | Light chain | QAVVTQEPSLTVSPGGTVTLTCRSSTGAVTTSNYANWVQQKPGQAPRGLIGGTNKRAPGVPARFSGSLLGDKAALTLLGAQPEDAEYYCALWYSNLWVFGQGTKLTVLGQPKAAPSVTLFPPSSEELQANKATLVCLISDFYPGAVTVAWKADSSPVKAGVETTTPSKQSNNKYAASSYLSLTPEQWKSHRSYSCQVTHEGSTVEKTVAPTECS |
| SEQ ID NO | | CD3 end HzSP34.97 in bispecific antibody |
| 126 | Heavy chain | EVQLVESGGGLVQPGGSLKLSCAASGFTFNTYAMNWVRQASGKGLEWVGRIRSKAGGYATYYADSVKDRFTISRDDSKSTLYLQMNSLKTEDTAVYYCARHGNFGQSYVSWFAYWGQGTTVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPEAAGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLSCAVKGFYPSDIAVEWESNGQPENNYKTTPPVLDSDGSFFLVSKLTVDKSRWQQGNVFSCSVMHEALHNHYTQKSLSLSPGK |
| 124 | Light chain | QAVVTQEPSLTVSPGGTVTLTCRSSTGAVTTSNYANWVQQKPGQAPRGLIGGTNKRAPGVPARFSGSLLGDKAALTLLGAQPEDAEYYCALWYSNLWVFGQGTKLTVLGQPKAAPSVTLFPPSSEELQANKATLVCLISDFYPGAVTVAWKADSSPVKAGVETTTPSKQSNNKYAASSYLSLTPEQWKSHRSYSCQVTHEGSTVEKTVAPTECS |

**Example 9. Determination of affinity of bispecific antibody**

The equilibrium dissociation constant (KD) of the bispecific antibody of the invention binding to human CD3 protein was determined by the Biolayer Interferometry (BLI). The affinity determination of BLI method was carried out according to the existing methods (Estep, P et al., High throughput solution based measurement of antibody-antigen affinity and affinity binding. MAbs, 2013.5 (2): 270-8).

An appropriate number of AHC (18-5060, Fortebio) sensors was taken and soaked in SD buffer (1× PBS, BSA 0.1%, Tween-20 0.05%) according to the number of samples at half an hour before the experiment. 100 µl of SD buffer, each bispecific antibody and human CD3 protein (CT026H0323H, Sino Biological Inc.) were added into 96-well black polystyrene semi-quantitative micro-well plate (Greiner, 675076). Fortebio Octet Red96 was used for detection, and the sensor position was selected according to the sample position. The setting parameters of the instrument were as follows: operation steps: Baseline, Loading~1 nm, Baseline, Association and Dissociation; the operation time of each step depended on the speed of sample combination and dissociation. The rotational speed was 1000 rpm and the temperature was 30 °C. ForteBio Octet analysis software was used to analyze KD value.

The affinity of bispecific antibodies was shown in Table 10. The affinity of CD3 moiety of 030 molecule was the highest, with 7.4nM. The affinity of CD3 moiety of 032 and 033 molecules decreased in turn, with 89nM and 440nM respectively.

Table 10. Affinity of CD3 moiety of the bispecific antibodies

|  | Ka(1/Ms) | Kd(1/s) | KD(M) |
|---|---|---|---|
| 030 | 6.927E+5 | 0.005164 | 7.455E-9 |
| 032 | 8.066E+5 | 0.07183 | 8.906E-8 |
| 033 | 3.788E+5 | 0.1689 | 4.459E-7 |

The equilibrium dissociation constant (KD) of binding to human CLDN18.2 was determined by surface plasma resonance (SPR). According to the manufacturer's instructions, the antigen human Claudin 18.2 (GenScrip, P50251802) was coupled to the surface of CM5 chip (GE Healthcare, 29-1496-03) using the amino-coupling kit (GE Healthcare, BR-1006-33). After coupling, 1 M ethanolamine was injected to block the remaining activation sites. According to the manufacturer's instructions, the binding and dissociation between the chip surface antigen and various bispecific antibodies in the mobile phase were detected by Biacore (GE Healthcare, T200) to obtain the affinity and kinetic constants. The antibody after serial dilution (0-100 nM, twice dilution) flowed through the chip surface in order from low concentration to high concentration, with binding time of 180 s and dissociation time of 600 s. Finally, 10 mM Glycine pH 1.5 (GE Healthcare, BR-1003-54) was used to regenerate the chip. The resulting data were analyzed using the Biacore T200 analysis software and the 1:1 combination model. Results was shown in Table 11, the same clone HB37A6 was used for 030, 032 and 033 bispecific antibodies at the CLDN18.2 moiety, and the affinity of the CLDN18.2 moiety was consistent with a very strong affinity of 0.57nM.

Table 11. Affinity of CLDN18.2 moiety of the bispecific antibodies

|  | Ka(1/Ms) | Kd(1/s) | KD(M) |
|---|---|---|---|
| 030 | | | |
| 032 | 3.47E+05 | 2.00E-04 | 5.76E-10 |
| 033 | | | |

**Example 10. T cell killing test *In vitro***

The 030, 032 and 033 bispecific antibodies obtained in Example 9 were used for T cell killing test *in vitro*. Human peripheral blood mononuclear cells (PBMC, Allcells or Saily) were re-suspended with complete medium RPMI-1640 (Hyclone, SH30809.01)+10% fetal bovine serum (FBS, Hyclone, SH30084.03), and PBMC was adjusted to $2 \times 10^6$ /ml.

NUGC-4 (JCRB cell bank, JCRB0834) or DAN-G tumor target cell DAN-G-hCLDN18.2 overexpressing Claudin 18.2 prepared as follows, non-target cell L363 (DSMZ, ACC49) were labeled with Far-Red (Invitrogen) for 10 min, washed twice and then resuspended in the complete medium, and then the cell concentration was adjusted to $2 \times 10^5$ /ml.

The construction of DAN-G-Hcldn18.2 was as follows:

The full-length gene of human CLDN18.2 (UniProt ID: P56856-2) was constructed into the vector pWPT-GFP (Addgene, 12255) to replace the GFP sequence. Said vectors, the lentivirus packaging vectors psPAX2 (Addgene, 12260) and pMD2. G (Addgene, 12259) were co-transfected into HEK293T (ATCC, CRL-3216) cells for virus packaging. The culture supernatant after 48 hours and 72 hours of culture were collected respectively, and PEG8000 was used to concentrate the lentivirus. The concentrated virus was used to transfect pancreatic cancer DAN-G cells, and then the cells expressing CLDN18.2 were sorted out by flow cytometry (MoFlo XDP, Beckman Coulter) to obtain a tumor cell line DAN-G-hCLDN18.2 stably transfected with CLDN18.2.

PBMC was mixed with bispecific antibodies 030, 032 and 033 respectively (the initial concentration of 030 and 032 were 1 nM, and the initial concentration of 033 was 400 nM. All antibodies were diluted five times, a total of 10 concentration points), incubated at 37 °C for 30 minutes, and then 50 $\mu$L tumor target cells ($1 \times 10^4$) were added to 50 $\mu$L PBMC effector cells with 10:1 ratio of effector cells: target cells, followed by incubation at 37 °C for 24 hours, centrifugation, resuspending of the cells with the final concentration of 10 $\mu$g/ml of propidium iodide (PI, Invitrogen), and then flow cytometry (BD, FACSCELESTA) was used to detect both Far-Red and PI positive cells. The killing ratio of tumor target cells was calculated by FACSDiva software (BD, Celestsa).

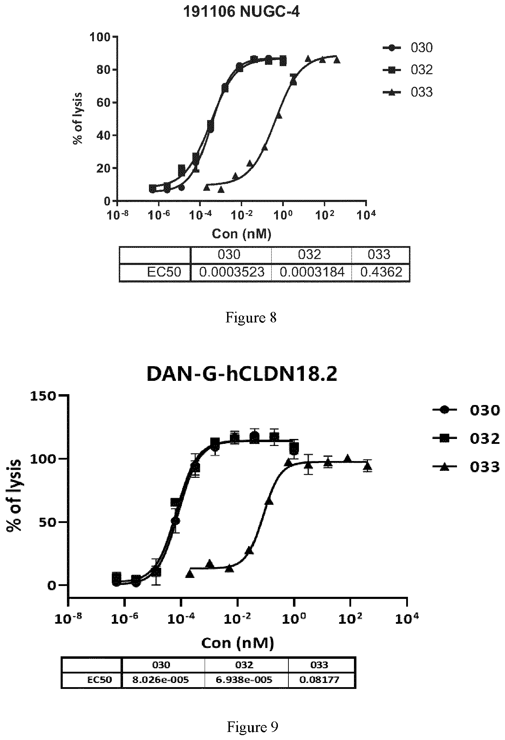

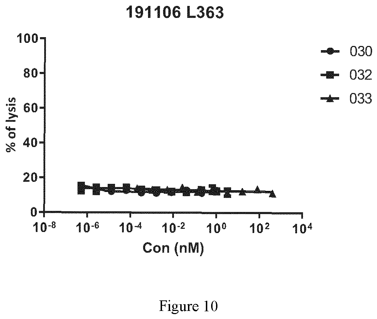

The results in Figure 8 showed that 030 and 032 molecules had very strong killing activity on the gastric cancer cell NUGC-4, and the EC50 value was less than 1pM. The results in Figure 9 showed that the EC50 values for both cells were even less than 0.1pM for DAN-G-hCLDN18.2, a pancreatic cancer cell with high expression of CLDN18.2. The killing activity of 033 molecule for the two tumor cell lines was weaker, lower than 030 and 032 about 1000 times, but it still reached the maximum killing (nearly 100% lysed cells). However, in the case of negative expression of CLDN18.2, 030, 032 and 033 molecules all did not have non-specific killing effect (Fig. 10). This indicates that 030, 032 and 033 molecules all exhibit the specific killing of tumor cells that depends on the expression of CLDN18.2. Moreover, the killing effect of the bispecific antibody of the invention is related to the abundance of CLDN18.2 on the cell surface. Within a certain range of expression abundance, the higher the expression of CLDN18.2 on the cell surface, the better the killing effect is.

## Example 11. Release test of cytokine in vitro

Human peripheral blood mononuclear cells (PBMC, Allcells or Saily) were resuspended with complete medium RPMI-1640 (Hyclone, SH30809.01)+10% fetal bovine serum (FBS, Hyclone, SH30084.03), and PBMCs were adjusted to $2\times10^6$ /ml. The concentrations of NUGC-4 or DAN-G tumor target cell DAN-G-hCLDN18.2 overexpressing Claudin 18.2 were adjusted to $2\times10^5$/ml.

PBMC was mixed with bispecific antibodies 030, 032 and 033 respectively, incubated at 37 °C for 30 min, and then 50 $\mu$L Tumor target cells ($1 \times 10^4$) were added to 50 $\mu$L PBMC effector cells with 10:1 ratio of effector cells: target cells, followed by incubation at 37°C for 24 hours, centrifugation, and obtaining cell supernatant. Human Th1/Th2/Th17 Kit (BD, article No. 560484) was used to detect cytokines followed by incubation at room temperature for 3 hours and detection by flow cytometry (BD, FACSCELESTA), and then the release of cytokines in the supernatant was analyzed by FCAP Array software (BD).

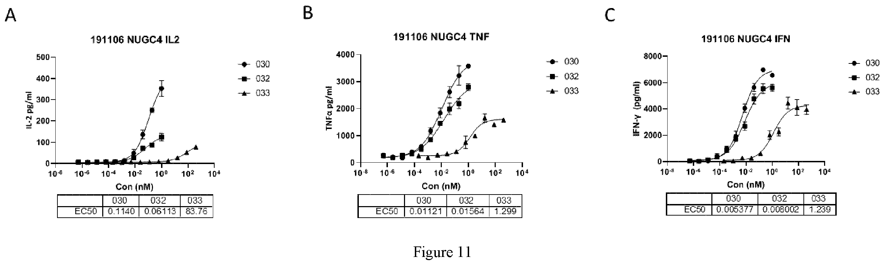

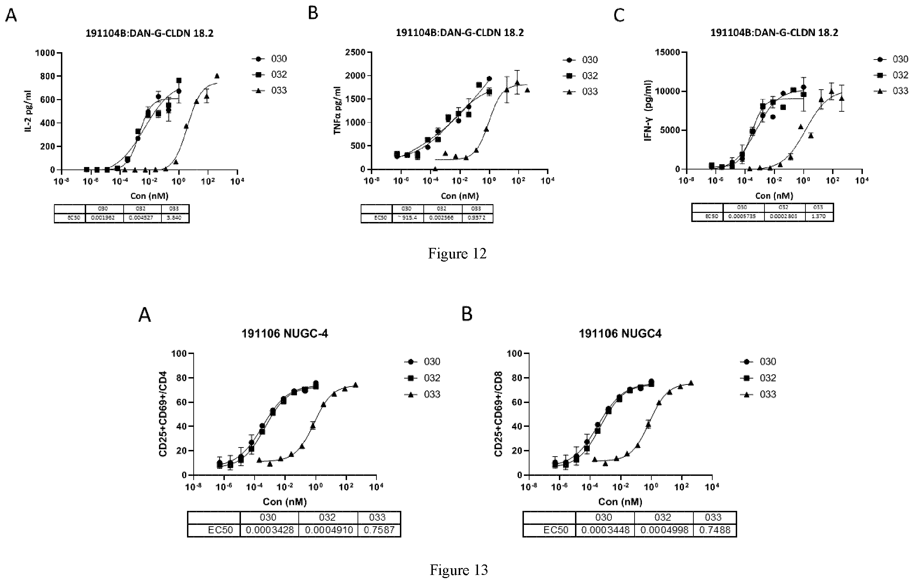

The results in Figure 11 and Figure 12 showed 030, 032 and 033 molecules was able to mediate the high release of IL-2, TNF$\alpha$ and IFNgamma cytokines on gastric cancer cell NUGC-4 and pancreatic cancer cell DAN-G-hCLDN18.2, which were positively correlated with the affinity of CD3 moiety.

## Example 12. T cell activation test *in vitro*

Human peripheral blood mononuclear cells (PBMC, Allcells or Saily) were re-suspended with complete medium RPMI-1640 (Hyclone, SH30809.01)+10% fetal bovine serum (FBS, Hyclone, SH30084.03), and PBMC were adjusted to $2\times10^6$/ml.

The concentration of NUGC-4 or DAN-G tumor target cell DAN-G-CLDN18.2 overexpressing Claudin 18.2 were adjusted to $2\times10^5$/ml. PBMC was mixed with bispecific antibodies 030, 032 and 033 respectively, incubated at 37 °C for 30 min, and then 50 $\mu$L tumor target cells ($1\times10^4$) were added to 50 $\mu$L PBMC effector cells with 10:1 ratio of effector cells: target cells, followed by incubation at 37 °C for 24 hours, centrifugation, removing the supernatant, and then the cells were incubated with BV421 anti-human CD3 (Biolegend, 317344), PerCP/Cy5.5 mouse anti-human CD4 (BD, 552838), APC/Cy7 anti-human CD8a (Biolegend, 300926), PE anti-human CD25 (Biolegend, 302606), FITC anti-human CD69 (Biolegend, 310904) for 1 hour at 4 °C, and then washed three times with $1 \times$ PBS, and the ratio of both CD25 and CD69 positive cells in CD4+and CD8+T cells was detected by flow cytometry (BD, FACSCELESTA). The ratio of both CD25 and CD69 positive cells in CD4+and CD8+T cells was calculated by FACSDiva software (BD, Celestsa), which is the ratio of CD4 and CD8+T cells in activation.

As shown in Figure 13, 030, 032 and 033 molecules could specifically activate T cells in the co-culture of gastric cancer cell NUGC-4, and the activation ability was positively correlated with the affinity of CD3 moiety.

## Example 13. Pharmacodynamic experiment in vivo - gastric cancer model

In this experiment, the anti-tumor effect of bispecific antibody on NUGC-4 tumor was studied in NOG female mice. 49 female NOG mice (Beijing Weitong Lihua Experimental Animal Technology Co., Ltd.) were selected.

PBMC cells (Allcells) were resuscitated and centrifuged. PBS ($1\times$) was used to disperse PBMC cells to obtain the cell suspension with a cell density of $2\times10^7$/ml. 200 $\mu$L cell suspension was taken to inject PBMC cells into the orbital vein of mice, $4\times10^6$/mouse.

**EP 4 223 777 A1**

NUGC-4 cells were routinely resuscitated and subcultured for subsequent experiments *in vivo*. Centrifuge and collect cells, disperse NUGC-4 cells with PBS (1×), and the cells with cell density of $6 \times 10^7$ cells/ml were mixed with matrigel gel at 1:1 to prepare cell suspension with a cell density of $3 \times 10^7$/ml. On the third day, 0.2 ml of cell suspension was subcutaneously inoculated into the right abdominal region of NOG humanized mice to establish a mice model bearing NUCG-4 tumor.

On the 7th day after cell inoculation, the maximum wide axis and the maximum long axis of the tumor in mouse were measured with a vernier caliper, and the tumor volume was calculated. The mice with tumor volume between 53.35 $mm^3$ and 168.07 $mm^3$ were picked, and the mice were divided into serpentine group according to the tumor volume (6 mice in each group). The bispecific antibodies 030, 032 and 033 of the invention and the negative control h-IgG (Equitech-Bio, batch number 160308-02) were injected intravenously into each mouse, with the dose of 0.3 mg/kg and 1 mg/kg, once a week, a total of 4 times, and the frequency of measuring tumor volume was twice a week. Meanwhile, the tumor inhibition rate (TGI%) was calculated as follows:

$$TGI\%=100\% * \text{(tumor volume of control group - tumor volume of treatment group)/(tumor volume of control group - tumor volume of control group before administration)}.$$

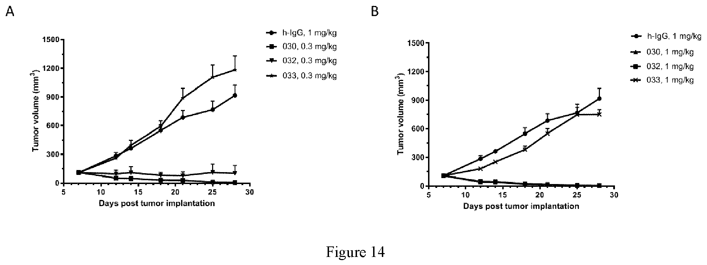

As shown in Figure 14, in the mouse model bearing human gastric cancer NUCG-4 tumor, 030 and 032 can reach 100% TGI at a low dose of 0.3mg/kg, and reach 50% CR (complete remission) at a high dose of 1mg/kg (3 of 6 mice have achieved complete tumor vanishment). However, 033 molecule almost had no effect at low dose, whereas TGI can reach 20% at a dose of 1mg/kg. During the whole experiment, the weight of mice in the experimental group and the control group did not drop.

**Example 14. Pharmacodynamic experiment in vivo - pancreatic cancer model**

In this experiment, the anti-tumor effect of bispecific antibody on DAN-G-Claudin 18.2 tumor was studied in NOG female mice. PBMC cells were injected into the orbital vein in 49 NOG mice (Beijing Weitong Lihua Experimental Animal Technology Co., Ltd.), $4 \times 10^6$/mouse, and inoculation volume was 200ul/mouse (as shown in Example 13). This was recorded as day 0.

The human pancreatic cancer cell DAN-G-CLDN18.2 constructed in Example 10 was routinely sub-cultured for subsequent in vivo experiments. The cells were centrifuged and collected. DAN-G-CLDN18.2 were dispersed with PBS (1×) to obtain the suspension with cell density of $10 \times 10^6$/ml. The cell suspension was mixed with matrigel gel at 1:1 to prepare the cell suspension with a concentration of $5 \times 10^6$ cells/ml. On day 0, 0.2 ml of cell suspension was taken and subcutaneously injected into the right abdominal region of NOD-SCID mice to establish a humanized model of DNA-G pancreatic cancer with CLDN18.2 overexpression.

7 days after the inoculation of tumor cells, the maximum wide axis and the maximum long axis of the tumor were measured with a vernier caliper, and the tumor volume was calculated. The mice with tumor volume in the range of 46.42 $mm^3$~120.64 $mm^3$ were divided into serpentine group according to the tumor size (6 mice in each group).

The bispecific antibodies 030, 032 and 033 of the invention and the negative control h-IgG (Equitech-Bio, batch number 160308-02) were injected intravenously into each mouse, with the intraperitoneal dose of 0.3 mg/kg and 1 mg/kg, once a week, a total of 4 times, and the frequency of measuring tumor volume was twice a week. Meanwhile, the tumor inhibition rate (TGI%) was calculated as follows:

$$TGI\%=100\% * \text{(tumor volume of control group - tumor volume of treatment group)/(tumor volume of control group - tumor volume of control group before administration)}.$$

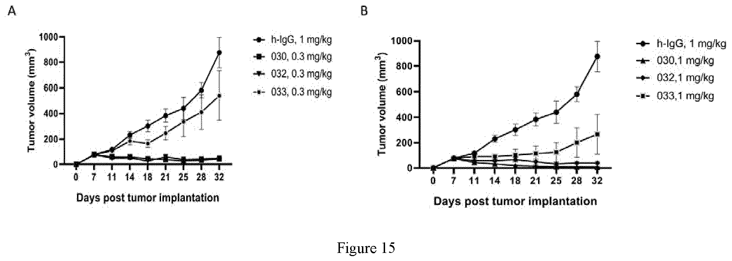

As shown in Figure 15, in the humanized model of DNA-G pancreatic cancer with CLDN18.2 overexpression, 030 and 032 molecules can reach 100% TGI at both doses. The 033 molecule also reached 42% TGI at 0.3mg/kg of and 76% TGI at 1mg/kg respectively, which may be related to the high expression of CLDN18.2 in DAN-G-CLDN18.2 pancreatic cancer cells. During the whole experiment, the weight of mice in the experimental group and the control group did not drop.

**Example 15. PK experiment in mouse**

In this study, female Balb/C mice (Vitoliva) were injected with 10 mg/kg of 030, 032 and 033 via tail vein to study their pharmacokinetics in mice. After administration, blood was taken from the eyes of mice at 0.086hr, 0.5hr, 2hr, 6hr, 24hr, 48hr, 4day, 7day, 14day and 21day respectively, and the blood was centrifuged at 4 °C at 3000rpm for 10 min to collect serum. The antibody content in serum was determined by ELISA, and the half-lives of 030, 032 and 033 in mice were calculated.

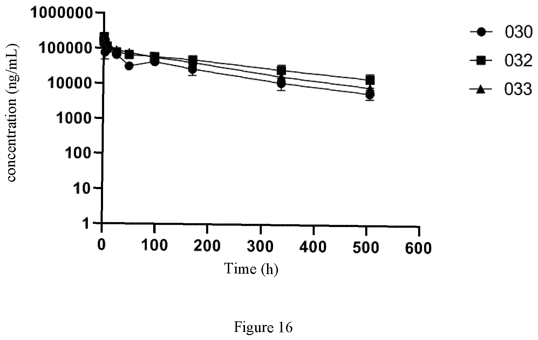

The experimental results were shown in Figure 16. The half-lives of 030, 032 and 033 in mice were similar to PK of normal monoclonal antibodies. It further showed that the bispecific antibody constructed by the invention did not affect the half-life of the antibody.

| Name of variable region sequence | HCDR1/LCDR1 | HCDR2/LCDR2 | HCDR3/LCDR3 | VH/VL | HC/LC |
|---|---|---|---|---|---|
| SP34_H | GFTFNTYAMN(SEQ ID NO:1) | RIRSKYNNYATYYADSVKD(SEQ ID NO:2) | HGNFGNSYVSWFAY(SEQ ID NO:3) | EVQLVESGGGLVQPKGSLKLSCAASGFTFNTYAMNWVRQAPGKGLEWVARIRSKYNNYATYYADSVKDRFTISRDDSQSILYLQMNNLKTEDTAMYYCVRHGNFGNSYVSWFAYWGQGTLVTVSS(SEQ ID NO:47) | ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPEAAGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK(SEQ ID NO:100) |
| husp34h.g0 | GFTFNTYAMN(SEQ ID NO:1) | RIRSKYNNYATYYADSVKD(SEQ ID NO:2) | HGNFGNSYVSWFAY(SEQ ID NO:3) | EVQLVESGGGLVQPGGSLKLSCAASGFTFNTYAMNWVRQASGKGLEWVGRIRSKYNNYATYYADSVKDRFTISRDDSKNTLYLQMNSLKTEDTAVYYCARHGNFGNSYVSWFAYWGQGTTVTVSS(SEQ ID NO:48) | SEQ ID NO:100 |
| husp34h.g1 | GFTFNTYAMN(SEQ ID | RIRSKYNNYATYYADSVKD( | HGNFGNSYVSWFAY(SEQ ID NO:3) | EVQLVESGGGLVQPGGSLKLSCAASGFTFNTYAMNWVRQASGKGLEWVGRIRSKYNNY | SEQ ID NO:100 |

| | NO:1) | SEQ ID NO:2) | | ATYYADSVKDRFTISRDDSKSTLYLQMNSLKTEDTAVYYCARHGNFGNSYVSWFAYWGQGTTVTVSS(SEQ ID NO:49) | |
|---|---|---|---|---|---|
| husp34h.g2 | GFTFNTYAMN(SEQ ID NO:1) | RIRSKYNNYATYYADSVKD(SEQ ID NO:2) | HGNFGQSYVSWFAY(SEQ ID NO:8) | EVQLVESGGGLVQPGGSLKLSCAASGFTFNTYAMNWVRQASGKGLEWVGRIRSKYNNYATYYADSVKDRFTISRDDSKSTLYLQMNSLKTEDTAVYYCARHGNFGQSYVSWFAYWGQGTTVTVSS(SEQ ID NO:50) | SEQ ID NO:100 |
| husp34h.g3 | GFTFNTYAMN(SEQ ID NO:1) | RIRSKYNNYATYYADSVKD(SEQ ID NO:2) | HGNFGQSYVSWFAY(SEQ ID NO:8) | EVQLVESGGGLVQPGGSLKLSCAASGFTFNTYAMNWVRQASGKGLEWVARIRSKYNNYATYYADSVKDRFTISRDDSKSTLYLQMNSLKTEDTAVYYCARHGNFGQSYVSWFAYWGQGTTVTVSS(SEQ ID NO:51) | SEQ ID NO:100 |
| husp34h.g2.1 | GFTFGTYAMN(SEQ ID NO:4) | RIRSKYNNYATYYADSVKD(SEQ ID NO:2) | HGNFGQSYVSWFAY(SEQ ID NO:8) | EVQLVESGGGLVQPGGSLKLSCAASGFTFGTYAMNWVRQASGKGLEWVGRIRSKYNNYATYYADSVKDRFTISRDDSKSTLYLQMNSLKTEDTAVYYCARHGNFGQSYVSWFAYWGQGTTVTVSS(SEQ ID NO:52) | SEQ ID NO:100 |
| husp34h.g2.2 | GFTFNGYAMN(SEQ ID NO:5) | RIRSKYNNYATYYADSVKD(SEQ ID NO:2) | HGNFGQSYVSWFAY(SEQ ID NO:8) | EVQLVESGGGLVQPGGSLKLSCAASGFTFNGYAMNWVRQASGKGLEWVGRIRSKYNNYATYYADSVKDRFTISRDDSKSTLYLQMNSLKTEDTAVYYCARHGNFGQSYVSWFAYWGQ | SEQ ID NO:100 |

| | | | | GTTVTVSS(SEQ ID NO:53) | |
|---|---|---|---|---|---|
| husp34h.g2.3 | GFTFNTAAMN(SEQ ID NO:6) | RIRSKYNNYATYYADSVKD(SEQ ID NO:2) | HGNFGQSYVSWFAY(SEQ ID NO:8) | EVQLVESGGGLVQPGGSLKLSCAASGFTFNTAAMNWVRQASGKGLEWVGRIRSKYNNYATYYADSVKDRFTISRDDSKSTLYLQMNSLKTEDTAVYYCARHGNFGQSYVSWFAYWGQGTTVTVSS(SEQ ID NO:54) | SEQ ID NO:100 |
| husp34h.g2.4 | GFTFNTYAMN(SEQ ID NO:1) | RISSKYNNYATYYADSVKD(SEQ ID NO:7) | HGNFGQSYVSWFAY(SEQ ID NO:8) | EVQLVESGGGLVQPGGSLKLSCAASGFTFNTYAMNWVRQASGKGLEWVGRISSKYNNYATYYADSVKDRFTISRDDSKSTLYLQMNSLKTEDTAVYYCARHGNFGQSYVSWFAYWGQGTTVTVSS(SEQ ID NO:55) | SEQ ID NO:100 |
| husp34h.g2.5 | GFTFNTYAMN(SEQ ID NO:1) | RIRLKYNNYATYYADSVKD(SEQ ID NO:9) | HGNFGQSYVSWFAY(SEQ ID NO:8) | EVQLVESGGGLVQPGGSLKLSCAASGFTFNTYAMNWVRQASGKGLEWVGRIRLKYNNYATYYADSVKDRFTISRDDSKSTLYLQMNSLKTEDTAVYYCARHGNFGQSYVSWFAYWGQGTTVTVSS(SEQ ID NO:56) | SEQ ID NO:100 |
| husp34h.g2.6 | GFTFNTYAMN(SEQ ID NO:1) | RIRSKANNYATYYADSVKD(SEQ ID NO:10) | HGNFGQSYVSWFAY(SEQ ID NO:8) | EVQLVESGGGLVQPGGSLKLSCAASGFTFNTYAMNWVRQASGKGLEWVGRIRSKANNYATYYADSVKDRFTISRDDSKSTLYLQMNSLKTEDTAVYYCARHGNFGQSYVSWFAYWGQGTTVTVSS(SEQ ID NO:57) | SEQ ID NO:100 |
| husp34h.g2.7 | GFTFNTYAMN(SEQ ID NO:1) | RIRSKYGNYATYYADSVKD( | HGNFGQSYVSWFAY(SEQ ID NO:8) | EVQLVESGGGLVQPGGSLKLSCAASGFTFNTYAMNWVRQASGKGLEWVGRIRSKYGNY | SEQ ID NO:100 |

| | | | | | |
|---|---|---|---|---|---|
| | NO:1) | SEQ ID NO:11) | | ATYYADSVKDRFTISRDDSKSTLYLQMNSLK TEDTAVYYCARHGNFGQSYVSWFAYWGQ GTTVTVSS(SEQ ID NO:58) | |
| husp34h.g 2.8 | GFTFNTYAM N(SEQ ID NO:1) | RIRSKYNGYA TYYADSVKD( SEQ ID NO:12) | HGNFGQSYVSWFA Y(SEQ ID NO:8) | EVQLVESGGGLVQPGGSLKLSCAASGFTFN TYAMNWVRQASGKGLEWVGRIRSKYNGY ATYYADSVKDRFTISRDDSKSTLYLQMNSLK TEDTAVYYCARHGNFGQSYVSWFAYWGQ GTTVTVSS(SEQ ID NO:59) | SEQ ID NO:100 |
| husp34h.g 2.9 | GFTFNTYAM N(SEQ ID NO:1) | RIRSKYNNYA TYYADSVKD( SEQ ID NO:2) | AGNFGQSYVSWFA Y(SEQ ID NO:13) | EVQLVESGGGLVQPGGSLKLSCAASGFTFN TYAMNWVRQASGKGLEWVGRIRSKYNNY ATYYADSVKDRFTISRDDSKSTLYLQMNSLK TEDTAVYYCARAGNFGQSYVSWFAYWGQ GTTVTVSS(SEQ ID NO:60) | SEQ ID NO:100 |
| husp34h.g 2.10 | GFTFNTYAM N(SEQ ID NO:1) | RIRSKYNNYA TYYADSVKD( SEQ ID NO:2) | HYNFGQSYVSWFA Y(SEQ ID NO:14) | EVQLVESGGGLVQPGGSLKLSCAASGFTFN TYAMNWVRQASGKGLEWVGRIRSKYNNY ATYYADSVKDRFTISRDDSKSTLYLQMNSLK TEDTAVYYCARHYNFGQSYVSWFAYWGQ GTTVTVSS(SEQ ID NO:61) | SEQ ID NO:100 |
| husp34h.g 2.11 | GFTFNTYAM N(SEQ ID NO:1) | RIRSKYNNYA TYYADSVKD( SEQ ID NO:2) | HGGFGQSYVSWFA Y(SEQ ID NO:15) | EVQLVESGGGLVQPGGSLKLSCAASGFTFN TYAMNWVRQASGKGLEWVGRIRSKYNNY ATYYADSVKDRFTISRDDSKSTLYLQMNSLK TEDTAVYYCARHGGFGQSYVSWFAYWGQ GTTVTVSS(SEQ ID NO:62) | SEQ ID NO:100 |
| husp34h.g 2.12 | GFTFNTYAM N(SEQ ID NO:1) | RIRSKYNNYA TYYADSVKD( SEQ ID NO:2) | HGNAGQSYVSWFA Y(SEQ ID NO:16) | EVQLVESGGGLVQPGGSLKLSCAASGFTFN TYAMNWVRQASGKGLEWVGRIRSKYNNY ATYYADSVKDRFTISRDDSKSTLYLQMNSLK TEDTAVYYCARHGNAGQSYVSWFAYWGQ GTTVTVSS(SEQ ID NO:63) | SEQ ID NO:100 |
| husp34h.g 2.13 | GFTFNTYAM N(SEQ ID NO:1) | RIRSKYNNYA TYYADSVKD( SEQ ID NO:2) | HGNFYQSYVSWFA Y(SEQ ID NO:17) | EVQLVESGGGLVQPGGSLKLSCAASGFTFN TYAMNWVRQASGKGLEWVGRIRSKYNNY ATYYADSVKDRFTISRDDSKSTLYLQMNSLK TEDTAVYYCARHGNFYQSYVSWFAYWGQ GTTVTVSS(SEQ ID NO:64) | SEQ ID NO:100 |
| husp34h.g 2.14 | GFTFNTYAM N(SEQ ID NO:1) | RIRSKYNNYA TYYADSVKD( SEQ ID NO:2) | HGNFGGSYVSWFA Y(SEQ ID NO:18) | EVQLVESGGGLVQPGGSLKLSCAASGFTFN TYAMNWVRQASGKGLEWVGRIRSKYNNY ATYYADSVKDRFTISRDDSKSTLYLQMNSLK TEDTAVYYCARHGNFGGSYVSWFAYWGQ GTTVTVSS(SEQ ID NO:65) | SEQ ID NO:100 |
| husp34h.g 2.15 | GFTFNTYAM N(SEQ ID NO:1) | RIRSKYNNYA TYYADSVKD( SEQ ID NO:2) | HGNFGQRYVSWFA Y(SEQ ID NO:19) | EVQLVESGGGLVQPGGSLKLSCAASGFTFN TYAMNWVRQASGKGLEWVGRIRSKYNNY ATYYADSVKDRFTISRDDSKSTLYLQMNSLK TEDTAVYYCARHGNFGQRYVSWFAYWGQ GTTVTVSS(SEQ ID NO:66) | SEQ ID NO:100 |
| husp34h.g 2.16 | GFTFNTYAM N(SEQ ID NO:1) | RIRSKYNNYA TYYADSVKD( SEQ ID | HGNFGQSAVSWFA Y(SEQ ID NO:20) | EVQLVESGGGLVQPGGSLKLSCAASGFTFN TYAMNWVRQASGKGLEWVGRIRSKYNNY ATYYADSVKDRFTISRDDSKSTLYLQMNSLK TEDTAVYYCARHGNFGQSAVSWFAYWGQ | SEQ ID NO:100 |

| | | NO:2) | | GTTVTVSS(SEQ ID NO:67) | |
|---|---|---|---|---|---|
| husp34h.g2.17 | GFTFNTYAMN(SEQ ID NO:1) | RIRSKYNNYATYYADSVKD(SEQ ID NO:2) | HGNFGQSYASWFAY(SEQ ID NO:21) | EVQLVESGGGLVQPGGSLKLSCAASGFTFNTYAMNWVRQASGKGLEWVGRIRSKYNNYATYYADSVKDRFTISRDDSKSTLYLQMNSLKTEDTAVYYCARHGNFGQSYASWFAYWGQGTTVTVSS(SEQ ID NO:68) | SEQ ID NO:100 |
| husp34h.g2.18 | GFTFSGSAMN(SEQ ID NO:22) | RIRSKYNNYATYYADSVKD(SEQ ID NO:2) | HGNFGQSYVSWFAY(SEQ ID NO:8) | EVQLVESGGGLVQPGGSLKLSCAASGFTFSGSAMNWVRQASGKGLEWVGRIRSKYNNYATYYADSVKDRFTISRDDSKSTLYLQMNSLKTEDTAVYYCARHGNFGQSYVSWFAYWGQGTTVTVSS(SEQ ID NO:69) | SEQ ID NO:100 |
| husp34h.g2.19 | GFTFNTYAMN(SEQ ID NO:1) | RISLKYNNYATYYADSVKD(SEQ ID NO:23) | HGNFGQSYVSWFAY(SEQ ID NO:8) | EVQLVESGGGLVQPGGSLKLSCAASGFTFNTYAMNWVRQASGKGLEWVGRISLKYNNYATYYADSVKDRFTISRDDSKSTLYLQMNSLKTEDTAVYYCARHGNFGQSYVSWFAYWGQGTTVTVSS(SEQ ID NO:70) | SEQ ID NO:100 |
| husp34h.g2.20 | GFTFNTYAMN(SEQ ID NO:1) | RIRSKAGGYATYYADSVKD(SEQ ID NO:24) | HGNFGQSYVSWFAY(SEQ ID NO:8) | EVQLVESGGGLVQPGGSLKLSCAASGFTFNTYAMNWVRQASGKGLEWVGRIRSKAGGYATYYADSVKDRFTISRDDSKSTLYLQMNSLKTEDTAVYYCARHGNFGQSYVSWFAYWGQGTTVTVSS(SEQ ID NO:71) | SEQ ID NO:100 |

| husp34h.g2.21 | GFTFNTYAMN(SEQ ID NO:1) | RIRSKYNNYATYYADSVKD(SEQ ID NO:2) | HYGFGQSYVSWFAY(SEQ ID NO:25) | EVQLVESGGGLVQPGGSLKLSCAASGFTFNTYAMNWVRQASGKGLEWVGRIRSKYNNYATYYADSVKDRFTISRDDSKSTLYLQMNSLKTEDTAVYYCARHYGFGQSYVSWFAYWGQGTTVTVSS(SEQ ID NO:72) | SEQ ID NO:100 |
|---|---|---|---|---|---|
| husp34h.g2.22 | GFTFNTYAMN(SEQ ID NO:1) | RIRSKYNNYATYYADSVKD(SEQ ID NO:2) | HGNAYQSYVSWFAY(SEQ ID NO:26) | EVQLVESGGGLVQPGGSLKLSCAASGFTFNTYAMNWVRQASGKGLEWVGRIRSKYNNYATYYADSVKDRFTISRDDSKSTLYLQMNSLKTEDTAVYYCARHGNAYQSYVSWFAYWGQGTTVTVSS(SEQ ID NO:73) | SEQ ID NO:100 |
| husp34h.g2.23 | GFTFNTYAMN(SEQ ID NO:1) | RIRSKYNNYATYYADSVKD(SEQ ID NO:2) | HGNFGGRYVSWFAY(SEQ ID NO:27) | EVQLVESGGGLVQPGGSLKLSCAASGFTFNTYAMNWVRQASGKGLEWVGRIRSKYNNYATYYADSVKDRFTISRDDSKSTLYLQMNSLKTEDTAVYYCARHGNFGGRYVSWFAYWGQGTTVTVSS(SEQ ID NO:74) | SEQ ID NO:100 |
| husp34h.g2.24 | GFTFNTYAMN(SEQ ID NO:1) | RIRSKYNNYATYYADSVKD(SEQ ID NO:2) | HGNFGQSAASWFAY(SEQ ID NO:28) | EVQLVESGGGLVQPGGSLKLSCAASGFTFNTYAMNWVRQASGKGLEWVGRIRSKYNNYATYYADSVKDRFTISRDDSKSTLYLQMNSLKTEDTAVYYCARHGNFGQSAASWFAYWGQGTTVTVSS(SEQ ID NO:75) | SEQ ID NO:100 |
| SP34_L | RSSTGAVTTSNYAN(SEQ ID NO:29) | GTNKRAP(SEQ ID NO:30) | ALWYSNLWV(SEQ ID NO:31) | QAVVTQESALTTSPGETVTLTCRSSTGAVTTSNYANWVQEKPDHLFTGLIGGTNKRAPGVPARFSGSLIGDKAALTITGAQTEDEAIYFCAL | GQPKAAPSVTLFPPSSEELQANKATLVCLISDFYPGAVTVAWKADSSPVKAGVETTTPSKQSNNKYAASSYLSLTPEQWKSHRSYSCQVTHEGSTVEKTVAPTECS (SEQ ID |

| | | | | WYSNLWVFGGGTKLTVL(SEQ ID NO:76) | NO:101) |
|---|---|---|---|---|---|
| husp34l.g0 | RSSTGAVTTSNYAN(SEQ ID NO:29) | GTNKRAP(SEQ ID NO:30) | ALWYSNLWV(SEQ ID NO:31) | QAVVTQEPSLTVSPGGTVTLTCRSSTGAVTTSNYANWFQQKPGQAPRTLIYGTNKRAPWTPARFSGSLLGGKAALTLLLGAQPEDEAEYYCALWYSNLWVFGQGTKLTVL(SEQ ID NO:77) | SEQ ID NO:101 |
| husp34l.g1 | RSSTGAVTTSNYAN(SEQ ID NO:29) | GTNKRAP(SEQ ID NO:30) | ALWYSNLWV(SEQ ID NO:31) | QAVVTQEPSLTVSPGGTVTLTCRSSTGAVTTSNYANWVQQKPGQAPRTLIGGTNKRAPGVPARFSGSLLGGKAALTLLLGAQPEDEAEYYCALWYSNLWVFGQGTKLTVL(SEQ ID NO:78) | SEQ ID NO:101 |
| husp34l.g2 | RSSTGAVTTSNYAN(SEQ ID NO:29) | GTNKRAP(SEQ ID NO:30) | ALWYSNLWV(SEQ ID NO:31) | QAVVTQEPSLTVSPGGTVTLTCRSSTGAVTTSNYANWVQQKPGQAPRGLIGGTNKRAPGVPARFSGSLLGGKAALTLLLGAQPEDEAEYYCALWYSNLWVFGQGTKLTVL(SEQ ID NO:79) | SEQ ID NO:101 |
| husp34l.g3 | RSSTGAVTTSNYAN(SEQ ID NO:29) | GTNKRAP(SEQ ID NO:30) | ALWYSNLWV(SEQ ID NO:31) | QAVVTQEPSLTVSPGGTVTLTCRSSTGAVTTSNYANWVQQKPGQAPRGLIGGTNKRAPGVPARFSGSLLGDKAALTLLLGAQPEDEAEYYCALWYSNLWVFGQGTKLTVL(SEQ ID NO:80) | SEQ ID NO:101 |
| husp34k.g0 | RSSTGAVTTSNYAN(SEQ ID NO:29) | GTNKRAP(SEQ ID NO:30) | ALWYSNLWV(SEQ ID NO:31) | EIVMTQSPATLSVSPGERATLSCRSSTGAVTTSNYANWYQQKPGQAPRLLIYGTNKRAPGIPARFSGSGSGTEFTLTISSLQSEDFAVYYCAL | RTVAAPSVFIFPPSDEQLKSGTASVVCLLNNFYPREAKVQWKVDNALQSGNSQESVTEQDSKDSTYSLSSTLTLSKADYEKHKV |

| | | | | WYSNLWVFGQGTKLTVL(SEQ ID NO:81) | YACEVTHQGLSSPVTKSFNRGEC (SEQ ID NO:102) |
|---|---|---|---|---|---|
| husp34k.g1 | RSSTGAVTTSNYAN(SEQ ID NO:29) | GTNKRAP(SEQ ID NO:30) | ALWYSNLWV(SEQ ID NO:31) | EIVMTQSPATLSVSPGERATLSCRSSTGAVTTSNYANWVQQKPGQAPRLLIGGTNKRAPGVPARFSGSGSGTEFTLTISSLQSEDFAVYYCALWYSNLWVFGQGTKLTVL(SEQ ID NO:82) | SEQ ID NO:102 |
| husp34k.g2 | RSSTGAVTTSNYAN(SEQ ID NO:29) | GTNKRAP(SEQ ID NO:30) | ALWYSNLWV(SEQ ID NO:31) | EIVMTQSPATLSVSPGERATLSCRSSTGAVTTSNYANWVQQKPGQAPRGLIGGTNKRAPGVPARFSGSGSGTEFTLTISSLQSEDFAVYYCALWYSNLWVFGQGTKLTVL(SEQ ID NO:83) | SEQ ID NO:102 |
| husp34k.g3 | RSSTGAVTTSNYAN(SEQ ID NO:29) | GTNKRAP(SEQ ID NO:30) | ALWYSNLWV(SEQ ID NO:31) | EIVMTQSPATLSVSPGERATLSCRSSTGAVTTSNYANWVQQKPGQAPRGLIGGTNKRAPGVPARFSGSGSGDEFTLTISSLQSEDFAVYYCALWYSNLWVFGQGTKLTVL(SEQ ID NO:84) | SEQ ID NO:102 |
| husp34lg3.1 | GSSTGAVTTSNYAN(SEQ ID NO:32) | GTNKRAP(SEQ ID NO:30) | ALWYSNLWV(SEQ ID NO:31) | QAVVTQEPSLTVSPGGTVTLTCGSSTGAVTTSNYANWVQQKPGQAPRGLIGGTNKRAPGVPARFSGSLLGDKAALTLLLGAQPEDEAEYYCALWYSNLWVFGQGTKLTVL(SEQ ID NO:85) | SEQ ID NO:101 |
| husp34lg3.2 | RSSTGAVGTSNYAN(SEQ | GTNKRAP(SEQ ID NO:30) | ALWYSNLWV(SEQ ID NO:31) | QAVVTQEPSLTVSPGGTVTLTCRSSTGAVGTSNYANWVQQKPGQAPRGLIGGTNKRAP | SEQ ID NO:101 |

| | | | | GVPARFSGSLLGDKAALTLLGAQPEDEAEYY CALWYSNLWVFGQGTKLTVL(SEQ ID NO:86) | |
|---|---|---|---|---|---|
| husp34lg3.3 | RSSTGAVTG SNYAN(SEQ ID NO:34) | GTNKRAP(SE Q ID NO:30) | ALWYSNLWV(SEQ ID NO:31) | QAVVTQEPSLTVSPGGTVTLTCRSSTGAVT GSNYANWVQQKPGQAPRGLIGGTNKRAP GVPARFSGSLLGDKAALTLLGAQPEDEAEYY CALWYSNLWVFGQGTKLTVL(SEQ ID NO:87) | SEQ ID NO:101 |
| husp34lg3.4 | RSSTGAVTTR NYAN(SEQ ID NO:35) | GTNKRAP(SE Q ID NO:30) | ALWYSNLWV(SEQ ID NO:31) | QAVVTQEPSLTVSPGGTVTLTCRSSTGAVTT RNYANWVQQKPGQAPRGLIGGTNKRAPG VPARFSGSLLGDKAALTLLGAQPEDEAEYYC ALWYSNLWVFGQGTKLTVL(SEQ ID NO:88) | SEQ ID NO:101 |
| husp34lg3.5 | RSSTGAVTTS GYAN(SEQ ID NO:36) | GTNKRAP(SE Q ID NO:30) | ALWYSNLWV(SEQ ID NO:31) | QAVVTQEPSLTVSPGGTVTLTCRSSTGAVTT SGYANWVQQKPGQAPRGLIGGTNKRAPG VPARFSGSLLGDKAALTLLGAQPEDEAEYYC ALWYSNLWVFGQGTKLTVL(SEQ ID NO:89) | SEQ ID NO:101 |
| husp34lg3.6 | RSSTGAVTTS NYAN(SEQ ID NO:29) | GTNKRAP(SE Q ID NO:30) | ALAYSNLWV(SEQ ID NO:37) | QAVVTQEPSLTVSPGGTVTLTCRSSTGAVTT SNYANWVQQKPGQAPRGLIGGTNKRAPG VPARFSGSLLGDKAALTLLGAQPEDEAEYYC ALAYSNLWVFGQGTKLTVL(SEQ ID NO:90) | SEQ ID NO:101 |
| husp34lg3. | RSSTGAVTTS | GTNKRAP(SE | ALWASNLWV(SEQ | QAVVTQEPSLTVSPGGTVTLTCRSSTGAVTT | SEQ ID NO:101 |

| 7 | NYAN(SEQ ID NO:29) | Q ID NO:30) | ID NO:38) | SNYANWVQQKPGQAPRGLIGGTNKRAPG VPARFSGSLLGDKAALTLLGAQPEDEAEYYC ALWASNLWVFGQGTKLTVL(SEQ ID NO:91) | |
|---|---|---|---|---|---|
| husp34lg3.8 | RSSTGAVTTS NYAN(SEQ ID NO:29) | GTNKRAP(SE Q ID NO:30) | ALWYRNLWV(SEQ ID NO:39) | QAVVTQEPSLTVSPGGTVTLTCRSSTGAVTT SNYANWVQQKPGQAPRGLIGGTNKRAPG VPARFSGSLLGDKAALTLLGAQPEDEAEYYC ALWYRNLWVFGQGTKLTVL(SEQ ID NO:92) | SEQ ID NO:101 |
| husp34lg3.9 | RSSTGAVTTS NYAN(SEQ ID NO:29) | GTNKRAP(SE Q ID NO:30) | ALWYSGLWV(SEQ ID NO:40) | QAVVTQEPSLTVSPGGTVTLTCRSSTGAVTT SNYANWVQQKPGQAPRGLIGGTNKRAPG VPARFSGSLLGDKAALTLLGAQPEDEAEYYC ALWYSGLWVFGQGTKLTVL(SEQ ID NO:93) | SEQ ID NO:101 |
| husp34lg3.10 | RSSTGAVGG SNYAN(SEQ ID NO:41) | GTNKRAP(SE Q ID NO:30) | ALWYSNLWV(SEQ ID NO:31) | QAVVTQEPSLTVSPGGTVTLTCRSSTGAVG GSNYANWVQQKPGQAPRGLIGGTNKRAP GVPARFSGSLLGDKAALTLLGAQPEDEAEYY CALWYSNLWVFGQGTKLTVL(SEQ ID NO:94) | SEQ ID NO:101 |
| husp34lg3.11 | RSSTGAVTTR GYAN(SEQ ID NO:42) | GTNKRAP(SE Q ID NO:30) | ALWYSNLWV(SEQ ID NO:31) | QAVVTQEPSLTVSPGGTVTLTCRSSTGAVTT RGYANWVQQKPGQAPRGLIGGTNKRAPG VPARFSGSLLGDKAALTLLGAQPEDEAEYYC ALWYSNLWVFGQGTKLTVL(SEQ ID | SEQ ID NO:101 |

| | | | | | |
|---|---|---|---|---|---|
| | | | | NO:95) | |
| husp34Ig3.12 | RSSTGAVTTSNYAN(SEQ ID NO:29) | GTNKRAP(SEQ ID NO:30) | ALAASNLWV(SEQ ID NO:43) | QAVVTQEPSLTVSPGGTVTLTCRSSTGAVTTSNYANWVQQKPGQAPRGLIGGTNKRAPGVPARFSGSLLGDKAALTLLGAQPEDEAEYYCALAASNLWVFGQGTKLTVL(SEQ ID NO:96) | SEQ ID NO:101 |
| husp34Ig3.13 | RSSTGAVTTSNYAN(SEQ ID NO:29) | GTNKRAP(SEQ ID NO:30) | ALWYRGLWV(SEQ ID NO:44) | QAVVTQEPSLTVSPGGTVTLTCRSSTGAVTTSNYANWVQQKPGQAPRGLIGGTNKRAPGVPARFSGSLLGDKAALTLLGAQPEDEAEYYCALWYRGLWVFGQGTKLTVL(SEQ ID NO:97) | SEQ ID NO:101 |
| husp34Ig3.14 | RSSTGAVTTSNYAN(SEQ ID NO:29) | GTNSRAP(SEQ ID NO:45) | ALWYSNLWV(SEQ ID NO:31) | QAVVTQEPSLTVSPGGTVTLTCRSSTGAVTTSNYANWVQQKPGQAPRGLIGGTNSRAPGVPARFSGSLLGDKAALTLLGAQPEDEAEYYCALWYSNLWVFGQGTKLTVL(SEQ ID NO:98) | SEQ ID NO:101 |
| husp34Ig3.15 | RSSTGAVTTSNYAN(SEQ ID NO:29) | GTNSRAP(SEQ ID NO:45) | ALWYSDLWV(SEQ ID NO:46) | QAVVTQEPSLTVSPGGTVTLTCRSSTGAVTTSNYANWVQQKPGQAPRGLIGGTNSRAPGVPARFSGSLLGDKAALTLLGAQPEDEAEYYCALWYSDLWVFGQGTKLTVL(SEQ ID NO:99) | SEQ ID NO:101 |

**Claims**

1. A humanized anti-CD3 antibody or antigen-binding fragment thereof, comprising:

    (i) VH comprising or consisting of an amino acid sequence shown in SEQ ID NO:50, and VL comprising or consisting of an amino acid sequence shown in SEQ ID NO:80;
    (ii) VH comprising or consisting of an amino acid sequence shown in SEQ ID NO:48, and VL comprising or consisting of an amino acid sequence shown in anyone of SEQ ID NO:77-84;
    (iii) VH comprising or consisting of an amino acid sequence shown in SEQ ID NO:49, and VL comprising or consisting of an amino acid sequence shown in anyone of SEQ ID NO:77-84;
    (iv) VH comprising or consisting of an amino acid sequence shown in SEQ ID NO:50, and VL comprising or consisting of an amino acid sequence shown in anyone of SEQ ID NO:77-84;
    (v) VH comprising or consisting of an amino acid sequence shown in SEQ ID NO:51, and VL comprising or consisting of an amino acid sequence shown in anyone of SEQ ID NO:77-84.

2. A humanized anti-CD3 antibody or antigen-binding fragment thereof, comprising:

    (i) a heavy chain variable region VH, which comprises or consists of the following amino acid sequence: an amino acid sequence of anyone of SEQ ID NO: 50, and the said amino acid sequence has the mutations selected from the following at 1, 2 or 3 positions of H31, H32, H33, H52, H52A, H52C, H53, H54, H95, H96, H97, H98, H99, H100, H100A, H100B, H100C (Kabat number) according to the Kabat numbering:
    amino acid Y, W or F is mutated into amino acid G, A, S; amino acid R, K or H is mutated into amino acid G, A or S; amino acid G is mutated to amino acid Y; amino acid N or Q is mutated into amino acid G, S, D or E; and/or amino acid T or S is mutated into G, L, R;
    (ii) a light chain variable region VL, which comprises or consists of the following amino acid sequence: an amino acid sequence of anyone of SEQ ID NO: 80, and the said amino acid sequence has the mutations selected from the following at 1, 2 or 3 positions of L24, L28, L29, L30, L31, L53, L91, L92, L93, L94 (Kabat number) according to the Kabat numbering:
    amino acid Y, W or F is mutated into amino acid G, A, S; amino acid R, K or H is mutated into amino acid G, A or S; amino acid G is mutated to amino acid Y; amino acid N or Q is mutated into amino acid G, S, D or E; and/or amino acid T or S is mutated into G, L, R.

3. The humanized anti-CD3 antibody or antigen-binding fragment thereof of claim 2, comprising:

(i) three complementary determining regions HCDR1, HCDR2 and HCDR3 contained in VH as shown in anyone of SEQ ID NO: 52-75, and three complementary determining regions LCDR1, LCDR2 and LCDR3 contained in VL as shown in SEQ ID NO: 80; or

(ii) three complementary determining regions HCDR1, HCDR2 and HCDR3 contained in VH as shown in SEQ ID NO: 50, and three complementary determining regions LCDR1, LCDR2 and LCDR3 contained in VL as shown in anyone of SEQ ID NO: 85-99.

**4.** The humanized anti-CD3 antibody or antigen-binding fragment thereof of claim 3, comprising:

(1) HCDR1 as shown in SEQ ID NO: 1, HCDR2 as shown in SEQ ID NO: 2, and HCDR3 as shown in SEQ ID NO: 8; LCDR1 as shown in SEQ ID NO: 29, LCDR2 as shown in SEQ ID NO: 30 and LCDR3 as shown in SEQ ID NO: 31;

(2) HCDR1 as shown in SEQ ID NO: 6, HCDR2 as shown in SEQ ID NO: 2, and HCDR3 as shown in SEQ ID NO: 8; LCDR1 as shown in SEQ ID NO: 29, LCDR2 as shown in SEQ ID NO: 30 and LCDR3 as shown in SEQ ID NO: 31;

(3) HCDR1 as shown in SEQ ID NO: 1, HCDR2 as shown in SEQ ID NO: 2, and HCDR3 as shown in SEQ ID NO: 14; LCDR1 as shown in SEQ ID NO: 29, LCDR2 as shown in SEQ ID NO: 30 and LCDR3 as shown in SEQ ID NO: 31;

(4) HCDR1 as shown in SEQ ID NO: 1, HCDR2 as shown in SEQ ID NO: 24, and HCDR3 as shown in SEQ ID NO: 8; LCDR1 as shown in SEQ ID NO: 29, LCDR2 as shown in SEQ ID NO: 30 and LCDR3 as shown in SEQ ID NO: 31;

(5) HCDR1 as shown in SEQ ID NO: 1, HCDR2 as shown in SEQ ID NO: 2, and HCDR3 as shown in SEQ ID NO: 26; LCDR1 as shown in SEQ ID NO: 29, LCDR2 as shown in SEQ ID NO: 30 and LCDR3 as shown in SEQ ID NO: 31;

(6) HCDR1 as shown in SEQ ID NO: 1, HCDR2 as shown in SEQ ID NO: 2, and HCDR3 as shown SEQ ID NO: 8; LCDR1 as shown in SEQ ID NO: 29, LCDR2 as shown in SEQ ID NO: 45 and LCDR3 as shown in SEQ ID NO: 46;

(7) HCDR1 as shown in anyone of SEQ ID NO: 4-6, HCDR2 as shown in SEQ ID NO: 2, and HCDR3 as shown in SEQ ID NO: 8; LCDR1 as shown in SEQ ID NO: 29, LCDR2 as shown in SEQ ID NO: 30 and LCDR3 as shown in SEQ ID NO: 31;

(8) HCDR1 as shown in SEQ ID NO: 1, HCDR2 as shown in anyone of SEQ ID NO: 7, 9, 10, 11 or 12, and HCDR3 as shown in SEQ ID NO: 8; LCDR1 as shown in SEQ ID NO: 29, LCDR2 as shown in SEQ ID NO: 30 and LCDR3 as shown in SEQ ID NO: 31; or

(9) HCDR1 as shown in SEQ ID NO: 1, HCDR2 as shown in SEQ ID NO: 2, and HCDR3 as shown in anyone of SEQ ID NO: 13-21; LCDR1 as shown in SEQ ID NO: 29, LCDR2 as shown in SEQ ID NO: 30 and LCDR3 as shown in SEQ ID NO: 31;

(10) HCDR1 as shown in SEQ ID NO: 1, HCDR2 as shown in SEQ ID NO: 23 or 24, and HCDR3 as shown in SEQ ID NO: 8; LCDR1 as shown in SEQ ID NO: 29, LCDR2 as shown in SEQ ID NO: 30 and LCDR3 as shown in SEQ ID NO: 31;

(11) HCDR1 as shown in SEQ ID NO: 22, HCDR2 as shown in SEQ ID NO: 2, and HCDR3 as shown in SEQ ID NO: 8; LCDR1 as shown in SEQ ID NO: 29, LCDR2 as shown in SEQ ID NO: 30 and LCDR3 as shown in SEQ ID NO: 31;

(12) HCDR1 as shown in SEQ ID NO: 1, HCDR2 as shown in SEQ ID NO: 2, and HCDR3 as shown in anyone of SEQ ID NO: 25-28; LCDR1 as shown in SEQ ID NO: 29, LCDR2 as shown in SEQ ID NO: 30 and LCDR3 as shown in SEQ ID NO: 31;

(13) HCDR1 as shown in SEQ ID NO: 1, HCDR2 as shown in SEQ ID NO: 2, and HCDR3 as shown in SEQ ID NO: 8; LCDR1 as shown in anyone of SEQ ID NO: 32-36, 41 and 42, LCDR2 as shown in SEQ ID NO: 30 and LCDR3 as shown in SEQ ID NO: 31;

(14) HCDR1 as shown in SEQ ID NO: 1, HCDR2 as shown in SEQ ID NO: 2, and HCDR3 as shown in SEQ ID NO: 8; LCDR1 as shown in SEQ ID NO: 29, LCDR2 as shown in SEQ ID NO: 30 and LCDR3 as shown in anyone of SEQ ID NO: 37-40, 43 and 44; or

(15) HCDR1 as shown in SEQ ID NO: 1, HCDR2 as shown in SEQ ID NO: 2, and HCDR3 as shown in SEQ ID NO: 8; LCDR1 as shown in SEQ ID NO: 29, LCDR2 as shown in SEQ ID NO: 45 and LCDR3 as shown in SEQ ID NO: 31 or 46;

(16) HCDR1 as shown in SEQ ID NO: 103, HCDR2 as shown in SEQ ID NO: 104, and HCDR3 as shown in anyone of SEQ ID NO: 105; LCDR1 as shown in SEQ ID NO: 29, LCDR2 as shown in SEQ ID NO: 30 and LCDR3 as shown in SEQ ID NO: 31;

(17) HCDR1 as shown in SEQ ID NO: 103, HCDR2 as shown in SEQ ID NO: 2, and HCDR3 as shown in SEQ ID NO: 8; LCDR1 as shown in SEQ ID NO: 29, LCDR2 as shown in SEQ ID NO: 30 and LCDR3 as shown in SEQ ID NO: 31;

(18) HCDR1 as shown in SEQ ID NO: 1, HCDR2 as shown in SEQ ID NO: 104, and HCDR3 as shown in SEQ ID NO: 8; LCDR1 as shown in SEQ ID NO: 29, LCDR2 as shown in SEQ ID NO: 30 and LCDR3 as shown in SEQ ID NO: 31;

(19) HCDR1 as shown in SEQ ID NO: 1, HCDR2 as shown in SEQ ID NO: 2, and HCDR3 as shown in SEQ ID NO: 105; LCDR1 as shown in SEQ ID NO: 29, LCDR2 as shown in SEQ ID NO: 30 and LCDR3 as shown in SEQ ID NO: 31;

(20) HCDR1 as shown in SEQ ID NO: 103, HCDR2 as shown in SEQ ID NO: 104, and HCDR3 as shown in SEQ ID NO: 8; LCDR1 as shown in SEQ ID NO: 29, LCDR2 as shown in SEQ ID NO: 30 and LCDR3 as shown in SEQ ID NO: 31;

(21) HCDR1 as shown in SEQ ID NO: 103, HCDR2 as shown in SEQ ID NO: 2, and HCDR3 as shown in SEQ ID NO: 105; LCDR1 as shown in SEQ ID NO: 29, LCDR2 as shown in SEQ ID NO: 30 and LCDR3 as shown in SEQ ID NO: 31;

(22) HCDR1 as shown in SEQ ID NO: 1, HCDR2 as shown in SEQ ID NO: 104, and HCDR3 as shown in SEQ ID NO: 105; LCDR1 as shown in SEQ ID NO: 29, LCDR2 as shown in SEQ ID NO: 30 and LCDR3 as shown in SEQ ID NO: 31;

(23) HCDR1 as shown in SEQ ID NO: 1, HCDR2 as shown in SEQ ID NO: 2, and HCDR3 as shown in SEQ ID NO: 8; LCDR1 as shown in SEQ ID NO: 106, LCDR2 as shown in SEQ ID NO: 30 or 45 and LCDR3 as shown in SEQ ID NO: 107;

(24) HCDR1 as shown in SEQ ID NO: 1, HCDR2 as shown in SEQ ID NO: 2, and HCDR3 as shown in SEQ ID NO: 8; LCDR1 as shown in SEQ ID NO: 106, LCDR2 as shown in SEQ ID NO: 30 or 45 and LCDR3 as shown in SEQ ID NO: 31;

(25) HCDR1 as shown in SEQ ID NO: 1, HCDR2 as shown in SEQ ID NO: 2, and HCDR3 as shown in SEQ ID NO: 8; LCDR1 as shown in SEQ ID NO: 29, LCDR2 as shown in SEQ ID NO: 30 or 45 and LCDR3 as shown in SEQ ID NO: 107; or

(26) HCDR1 as shown in SEQ ID NO: 103, HCDR2 as shown in SEQ ID NO: 104, and HCDR3 as shown in SEQ ID NO: 105; LCDR1 as shown in SEQ ID NO: 106, LCDR2 as shown in SEQ ID NO: 30 or 45 and LCDR3 as shown in SEQ ID NO: 107.

**5.** The humanized antibody of claim 3 or 4, comprising

(i) VH comprising or consisting an amino acid sequence of SEQ ID NO:50 or an amino acid sequence having at least 90% identity with the amino acid sequence of SEQ ID NO:50, and VL comprising or consisting an amino acid sequence of SEQ ID NO:80 or an amino acid sequence having at least 90% identity with the amino acid sequence of SEQ ID NO:80;

(ii) VH comprising or consisting an amino acid sequence of SEQ ID NO:54 or an amino acid sequence having at least 90% identity with the amino acid sequence of SEQ ID NO:54, and VL comprising or consisting an amino acid sequence of SEQ ID NO:80 or an amino acid sequence having at least 90% identity with the amino acid sequence of SEQ ID NO:80;

(iii) VH comprising or consisting an amino acid sequence of SEQ ID NO:61 or an amino acid sequence having at least 90% identity with the amino acid sequence of SEQ ID NO:61, and VL comprising or consisting an amino acid sequence of SEQ ID NO:80 or an amino acid sequence having at least 90% identity with the amino acid sequence of SEQ ID NO:80;

(iv) VH comprising or consisting an amino acid sequence of SEQ ID NO:71 or an amino acid sequence having at least 90% identity with the amino acid sequence of SEQ ID NO:71, and VL comprising or consisting an amino acid sequence of SEQ ID NO:80 or an amino acid sequence having at least 90% identity with the amino acid sequence of SEQ ID NO:80;

(v) VH comprising or consisting an amino acid sequence of SEQ ID NO:73 or an amino acid sequence having at least 90% identity with the amino acid sequence of SEQ ID NO:73, and VL comprising or consisting an amino acid sequence of SEQ ID NO:80 or an amino acid sequence having at least 90% identity with the amino acid sequence of SEQ ID NO:80;

(vi) VH comprising or consisting an amino acid sequence of SEQ ID NO:50 or an amino acid sequence having at least 90% identity with the amino acid sequence of SEQ ID NO:50, and VL comprising or consisting an amino acid sequence of SEQ ID NO:99 or an amino acid sequence having at least 90% identity with the amino acid sequence of SEQ ID NO:99;

(vii) VH comprising or consisting an amino acid sequence of anyone of SEQ ID NO:52-75 or an amino acid sequence having at least 90% identity with the amino acid sequence of anyone of SEQ ID NO:52-75, and VL comprising or consisting an amino acid sequence of SEQ ID NO:80 or an amino acid sequence having at least 90% identity with the amino acid sequence of SEQ ID NO:80; or

(viii) VH comprising or consisting an amino acid sequence of SEQ ID NO:50 or an amino acid sequence having at least 90% identity with the amino acid sequence of SEQ ID NO:50, and VL comprising or consisting an amino acid sequence of anyone of SEQ ID NO:85-99 or an amino acid sequence having at least 90% identity with the amino acid sequence of anyone of SEQ ID NO:85-99.

6. The antibody or antigen-binding fragment thereof of anyone of claim 1-5, further comprises heavy chain constant region and/or light chain constant region.

7. The antibody or antigen-binding fragment thereof of claim 6, wherein
the heavy chain constant region HC

   (i) comprises or consists of an amino acid sequence having at least 85%, 90%, 91%, 92%, 93%, 94%, 95%, 96%, 97%, 98% or 99% identity with the amino acid sequence of SEQ ID NO: 100;
   (ii) comprises or consists of an amino acid sequence of SEQ ID NO: 100; or
   (iii) comprises or consists of an amino acid sequence having one or more (preferably no more than 20 or 10, more preferably no more than 5, 4, 3, 2, 1) amino acid changes (preferably amino acid substitution, more preferably amino acid conservative substitution), compared to the amino acid sequence of SEQ ID NO: 100; and/or

   the light chain constant region LC

   (i) comprises or consists of an amino acid sequence having at least 85%, 90%, 91%, 92%, 93%, 94%, 95%, 96%, 97%, 98% or 99% identity with the amino acid sequence selected from SEQ ID NO: 101 or 102;
   (ii) comprises or consists of an amino acid sequence selected from SEQ ID NO: 101 or 102; or
   (iii) comprises or consists of an amino acid sequence having one or more (preferably no more than 20 or 10, more preferably no more than 5, 4, 3, 2, 1) amino acid changes (preferably amino acid substitution, more preferably amino acid conservative substitution), compared to the amino acid sequence selected from SEQ ID NO: 101 or 102.

8. The antibody binding to CD3 or antigen-binding fragment thereof of anyone of claim 1-7, wherein the said antibody is an antibody or antigen-binding fragment of IgG1 format or IgG2 format or IgG3 format or IgG4 format, and preferably the said antibody is an antibody or antigen-binding fragment of IgG1 format.

9. The antibody binding to CD3 or antigen-binding fragment thereof of anyone of claim 1-8, wherein the said antibody is a monoclonal antibody.

10. The antibody or antigen-binding fragment thereof of anyone of claim 1-9, wherein the said antigen-binding fragment is selected from the following antibody fragment: Fab, Fab' , Fab'-SH, Fv, a single chain antibody (such as scFv), (Fab')$_2$, a single domain antibody, such as VHH, dAb or a linear antibody.

11. The antibody or antigen-binding fragment thereof of anyone of claim 1-9, wherein the said antibody is multispecific antibody, such as bispecific antibody, which comprises a first antigen-binding region specifically binding to CD3 and a second antigen-binding region binding to a tumor associated antigen, and preferably the tumor associated antigens are HER2 or CD70 or CLAUDIN18.2.

12. An isolated nucleic acid that encodes the light chain variable region or heavy chain variable region, or light chain or heavy chain of the antibody binding to CD3 or antigen-binding fragment thereof of anyone of claims 1 to 11.

13. A vector comprising the nucleic acid of claim 12, the said vector is an expression vector.

14. A host cell comprising the nucleic acid of claim 12 or the vector of claim 13, preferably, the said host cell is prokaryotic or eukaryotic cell, more preferably selected from yeast cells, mammalian cells (such as 293 cells or CHO cells, such as CHO-S cells or HEK293 cells) or other cells suitable for preparing antibodies or antigen-binding fragments thereof.

15. A method for preparing an antibody binding to CD3 or antigen-binding fragment thereof, the said method comprises culturing the host cell of claim 14 under the conditions suitable for expressing the nucleic acid encoding the antibody binding to CD3 or antigen-binding fragment thereof of anyone of claim 1 to 11, optionally isolating the antibody or antigen-binding fragment thereof, and optionally the said method further comprises recovering the said antibody binding to CD3 or antigen-binding fragment thereof from the said host cell.

16. A immunoconjugate, which comprise the anti-CD3 antibody or antigen-binding fragment thereof of anyone of claim 1 to 11 and other substances, such as labels.

17. A pharmaceutical composition, which comprises the antibody binding to CD3 or antigen-binding fragment thereof of anyone of claims 1 to 11 or the immunoconjugate of claim 16, and optionally one or more other therapeutic agents, such as chemotherapeutic agents, cytokines, cytotoxic agents, other antibodies, small molecular drugs or immunomodulators, and optionally pharmaceutical excipients.

18. A pharmaceutical combination, which comprises the antibody binding to CD3 or antigen-binding fragment thereof of anyone of claims 1 to 11 or the immunoconjugate of claim 16, and one or more other therapeutic agents, such as chemotherapeutic agents, cytokines, cytotoxic agents, other antibodies, small molecular drugs or immunomodulators.

19. A method for preventing or treating tumor in a subject, the said method comprises administering to the subject an effective amount of the antibody binding to CD3 or antigen-binding fragment thereof of anyone of claims 1 to 11, or the immunoconjugate of claim 16, or the drug composition of claim 17, or the pharmaceutical combination of claim 18.

20. The method of claim 19, the said method further comprises administering to the patient one or more therapies, such as therapeutic modes and/or other therapeutic agents, preferably the therapeutic mode comprise radiotherapy or surgery, or therapeutic agents comprise chemotherapy agents, cytokines, cytotoxic agents, other antibodies, small molecule drugs or immune modulators.

21. A method for detecting CD3 in a sample, the said method comprises

(a) Contacting a sample with the antibody or antigen-binding fragment thereof of anyone of claims 1 to 11, or the immunoconjugate of claim 16; and
(b) Detecting the formation of complex between the antibody or antigen-binding fragment thereof with CD3; optionally, the antibody is detectably labeled.

A

B

Figure 1

C

| ■ 500 | ■ 166.6666667 | ■ 55.55555556 | ■ 18.51851852 |
| ■ 6.172839506 | ■ 2.057613169 | ■ 0.685871056 | ■ 0.228623685 |
| ■ 0.076207895 | ■ 0.025402632 | ■ 0.008467544 | ■ 0.002822515 |

D

Jurkat cell line binding

| | sp34 | hzsp34.24 | hzsp34.38 | hzsp34.40 | hzsp34.42 | hzsp34.45 |
|---|---|---|---|---|---|---|
| EC50 | 1.998 | 1.255 | 7.854 | 91.86 | 39.20 | 122.4 |

Figure 1(cont)

Figure 2

A

■ 900  ■ 300  ■ 100

B

■ 900  ■ 300  ■ 100

C

Jurkat cell line binding

Figure 3

Figure 4

A

anti-Her2 variable region

anti-CD3 variable region

Knob hole

B

Jurkat Reporting Experiment depending on SK-BR-3

Figure 5

A.

anti-CD70 variable region

anti-CD3 variable region

B.

Jurkat Reporting Experiment depending on NOMO

Figure 6

Figure 7

**191106 NUGC-4**

| | 030 | 032 | 033 |
|---|---|---|---|
| EC50 | 0.0003523 | 0.0003184 | 0.4362 |

Figure 8

**DAN-G-hCLDN18.2**

| | 030 | 032 | 033 |
|---|---|---|---|
| EC50 | 8.026e-005 | 6.938e-005 | 0.08177 |

Figure 9

**191106 L363**

Figure 10

Figure 11

Figure 12

Figure 13

Figure 14

A

B

Figure 15

Figure 16

## INTERNATIONAL SEARCH REPORT

| International application No. |
| --- |
| **PCT/CN2021/121285** |

### A. CLASSIFICATION OF SUBJECT MATTER

C07K 16/28(2006.01)i;  C12N 5/10(2006.01)i;  C12N 15/13(2006.01)i;  C12N 15/63(2006.01)i;  A61K 39/395(2006.01)i; A61P 35/00(2006.01)i

According to International Patent Classification (IPC) or to both national classification and IPC

### B. FIELDS SEARCHED

Minimum documentation searched (classification system followed by classification symbols)

A61K; C07K; C12N; A61P

Documentation searched other than minimum documentation to the extent that such documents are included in the fields searched

Electronic data base consulted during the international search (name of data base and, where practicable, search terms used)

VEN, USTXT, WOTXT, STN, SpringerLink, ISI Web of Knowledge, Wiley InterScience, Embase, NCBI, EBI, Unipro, PUBMED, CNKI, 万方, 中国专利生物序列检索系统, SEQ1-51, SEQ 77-99, CD3抗体, 人源化, 双特异性抗体, bispecific, antibody, variable, mutat.

### C. DOCUMENTS CONSIDERED TO BE RELEVANT

| Category* | Citation of document, with indication, where appropriate, of the relevant passages | Relevant to claim No. |
| --- | --- | --- |
| PX | WO 2021063330 A1 (HARBOUR BIOMED SUZHOU CO., LTD.) 08 April 2021 (2021-04-08)<br>  claims 1-15, and embodiment 4 | 1-21 |
| Y | CN 111315779 A (GREEN CROSS CORP et al.) 19 June 2020 (2020-06-19)<br>  embodiments 1-4, SEQ ID NO:1, SEQ ID NO:19 | 1-21 |
| Y | CN 105051069 A (XENCOR INC.) 11 November 2015 (2015-11-11)<br>  claims 7, 12, 22, 28, description paragraphs 165-166, SEQ ID NO:97, SEQ ID NO:98 | 1-21 |
| Y | CN 111518214 A (L&L BIOPHARMA, CO., LTD.) 11 August 2020 (2020-08-11)<br>  claims 1-17 | 1-21 |
| Y | CN 111662382 A (REYOUNG (SUZHOU) BIOPHARMACEUTICALS., LTD.) 15 September 2020 (2020-09-15)<br>  claims 1-10, and embodiment 1 | 1-21 |
| Y | CN 110914296 A (WUHAN YZY BIOPHARMA CO., LTD.) 24 March 2020 (2020-03-24)<br>  claims 1-12, 17, table 3 | 1-21 |

☐ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **16 December 2021** | **30 December 2021** |

| Name and mailing address of the ISA/CN | Authorized officer |
| --- | --- |
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

| International application No. |
| --- |
| **PCT/CN2021/121285** |

**Box No. I**      **Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑ forming part of the international application as filed:

        ☑ in the form of an Annex C/ST.25 text file.

        ☐ on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

## INTERNATIONAL SEARCH REPORT

| International application No. |
| --- |
| **PCT/CN2021/121285** |

| **Box No. II**     **Observations where certain claims were found unsearchable (Continuation of item 2 of first sheet)** |
| --- |

This international search report has not been established in respect of certain claims under Article 17(2)(a) for the following reasons:

1. ☑   Claims Nos.: **19-20**
   because they relate to subject matter not required to be searched by this Authority, namely:

   [1]   Claims 19-20 relate to a method for preventing or treating tumors in a patient, and therefore do not comply with PCT Rule 39.1(iv), but a search has still been carried out on the basis of a corresponding pharmaceutical use.

2. ☐   Claims Nos.:
   because they relate to parts of the international application that do not comply with the prescribed requirements to such an extent that no meaningful international search can be carried out, specifically:

3. ☐   Claims Nos.:
   because they are dependent claims and are not drafted in accordance with the second and third sentences of Rule 6.4(a).

Form PCT/ISA/210 (continuation of first sheet) (January 2015)

**INTERNATIONAL SEARCH REPORT**
Information on patent family members

International application No.

**PCT/CN2021/121285**

| Patent document cited in search report | | | Publication date (day/month/year) | Patent family member(s) | | | Publication date (day/month/year) |
|---|---|---|---|---|---|---|---|
| WO | 2021063330 | A1 | 08 April 2021 | CN | 113015749 | A | 22 June 2021 |
| CN | 111315779 | A | 19 June 2020 | SG | 11202003273 P | A | 28 May 2020 |
| | | | | JP | 2021501571 | A | 21 January 2021 |
| | | | | IL | 274007 | D0 | 31 May 2020 |
| | | | | WO | 2019078697 | A3 | 04 July 2019 |
| | | | | WO | 2019078697 | A2 | 25 April 2019 |
| | | | | BR | 112020007770 | A2 | 20 October 2020 |
| | | | | EP | 3699192 | A2 | 26 August 2020 |
| | | | | EA | 202090944 | A1 | 16 July 2020 |
| | | | | WO | 2019078697 | A8 | 09 April 2020 |
| | | | | CA | 3077007 | A1 | 25 April 2019 |
| | | | | KR | 101973060 | B1 | 26 April 2019 |
| | | | | AU | 2018353787 | A1 | 21 May 2020 |
| | | | | US | 2020317779 | A1 | 08 October 2020 |
| CN | 105051069 | A | 11 November 2015 | EP | 3620473 | A1 | 11 March 2020 |
| | | | | JP | 2020018318 | A | 06 February 2020 |
| | | | | AU | 2014205086 | B2 | 18 April 2019 |
| | | | | CN | 105051069 | B | 10 December 2019 |
| | | | | KR | 20150105472 | A | 16 September 2015 |
| | | | | US | 2014370013 | A1 | 18 December 2014 |
| | | | | AU | 2019204841 | A1 | 25 July 2019 |
| | | | | EP | 2943511 | A4 | 31 August 2016 |
| | | | | JP | 6618362 | B2 | 11 December 2019 |
| | | | | KR | 20200065110 | A | 08 June 2020 |
| | | | | IL | 239941 | A | 30 April 2020 |
| | | | | US | 2021163627 | A1 | 03 June 2021 |
| | | | | EP | 2943511 | A1 | 18 November 2015 |
| | | | | AU | 2014205086 | A1 | 23 July 2015 |
| | | | | KR | 102211837 | B1 | 03 February 2021 |
| | | | | JP | 2016504418 | A | 12 February 2016 |
| | | | | IL | 281703 | D0 | 31 May 2021 |
| | | | | SI | 2943511 | T1 | 31 January 2020 |
| | | | | IL | 239941 | D0 | 31 August 2015 |
| | | | | LT | 2943511 | T | 11 November 2019 |
| | | | | DK | 2943511 | T3 | 21 October 2019 |
| | | | | WO | 2014110601 | A1 | 17 July 2014 |
| | | | | US | 2018142040 | A1 | 24 May 2018 |
| | | | | US | 9650446 | B2 | 16 May 2017 |
| | | | | IL | 273320 | A | 25 March 2021 |
| | | | | HR | P20191865 | T1 | 10 January 2020 |
| | | | | EP | 2943511 | B1 | 07 August 2019 |
| | | | | IL | 273320 | D0 | 30 April 2020 |
| | | | | US | 10738133 | B2 | 11 August 2020 |
| | | | | CA | 2898100 | A1 | 17 July 2014 |
| | | | | US | 10738132 | B2 | 11 August 2020 |
| | | | | CN | 110981964 | A | 10 April 2020 |
| | | | | US | 2014288275 | A1 | 25 September 2014 |
| CN | 111518214 | A | 11 August 2020 | None | | | |
| CN | 110914296 | A | 24 March 2020 | CN | 110914296 | B | 19 February 2021 |
| | | | | CN | 112142847 | A | 29 December 2020 |

Form PCT/ISA/210 (patent family annex) (January 2015)

## INTERNATIONAL SEARCH REPORT
### Information on patent family members

International application No.

**PCT/CN2021/121285**

| Patent document cited in search report | Publication date (day/month/year) | Patent family member(s) | | Publication date (day/month/year) |
|---|---|---|---|---|
| | | WO 2020168554 A1 | | 27 August 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5731168 A
- US 7695936 B
- WO 2004073656 A
- CN 202010570517X
- US 8236308 B
- GE 11003495

### Non-patent literature cited in the description

- **CHOTHIA et al.** *Nature,* 1989, vol. 342, 877-883
- **AL-LAZIKANI et al.** Standard conformations for the canonical structures of immunoglobulins. *Journal of Molecular Biology,* 1997, vol. 273, 927-948
- **KABAT et al.** Sequences of Proteins of Immunological Interest. U.S. Department of Health and Human Services, National Institutes of Health, 1987
- **KABAT et al.** Sequences of Proteins of Immunological Interest. Public Health Service, National Institutes of Health, 1991
- **NEEDLEMA ; WUNSCH.** *J. Mol. Biol.,* 1970, vol. 48, 444-453
- *CABIOS,* 1989, vol. 4, 11-17
- Current Protocols in Molecular Biology. John Wiley & Sons, 1989, 6.3.1-6.3.6
- **FAN et al.** *Journal of Hematology & Oncology,* 2015, vol. 8, 130
- **R.C. ROWE ; P.J. SESKEY ; S.C. OWEN.** Handbook of Pharmaceutical Excipients. Pharmaceutical Press
- *J. Immunol. Methods.,* 1994, vol. 178, 195
- **ESTEP, P et al.** High throughput solution Based measurement of antibody-antigen affinity and affinity binding. *MAbs,* 2013, vol. 5 (2), 270-8
- **A. MARGARET MERCHANT et al.** *Nature Biotechnology,* 1998
- **ESTEP, P et al.** High throughput solution based measurement of antibody-antigen affinity and affinity binding. *MAbs,* 2013, vol. 5 (2), 270-8